(19) Europäisches Patentamt
European Patent Office
Office européen des brevets

(11) **EP 3 636 777 A1**

(12) **EUROPEAN PATENT APPLICATION**

(43) Date of publication:
**15.04.2020  Bulletin 2020/16**

(51) Int Cl.:
***C12Q 1/6869*** (2018.01)    ***G16B 30/00*** (2019.01)

(21) Application number: **19204072.3**

(22) Date of filing: **13.07.2016**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR** | (72) Inventors:<br>• **ALLEMEERSCH, Joke**<br>  **3200 Aarschot (BE)**<br>• **DEVOGELAERE, Benoit**<br>  **1800 Vilvoorde (BE)** |
| (30) Priority: **13.07.2015  EP 15176401**<br>**13.07.2015  US 201562191697 P**<br>**13.07.2015  US 201562191700 P**<br>**13.07.2015  EP 15176404** | (74) Representative: **Brantsandpatents bvba**<br>**Pauline Van Pottelsberghelaan 24**<br>**9051 Ghent (BE)** |
| (62) Document number(s) of the earlier application(s) in accordance with Art. 76 EPC:<br>**16738186.2 / 3 322 816** | Remarks:<br>This application was filed on 18.10.2019 as a divisional application to the application mentioned under INID code 62. |
| (71) Applicant: **Agilent Technologies Belgium NV**<br>**1831 Machelen (BE)** | |

(54) **SYSTEM AND METHODOLOGY FOR THE ANALYSIS OF GENOMIC DATA OBTAINED FROM A SUBJECT**

(57)    The present teachings describe a method for identifying the presence of tumor-derived cell-free DNA in a mammal.

EP 3 636 777 A1

**Description**

**Technical Field**

The present teachings pertain to a method and system for the analysis of genomic data from a subject. In particular, the present teachings relate to technologies for sensitive and high accuracy determination of the presence copy number variations and chromosomal abnormalities associated with a biological sample.

**INTRODUCTION**

The availability of high-throughput DNA sequencing technologies permits comprehensive investigations into the number and types of sequence variants possessed by individuals in different populations and with different diseases. Whole genome sequencing has become more accessible in clinical and diagnostic settings as high-throughput sequencing costs and efficiency continues to improve. As costs decline, high-throughput sequencing can be expected to become a mainstay tool, not only in human phenotype based sequencing projects, but also in forward genetics applications in model organisms, and for the diagnosis of diseases previously considered to be idiopathic.

In various applications, when a sample sequence is obtained, efforts can be made to identify the location and character of those portions of the sample sequence that differ from one or more "standard" reference sequences, with sequence differences commonly referred to as variants. Sequence analysis can aid in the identification of those portions of an individual's genome that could potentially contribute to a clinical condition or other trait of the individual. For example, comparison of the sequence for a selected individual with a reference human genome sequence such as that maintained by the University of California, Santa Cruz, can be performed to generate a list of the variants that exist between an individual's sequence and the reference sequence.

This variant list may include thousands, if not millions, of variants, but may provide little if any readily accessible information on the impact any particular variant may have on gene function. Research programs around the world are continually gathering information relating particular variants to gene function, disease states, and the like. Furthermore, a variety of computational methods have been developed to deduce possible physiological effects of some types of variants based on their location on the genome and the nature of the variant, even if no laboratory biochemical or clinical studies have been undertaken on that particular variant.

Clinical evaluations are increasingly leveraging genomic analysis. In this regard, a particularly useful application of high throughput sequencing technologies relates to prenatal evaluation of a fetus.

The presence of circulating extracellular DNA in the peripheral urine is a phenomenon that can aid in diagnostic and clinical workflows as an alternative to more invasive techniques. It has been shown that in the case of a pregnant woman extracellular fetal DNA is present in the maternal blood circulation and can be detected in maternal plasma or serum. While this type of DNA may be referred to as fetal DNA, it may actually arise from placental DNA, and differences may occur between fetal and placental DNA due, for example, to mosaicism originating during embryogenesis. Throughout this document, the term "fetal DNA" will be used as this is commonly used terminology to describe this type of DNA. Studies have shown that circulating fetal genetic material can be used for the sensitive determination, e.g. by PCR (polymerase chain reaction) technology, of fetal genetic loci which are completely absent from the maternal genome. Examples of such fetal genetic loci are the fetal RhD gene in pregnancies at risk for HDN (hemolytic disease of the fetus and newborn) or fetal Y chromosome-specific sequences in pregnancies at risk for an X chromosome-linked disorder e.g. hemophilia or fragile X syndrome.

According to some statistics, fetal aneuploidy and other chromosomal aberrations may affect approximately 9 out of 1000 live births. Current standards for diagnosing chromosomal abnormalities often involve karyotyping of fetal cells obtained via invasive procedures such as chorionic villus sampling and amniocentesis. These procedures impose small but potentially significant risks to both the fetus and the mother. In the past years, progression has been made to develop non-invasive screening methods for fetal chromosomal abnormalities.

Since the recognition of intact fetal cells in maternal blood, there has been intense interest in using maternal blood as a diagnostic window into fetal genetics.

EP2183693 and family members describe a methodology for performing a prenatal diagnosis of a fetal chromosomal aneuploidy in a biological sample obtained from the pregnant mother. The sample is randomly sequenced and the obtained sequences are used to determine a parameter which is a used to evaluate the presence or absence of a fetal aneuploidy.

US8195415 describes a method for evaluating whether a chromosome has an abnormal distribution in a sample obtained from a subject. The method includes shotgun sequencing of the DNA present in the sample, and subsequently aligning the obtained sequence tags to chromosome portions. Values are determined based on the number of alignments obtained which are then used to calculate a differential which is determinative of whether or not an abnormal distribution exists.

Bayindir et al., 2015 describes a noninvasive prenatal testing methodology based on the Z and ZZ score, whereby said Z-score is a chromosomal-wide-Z-score and the ZZ-score is calculated as the standard score of the Z-score of a given autosome in comparison with the Z-scores of remaining autosomes.

Although above mentioned methodologies have their value, the ratio of false positives and negatives remain high in the field. It is therefore highly desirable to provide methodologies that are able to lower the percentage of false positives and especially false negatives, in order to provide more accurate and robust screening.

The present teachings provide a highly accurate non-invasive analytical workflows and methodologies for identifying copy number variations, fetal aneuploidy, and may be used in connection with other cell-free methods for disease analysis (e.g. liquid biopsies). Applying improved methods for chromosomal assessment along with methods for determination of the fetal fraction for a mixed maternal / fetal sample provides useful quality metrics to reduce overall false-negatives.

From a broader perspective, the present teachings also provide a methodology and tools to analyzing genomic data, e.g. for genomic variant annotation. For example, the present teachings may be adapted to provide accurate, essentially non-invasive methodologies for determining whether an individual has tumor-derived cell-free DNA in his or her peripheral blood, for confirming a cancer diagnosis, for aiding in the classification of a cancer, for assessing the treatment response, and for improved monitoring of the patient.

An important endeavor in human medical research is the discovery of genetic abnormalities that produce adverse health consequences. In many cases, specific genes and/or critical diagnostic markers have been identified in segments of the genome that are present at abnormal copy numbers. For example, in prenatal diagnosis, extra or missing copies of whole chromosomes are frequently occurring genetic abnormalities. In cancer, deletion or amplification of copies of whole chromosomes or chromosomal segments or specific regions of the genome, may be relatively common occurrences.

Much information about copy number variation has been provided by cytogenetic resolution that has permitted recognition of structural abnormalities. Conventional procedures for genetic screening and biological dosimetry have utilized invasive procedures e.g. amniocentesis or solid tumor biopsies, to obtain cells for the analysis of karyotypes. Recognizing the need for more rapid testing methods that do not require cell culture, fluorescence in situ hybridization (FISH), quantitative fluorescence PCR (QF-PCR) and array-Comparative Genomic Hybridization (array-CGH) have been developed as molecular-cytogenetic methods for the analysis of copy number variations.

The advent of technologies that allow for sequencing entire genomes in relatively short time, and the discovery of circulating cell-free DNA (cfDNA) have provided the opportunity to compare genetic material originating from one chromosome to be compared to that of another without the risks associated with invasive sampling methods.

US20130034546 and US20130310263 both describe methods for identifying the presence of cancer or the risk to develop the latter based on an analysis of obtained sequence reads obtained from a cell free DNA fraction in a sample.Vandenberghe et al., "Non-invasive detection of genomic imbalances in Hodgkin/Reed-Sternberg cells in early and advanced stage Hodgkin's lymphoma by sequencing of circulating cell-free DNA: a technical proof-of-principle study", 2015 describes a methodology for identifying genomic imbalances in a presymptomatic Hodgkin lymphoma cancer patient by massive parallel sequencing of circulating cell-free DNA.

It is observed that limitations in these methods exist, which include insufficient sensitivity stemming from the limited levels of cell-free DNA (cfDNA), as well as sequencing bias for the technology stemming from the inherent nature of genomic information. Thus there is a continuing need for noninvasive methods that provide high specificity, sensitivity, and applicability to reliably diagnose copy number changes in a variety of clinical settings.

In the above-mentioned methodologies it is important to avoid false positives and negatives. In fact, conventional approaches generally are limited by high error rates that reduce the overall value of the methods in clinical contexts. It is therefore important to provide analytical approaches that are able to reduce or eliminate false positives and especially false negatives, in order to provide more accurate screening tools. The present teachings a desirably provide increased accuracy and reduce false calls as described in greater detail herein below.

## SUMMARY

In various embodiments, the present teachings pertain to methods for determining the presence or absence of aneuploidy according to claim 1 or any of the dependent claims. The claimed methodology allows evaluating the presence or absence of such aneuploidy relative to a reference. The current methodology may further be used to provide a reliable and robust parameter set for determining the presence of an aneuploidy or for verifying and/or improving the accuracy of an aneuploidy calling. The methodology is highly sensitive and minimizes false positives and false negatives. The present teachings equally provide a method for setting up a non-invasive diagnostic or predictive tool for the prediction or diagnosis of an aneuploidy.

The present teachings also provide for a method to determine the fetal fraction according to claim 37 and dependent claims. The method provides a reliable estimation of the fetal fraction in a sample based on the available low-coverage, random sequencing data. The latter also serves as an additional quality control for the aneuploidy detection, especially for the cases where no aneuploidy was detected (as some of these could be false-negatives, because the fetal fraction was too low to enable the detection of an aneuploidy using low-coverage sequencing).

Finally, the current methodology provides for a computer program product according to claim 29, able to perform one or more operations according to the present teachings and a report generated thereby according to claim 35 that may be useful in assessing the status of a sample.

In a second aspect, the present teachings pertain to a method for identifying the presence of tumor-derived cell-free DNA in a mammal according to claim 41, a computer program according to claim 67 and a kit and report according to respectively claim 69 and 70. The methodology offers a noninvasive method that provides high specificity, sensitivity, and applicability to reliably diagnose copy number changes in a variety of clinical settings.

**FIGURES**

**Figure 1** shows a report according to an embodiment of the present teachings, displaying calculated parameters and visual representation for chromosome 21 in a sample A, whereby said chromosome 21 was identified as abnormal by the method according to the present teachings.

**Figure 2** shows a plot of the parameters obtained for all chromosomes within one sample A. Only chromosome 21 showed an aberration.

**Figure 3** shows a report according to an embodiment of the present teachings, displaying calculated parameters and visual representation for chromosome 11 in sample A. No aberrations are observed.

**Figure 4** shows a plot of the calculated secondary parameters according to an embodiment of the present teachings, calculated for all chromosomes in sample A. A trisomy was observed.

**Figure 5** depicts a report according to an embodiment of the present teachings, displaying calculated parameters and visual representation for chromosome 16 in sample B. A trisomy was observed.

**Figure 6** shows a plot of the parameters obtained for all chromosomes within sample B. Only one chromosome, chromosome 16, showed an aberration.

**Figure 7** shows a report according to an embodiment of the present teachings, displaying calculated parameters and visual representation for chromosome 1 in sample B. No trisomy was observed.

**Figure 8** shows a plot of the calculated secondary parameters according to an embodiment of the present teachings, calculated for all chromosomes in sample B. A trisomy was observed.

**Figure 9** shows a report according to an embodiment of the present teachings, displaying calculated parameters and visual representation for chromosome 21 in a sample A, whereby said chromosome 21 was identified as abnormal by the method according to the current invention.

**Figure 10** shows a plot of the parameters obtained for all chromosomes within one sample A. Only one chromosome showed an aberration.

**Figure 11** shows a report according to an embodiment of the present teachings, displaying calculated parameters and visual representation for chromosome 11 in sample A. No aberrations are observed.

**Figure 12** depicts a report according to an embodiment of the present teachings, displaying calculated parameters and visual representation for chromosome 16 in sample B. An aneuploidy was observed.

**Figure 13** shows a plot of the parameters obtained for all chromosomes within sample B. Only one chromosome 16 showed an aberration.

**Figure 14** shows a report according to an embodiment of the present teachings, displaying calculated parameters and visual representation for chromosome 1 in sample B. No trisomy was observed.

**Figure 15** show a plot of a secondary parameter indicative for the success rate of the experiment obtained for all chromosomes in sample A according to an embodiment of the current example. The experiment was successful.

**Figure 16** show a plot of a secondary parameter indicative for the success rate of the experiment obtained for all chromosomes in sample B according to an embodiment of the current example. The experiment was successful.

**Figure 17** shows a plot for a chromosome in a sample, obtained by an embodiment of the present teachings, indicating the presence of a polymorphic site, more particularly a Copy Number Variation that could be present in the maternal genome instead of the fetal genome.

**Figures 18 to 24** show plots for a chromosome in a sample obtained by an embodiment of the present teachings. The methodology of the present teachings is proven to have a higher sensitivity in view of prior art methods.

**Figure 25** shows a plot with the number of reads mapping to the Y chromosome for samples that were categorized as male, female, or of undetermined gender.

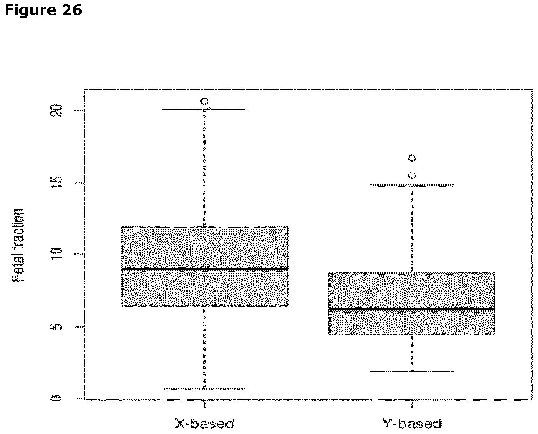

**Figure 26** shows a plot with of the X and Y-based fetal fraction estimations for a set of male pregnancies.

**Figure 27** shows the histograms of the normalized counts for a particular polymorphic site within a set of test samples.

**Figure 28 and 29** show a plot of the estimated fetal fraction for a set of male pregnancies based on one particular informative polymorphic site, as calculated using an embodiment of present teachings (X-axis) versus based on the read counts of chromosome X or Y (Y-axis).

**Figure 30** shows a plot visualizing the estimated fetal fraction for a set of male pregnancies based on the informative polymorphic site identified in the sample as calculated using an embodiment of the present teachings (X-axis) versus the estimated fetal fraction based on the read counts of chromosome X or Y (Y-axis).

**Figure 31** shows a plot of secondary parameters obtained according to an embodiment of the present teachings per chromosome from a sample.

**Figure 32** shows histograms/reports of chromosomes from a sample, whereby the calculation of a parameter according to an embodiment of the present teachings indicates genome-wide instability which could be suggestive for the presence of a tumor.

**Figure 33** shows various plots of chromosomal behavior in a test sample compared to the same chromosome in a reference sample (behaving normal).

**Figures 34 and 35** show the distribution of chromosomes in a reference set based on the Z-score and the ZofZ parameter calculated according to an embodiment of the present teachings.

**Figure 36** shows the calculated chromosome doses for each chromosome in a test sample, either by using chromosome 7 or chromosome 11 as normalizing chromosome.

**Figures 37 to 43** show various reports according to an embodiment of the present teachings, providing information on the presence or absence of an aneuploidy.

**Figure 44** provides a schematic overview of the methodology according to the present teachings.

## DEFINITIONS

Unless otherwise defined, all terms used in disclosing the present teachings, including technical and scientific terms, have the meaning as commonly understood by one of ordinary skill in the art to which the present teachings belong. By means of further guidance, term definitions are included to better appreciate the teachings of the present teachings.

The term "biological sample" as used herein refers to any sample that is taken from a subject or organism (e.g., a human, such as a pregnant woman) and contains one or more nucleic acid molecule(s) of interest. A biological sample may for instance be a blood sample, serum, urine, saliva, feces, a biopsy, etc..

The term "nucleic acid" or "polynucleotide" refers to a deoxyribonucleic acid (DNA) or ribonucleic acid (RNA) and a polymer thereof in either single- or double-stranded form. Unless specifically limited, the term encompasses nucleic acids containing known analogs of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, Single Nucleotide Polymorphisms (SNPs), and complementary sequences as well as the sequence explicitly indicated. Specifically, degenerate codon substitutions may be achieved by generating sequences in which the third position of one or more selected (or all) codons is substituted with mixed-base and/or deoxyinosine residues. The term nucleic acid is used interchangeably with gene, DNA, cDNA, mRNA, small noncoding RNA, micro RNA (miRNA), Piwi-interacting RNA, and short hairpin RNA (shRNA) encoded by a gene or locus.

The term "gene" means the segment of DNA involved in producing a polypeptide chain. It may include regions preceding and following the coding region (leader and trailer) as well as intervening sequences (introns) between individual coding segments (exons).

The term "reaction" as used herein refers to any process involving a chemical, enzymatic, or physical action that is indicative of the presence or absence of a particular polynucleotide sequence of interest. An example of a "reaction" is an amplification reaction such as a polymerase chain reaction (PCR). Another example of a "reaction" is a sequencing reaction, either by synthesis, ligation, hybridization or by passing the DNA through a pore and measuring signals that are indicative for a particular nucleotide. An "informative reaction" is one that indicates the presence of one or more particular polynucleotide sequence of interest, and in one case where only one sequence of interest is present. The term "well" as used herein refers to a reaction at a predetermined location within a confined structure, e.g., a well-shaped vial, cell, or chamber in a PCR array or e.g. the individual reaction volumes in which sequencing reactions take place (thereby including so-called patterned flow cells from Illumina).

The term "clinically relevant nucleic acid sequence" or "target chromosome or chromosomal segment" as used herein can refer to a polynucleotide sequence corresponding to a segment of a larger genomic sequence whose potential imbalance is being tested or to the larger genomic sequence itself. One example is the sequence of chromosome 21. Other examples include chromosome 18, 13, X and Y. Yet other examples include mutated genetic sequences or genetic polymorphisms or copy number variations (CNVs) that a fetus may inherit from one or both of its parents. Yet other examples include sequences which are mutated, deleted, or amplified in a malignant tumor, e.g. sequences in which loss of heterozygosity or gene duplication occur. In some embodiments, multiple clinically relevant nucleic acid sequences, or equivalently multiple makers of the clinically relevant nucleic acid sequence, can be used to provide data for detecting the imbalance. For instance, data from five non-consecutive sequences on chromosome 21 can be used in an additive fashion for the determination of possible chromosomal 21 imbalance, effectively reducing the need of sample volume to 1/5.

The term "overrepresented nucleic acid sequence" as used herein refers to the nucleic acid sequence among two sequences of interest (e.g., a clinically relevant sequence and a background sequence) that is in more abundance than the other sequence in a biological sample.

The term "based on" as used herein means "based at least in part on" and refers to one value (or result) being used in the determination of another value, such as occurs in the relationship of an input of a method and the output of that method. The term "derive" as used herein also refers to the relationship of an input of a method and the output of that method, such as occurs when the derivation is the calculation of a formula.

The term "parameter" herein refers to a numerical value that characterizes a quantitative data set and/or a numerical relationship between quantitative data sets. For example, a correlation (or function of a correlation) between the number of sequence reads mapped to a chromosome and the length of the chromosome to which the reads are mapped, is a parameter.

The term "score" as used herein refers to a numerical value or other representation which is linked or based on a specific feature, e.g. the number of reads or read counts of a certain sequence present in a sample. The term "first score" is used herein to refer to a numerical value or other representation linked to the target chromosome or chromosomal segment. Another example of a score may be represented by a Z score that quantifies how much the number of reads of a certain sequence differs from the number of reads that were obtained from the same sequence in a set of reference samples. It is known to a person skilled in the art how such a Z score can be calculated.

The term "cutoff value" or "threshold" as used herein means a numerical value or other representation whose value is used to arbitrate between two or more states (e.g. diseased and non-diseased) of classification for a biological sample. For example, if a parameter is greater than the cutoff value, a first classification of the quantitative data is made (e.g. diseased state); or if the parameter is less than the cutoff value, a different classification of the quantitative data is made (e.g. non-diseased state).

The term "imbalance" as used herein means any significant deviation as defined by at least one cutoff value in a quantity of the clinically relevant nucleic acid sequence from a reference quantity. For example, the reference quantity could be a ratio of 3/5, and thus an imbalance would occur if the measured ratio is 1: 1.

The term "random sequencing" as used herein refers to sequencing whereby the nucleic acid fragments sequenced have not been specifically identified or targeted before the sequencing procedure. Sequence-specific primers to target specific gene loci are not required. The pools of nucleic acids sequenced vary from sample to sample and even from analysis to analysis for the same sample. The identities of the sequenced nucleic acids are only revealed from the sequencing output generated. In some embodiments of the present teachings, the random sequencing may be preceded by procedures to enrich a biological sample with particular populations of nucleic acid molecules sharing certain common features. In one embodiment, each of the DNA fragments in the biological sample have an equal probability of being sequenced.

The term "fraction of the human genome" or "portion of the human genome" as used herein refers to less than 100% of the nucleotide sequences in the human genome which comprises of some 3 billion basepairs of nucleotides. In the context of sequencing, it refers to less than 1-fold coverage of the nucleotide sequences in the human genome. The term may be expressed as a percentage or absolute number of nucleotides/basepairs. As an example of use, the term may be used to refer to the actual amount of sequencing performed. Embodiments may determine the required minimal value for the sequenced fraction of the human genome to obtain an accurate diagnosis. As another example of use, the term may refer to the amount of sequenced data used for deriving a parameter or amount for disease classification.

The term "summary statistics" as used herein is to be understood as a statistical term, and is to be understood as an indication of the extend of a distribution of values or scores, or as in indication of the score/value present in the middle of the distribution. This can be e.g. a mean or median or standard deviation (StDev) or median absolute deviation (mad) or mean absolute deviation of a collection of scores.

The term "fetal fraction" as used herein refers to the fraction of fetal nucleic acids present in a sample comprising fetal and maternal nucleic acids.

The term "copy number variation" or "CNV" herein refers to variation in the number of copies of a nucleic acid sequence that is a few base-pairs (bp) or larger present in a test sample in comparison with the copy number of the nucleic acid sequence present in a qualified or reference sample. A "copy number variant" refers to the few bp or larger sequence of nucleic acid in which copy-number differences are found by comparison of a sequence of interest in test sample with that present in a qualified sample. Copy number variants/variations include deletions, including microdeletions, insertions, including microinsertions, duplications, multiplications. CNVs encompass chromosomal aneuploidies and partial aneuploidies.

The term "aneuploidy" herein refers to an imbalance of genetic material caused by a loss or gain of a whole chromosome, or part of a chromosome. Aneuploidy refers to both chromosomal as well as sub-chromosomal imbalances, such as, but not limiting to deletions, microdeletions, insertions, microinsertions, copy number variations, duplications. Copy number variations may vary in size in the range of a few bp to multiple Mb, or in particular cases from 1 kb to multiple Mb. Large subchromosomal abnormalities that span a region of tens of MBs and/or correspond to a significant portion of a chromosome arm, can also be referred to as segmental aneuploidies.

The term "chromosomal aneuploidy" herein refers to an imbalance of genetic material caused by a loss or gain of a whole chromosome, and includes germline aneuploidy and mosaic aneuploidy.

The term "partial aneuploidy" herein refers to an imbalance of genetic material caused by a loss or gain of a part of a chromosome e.g. partial monosomy and partial trisomy, and encompasses imbalances resulting from translocations, deletions and insertions.

The terms "polymorphism, polymorphic target nucleic acid", "polymorphic sequence", "polymorphic target nucleic acid sequence" and "polymorphic nucleic acid" are used interchangeably herein to refer to a nucleic acid sequence that contains one or more polymorphic sites.

The term "polymorphic site" herein refers to a single nucleotide polymorphism (SNP), a small-scale multi-base deletion or insertion, a Multi-Nucleotide Polymorphism (MNP) or a Short Tandem Repeat (STR) or a CNV (copy number variation).

The term "plurality" is used herein in reference to a number of nucleic acid molecules or sequence tags or reads that is sufficient to identify significant differences in copy number variations (e.g. chromosome doses) in test samples and qualified samples using the methods of the present teachings. In some embodiments, at least about $3 \times 10E6$ sequence tags, at least about $5 \times 10E6$ sequence tags, at least about $8 \times 10E6$ sequence tags, at least about $10 \times 10E6$ sequence tags, at least about $15 \times 10E6$ sequence tags, at least about $20 \times 10E6$ sequence tags, at least about $30 \times 10E6$ sequence tags, at least about $40 \times 10E6$ sequence tags, or at least about $50 \times 10E6$ sequence tags are obtained for each test sample. Each sequence tag can be a single sequence read of 20 to 400 bp, or a couple of 2 paired-end sequence reads of each 20 to 400 bp.

The terms "polynucleotide", "nucleic acid" and "nucleic acid molecules" are used interchangeably and refer to a covalently linked sequence of nucleotides (i.e., ribonucleotides for RNA and deoxyribonucleotides for DNA) in which the 3' position of the pentose of one nucleotide is joined by a phosphodiester group to the 5' position of the pentose of the next, include sequences of any form of nucleic acid, including, but not limited to RNA and DNA molecules. The term "polynucleotide" includes, without limitation, single- and double-stranded polynucleotide.

The term "portion" when used in reference to the amount of sequence information of fetal and maternal nucleic acid molecules in a biological sample herein refers to the amount of sequence information of fetal and maternal nucleic acid molecules in a biological sample that in sum amount to less than the sequence information of <1 human genome.

The term "test sample" herein refers to a sample comprising a mixture of nucleic acids comprising at least one nucleic acid sequence whose copy number is suspected of having undergone variation or at least one nucleic acid sequence for which it is desired to determine whether a copy number variation exists. Nucleic acids present in a test sample are referred to as test nucleic acids or target nucleic acids or target chromosomes or target chromosomal segments.

The term "reference sample" herein refers to a sample comprising a mixture of nucleic acids from which the sequencing data are used along with the test sample sequencing data to calculate scores and parameters as described in the present teachings. Though not necessary, a reference sample is preferably normal (e.g. not aneuploid) for the sequence of interest. So preferably, a reference sample may be a qualified sample that does not carry an aneuploidy and that can be used for identifying the presence of e.g. an aneuploidy like trisomy 21 or any other aneuploidy in a test sample.

The term "reference set" comprises a plurality of "reference samples".

The term "enrich" herein refers to the process of amplifying certain target or selected nucleic acids contained in a portion of a maternal sample.

The term "sequence of interest" herein refers to a nucleic acid sequence that is associated with a difference in sequence representation in healthy versus diseased individuals. A sequence of interest can be a sequence on a chromosome that is misrepresented i.e. over- or under-represented, in a disease or genetic condition. A sequence of interest may also be a portion of a chromosome, or a chromosome. For example, a sequence of interest can be a chromosome that is over-represented in an aneuploidy condition. Sequences of interest include sequences that are over- or under-represented in the total population, or a subpopulation of cells of a subject

The term "plurality of polymorphic target nucleic acids" herein refers to a number of nucleic acid sequences each comprising at least one polymorphic site e.g. one SNP or CNV, such that at least 1, 2, 3, 4, 5, 6, 7, 8, 9, 10, 15, 20, 25, 30, 40 or more different polymorphic sites are targeted, selected, amplified and/or sequenced.

The term "group of chromosomes" herein refers to two or more chromosomes. The term "collection" refers to a set of chromosomes or chromosomal segments, but may also refer to a set of values or scores derived from a corresponding set of chromosomes or chromosomal segments.

The term "read" refers to an experimentally obtained DNA sequence of sufficient length (e.g., at least about 20 bp) that can be used to identify a larger sequence or region, e.g. that can be aligned and specifically assigned to a chromosome location or genomic region or gene. The terms 'read' and 'sequences' may be sued interchangeably throughout the draft.

The term "read count" refers to the number of reads retrieved from a sample that are mapped to a reference genome or a portion of said reference genome (bin).

The term "bin" of a genome is to be understood as a representation of a segment of the genome. A genome can be divided in several bins, either of a fixed or predetermined size or a variable size. A possible fixed bin size can be e.g. 10 kB, 20 kB, 30 kB, 40 kB, 50 kB, 60 kB, 70 kB, etc. in which kB stands for kilobasepairs, a unit that corresponds to 1000 basepairs.

The term "window" may comprise a plurality of bins and / or represent a region of a genome.

The terms "aligned", "alignment", "mapped" or "aligning", "mapping" refer to one or more sequences that are identified as a match in terms of the order of their nucleic acid molecules to a known sequence from a reference genome. Such alignment can be done manually or by a computer algorithm, examples including the Efficient Local Alignment of Nucleotide Data (ELAND) computer program distributed as part of the Illumina Genomics Analysts pipeline. The matching of a sequence read in aligning can be a 100% sequence match or less than 100% (non-perfect match).

The term "reference genome" as used herein may refer to a digital or previously identified nucleic acid sequence database, assembled as a representative example of a species or subject. Reference genomes may be assembled from the nucleic acid sequences from multiple subjects, sample or organisms and does not necessarily represent the nucleic acid makeup of a single person. Reference genomes may be used to for mapping of sequencing reads from a sample to chromosomal positions.

The term "clinically-relevant sequence" herein refers to a nucleic acid sequence that is known or is suspected to be associated or implicated with a genetic or disease condition. Determining the absence or presence of a clinically-relevant sequence can be useful in determining a diagnosis or confirming a diagnosis of a medical condition, or providing a prognosis for the development of a disease.

The term "derived" when used in the context of a nucleic acid or a mixture of nucleic acids, herein refers to the means whereby the nucleic acid(s) are obtained from the source from which they originate. For example, in one embodiment, a mixture of nucleic acids that is derived from two different genomes means that the nucleic acids e.g. cell-free DNA, were naturally released by cells through naturally occurring processes such as necrosis or apoptosis, or through lysis of the cells due to improper storage or transport conditions

The term "maternal sample" herein refers to a biological sample obtained from a pregnant subject or organism (e.g. a woman).

The term "biological fluid" herein refers to a liquid taken from a biological source and includes, for example, blood, serum, plasma, sputum, lavage fluid, cerebrospinal fluid, urine, semen, sweat, tears, saliva, blastocoel fluid and the like. It also refers to the medium in which biological samples can be grown, like in vitro culture medium in which cells, tissue or embryo can be cultured. As used herein, the terms "blood," "plasma" and "serum" expressly encompass fractions or processed portions thereof. Similarly, where a sample is taken from a biopsy, swab, smear, etc., the "sample" expressly encompasses a processed fraction or portion derived from the biopsy, swab, smear, etc.

The terms "maternal nucleic acids" and "fetal nucleic acids" herein refer to the nucleic acids of a pregnant female subject and the nucleic acids of the fetus being carried by the pregnant female, respectively. As explained before, "fetal nucleic acids" and "placental nucleic acids" are often used to refer to the same type of nucleic acids, though biological differences may exist between the two types of nucleic acids.

The term "corresponding to" herein refers to a nucleic acid sequence e.g. a gene or a chromosome, that is present in the genome of different subjects, and which does not necessarily have the same sequence in all genomes, but serves to provide the identity rather than the genetic information of a sequence of interest e.g. a gene or chromosome.

The term "substantially cell free" herein refers to preparations of the desired sample from which components that are normally associated with it are removed. For example, a plasma sample is rendered essentially cell free by removing blood cells e.g. white blood cells, which are normally associated with it. In some embodiments, substantially free samples are processed to remove cells that would otherwise contribute to the genetic material that is to be tested for an aneuploidy.

As used herein the term "chromosome" refers to the heredity-bearing gene carrier of a living cell which is derived from chromatin and which comprises DNA and protein components (especially histones). The conventional internationally recognized individual human genome chromosome numbering system is employed herein. The term "chromosomal segments" is to be understood as a part of a chromosome. Said segment may refer to a bin, window or specific region within a chromosome, e.g. known to comprise for instance deletions or insertions or copy number variations.

As used herein, the term "polynucleotide length" refers to the number of nucleic acid molecules (nucleotides) in a sequence or in a region of a reference genome. The term "chromosome length" refers to the known length of the chromosome given in base pairs.

The term "subject" herein refers to a human subject as well as a non-human subject such as a mammal, an invertebrate, a vertebrate, a fungus, a yeast, a bacteria, and a virus. Although the examples herein concern human genomes and the language is primarily directed to human concerns, the concept of the present teachings is applicable to genomes from any plant or animal, and is useful in the fields of veterinary medicine, animal sciences, research laboratories and such.

The term "condition" herein refers to "medical condition" as a broad term that includes all diseases and disorders, but can include injuries and normal health situations, such as pregnancy, that might affect a person's health, benefit from medical assistance, or have implications for medical treatments. Said condition may be linked to the presence of a tumor.

The term "attribute" is to be understood as a property or value of an object or element. This can e.g. be a certain correction factor that is used to correct the read count for a particular polymorphism. An attribute may have been experimentally defined using a set of samples.

## DETAILED DESCRIPTION

The present teachings pertains in a first aspect to a method for determining the presence or absence of a fetal chromosomal aneuploidy in a pregnant female. This determination may be done by the calculation of one or more parameters linked to chromosomal data obtained from a biological sample. Also provided is a computer readable medium encoded with a plurality of instructions for controlling a computer system to perform the methods.

In a second aspect, the present teachings describe a methodology for the determination of the fetal fraction in a sample. In particular, the method enables the determination of the fraction of cell-free DNA (cfDNA) contributed by a fetus to the mixture of fetal and maternal cfDNA in a maternal sample e.g. a plasma sample. In a preferred embodiment, the present teachings allow both the determination of the presence or absence of a fetal chromosomal aneuploidy in a pregnant female and the determination of the fetal fraction, independent from the gender of the fetus.

In a third aspect, the present teachings describe a methodology for determining whether a subject has tumor-derived cell-free DNA in his or her peripheral blood, for confirming a cancer diagnosis, for aiding in the classification of a cancer, for assessing the treatment response, for monitoring the subject, for identifying the presence of a cancer and/or an increased risk of a cancer in a subject, said subject is preferably a mammal.

In one aspect, read counts are determined from the sequencing of nucleic acid molecules in a (maternal) sample, such as urine, plasma, serum, blastocoel fluid and other suitable biological samples. Nucleic acid molecules of the biological sample are randomly sequenced, such that a fraction of the genome is sequenced. One or more cutoff values are chosen for determining whether a change compared to a reference quantity exists (i.e. an imbalance), for example, with regards to the ratio of amounts of two chromosomal regions (or sets of regions).

The change detected in the reference quantity may be any deviation (upwards or downwards) in the relation of the clinically-relevant nucleic acid sequence or target chromosome or chromosomal segment to the other non-clinically-relevant sequences. Thus, the reference state may be any ratio, correlation or other quantity (e.g. other than a 1-1 correspondence), and a measured state signifying a change may be any ratio, correlation, or other quantity that differs from the reference quantity as determined by the one or more cutoff values.

The clinically relevant chromosomal region (also called a clinically relevant nucleic acid sequence or target chromosome or chromosomal segment) and the background nucleic acid sequence may come from a first type of cells and from one or more second types of cells. For example, fetal nucleic acid sequences originating from fetal/placental cells are present in a biological sample, such as maternal plasma, which contains a background of maternal nucleic acid sequences originating from maternal cells. Note the percentage of fetal sequences in a sample may be determined by any fetal-derived loci and not limited to measuring the clinically-relevant nucleic acid sequences.

## A. Fetal aneuploidy

### I. General method for evaluating a fetal aneuploidy

The present teachings describe a methodology for detecting the presence or absence of a fetal chromosomal aneuploidy and/or the determination of the fetal fraction present in a biological sample.

In a first aspect, the method for detecting the presence or absence of a fetal chromosomal aneuploidy is based on the determination of a parameter from the nucleic acid content of a biological sample. The biological sample may be plasma, urine, serum, blastocoel fluid or any other suitable sample. The sample contains nucleic acid molecules from the fetus and the pregnant female. For example, the nucleic acid molecules may be fragments from chromosomes.

At least a portion of a plurality of the nucleic acid molecules contained in the biological sample is randomly sequenced to obtain a number of sequences. The portion sequenced represents a fraction of the human genome and may be isolated from the sample by conventional means (e.g. cell-free DNA extraction and preparation of a NGS library). In one embodiment, the nucleic acid molecules are fragments of respective chromosomes. One end (e.g. 50 basepairs (bp)), both ends, or the entire fragment may be sequenced. A subset of the nucleic acid molecules in the sample may be sequenced, and this subset is randomly chosen, as will be described in more detail later.

In one embodiment, the random sequencing is done using massively parallel sequencing. Massively parallel sequencing, such as that achievable on the HiSeq2000, HiSeq2500, HiSeq3000, HiSeq4000, HiSeq X, MiSeq, MiSeqDx, NextSeq500, NextSeq550 flowcell, the 454 platform (Roche), Illumina Genome Analyzer (or Solexa platform) or SOLiD System (Applied Biosystems) or PGM or Proton platform (IonTorrent) or GeneRead (Qiagen) or the Helicos True Single Molecule DNA sequencing technology, the single molecule, real-time (SMRT™) technology of Pacific Biosciences, and nanopore sequencing as in MinION, PromethION, GridION (Oxford Nanopore technologies), allow the sequencing of many nucleic acid molecules isolated from a specimen at high orders of multiplexing in a parallel fashion. Each of these platforms sequences clonally expanded or even non-amplified single molecules of nucleic acid fragments. Clonal expansion can be achieved via bridge amplification, emulsion PCR, or Wildfire technology.

As a high number of sequencing reads, in the order of hundred thousand to millions or even possibly hundreds of millions or billions, are generated from each sample in each run, the resultant sequenced reads form a representative profile of the mix of nucleic acid species in the original specimen. For example, the haplotype, transcriptome and methylation profiles of the sequenced reads resemble those of the original specimen. Due to the large sampling of sequences from each specimen, the number of identical sequences, such as that generated from the sequencing of a nucleic acid pool at several folds of coverage or high redundancy, is also a good quantitative representation of the count of a particular nucleic acid species or locus in the original sample.

Based on the sequencing (e.g. data from the sequencing), a first score of a target chromosome or chromosomal segment (e.g. the clinically relevant chromosome) is determined. The first score is determined from sequences identified as originating from (i.e. aligning) to the target chromosome or chromosomal segment. For example, a bioinformatics procedure may then be used to locate each of these DNA sequences to the human genome or a reference genome. It is possible that a proportion of such sequences will be discarded from subsequent analysis because they are present in the repeat regions of the human genome, or in regions subjected to inter-individual variations, e.g. copy number variations. A score of the target chromosome or chromosomal segment and of one or more other chromosomes may thus be determined.

Based on the sequencing, a collection of scores of one or more chromosomes or chromosomal segments is determined from sequences identified as originating from (i.e. aligning to) a set of one of more chromosomes. In one embodiment, said set contains all of the other chromosomes besides the first one (i.e. the one being tested). In another embodiment, said set contains just a single other chromosome. In a most preferred embodiment, said set contains chromosomes or chromosomal segments and includes the target chromosome or chromosomal segment.

There are a number of ways of determining a score. By preference, said score is based on the read counts obtained from sequencing. Said read counts can include, but are not limiting to counting the number of sequenced reads, the number of sequenced nucleotides (basepairs) or the accumulated lengths of sequenced nucleotides (basepairs) originating from particular chromosome(s) or chromosomal segments such as bins or windows or clinically-relevant chromosome portions.

Rules may be imposed on the results of the sequencing to determine what gets counted. In one aspect, a read count may be obtained based on a proportion of the sequenced output. For example, sequencing output corresponding to nucleic acid fragments of a specified size range could be selected.

In one embodiment, said score is the raw read count for a certain chromosome or chromosomal segment.

In a preferred embodiment, said read counts are subjected to mathematical functions or operations in order to derive said score of said read counts. Such operations include but are not limiting to statistical operations, regression models standard calculations (sum, subtraction, multiplying and division), whereby said standard calculations are preferably based on one or more obtained read counts.

In a preferred embodiment, said first score is a normalized value derived from the read counts or mathematically modified read counts. In a further preferred embodiment, said score is a Z score or standard score relating to the read counts of a certain chromosome, chromosomal segment, or the mathematically amended counts thereof, in which the Z score quantifies how much the number of reads of a certain sequence differs from the number of reads that were obtained from the same sequence in a set of reference samples. It is known to a person skilled in the art how such a Z score can be calculated.

In a preferred embodiment, a parameter is determined based on a first score (corresponding to the target chromosome or chromosomal segment) and a collection of scores. The parameter preferably represents a relative score between the first score and a summary statistic of the collection of scores. The parameter may be, for example, a ratio or correlation of the first score to a summary statistic of the collection of scores. In one aspect, each score could be an argument to a function or separate functions, where a ratio or correlation may be then taken of these separate functions.

In a preferred embodiment, the parameter may be obtained by a ratio or correlation between:

- a first function whereby the first score and the collection of scores are the arguments;
- a second function whereby the collection of scores is the argument.

In a more preferred embodiment, said first function is defined as a difference, preferably the difference between the first score and a summary statistic of the collection of scores, whereby said summary statistic is preferably selected from the mean, median, standard deviation or median absolute deviation (mad) or mean absolute deviation.

In a further preferred embodiment, said second function is defined as a variability summary statistic of the collection of scores, whereby said summary statistic may be an average or a measure of variability and is preferably selected from the mean, median, standard deviation or median absolute deviation (mad) or mean absolute deviation.

Typically, a suitable embodiment according to the present teachings involves the following steps (after having obtained DNA sequences from a random, low-coverage sequencing process on a biological sample).

- aligning sequences to a reference genome;
- obtaining the read counts per chromosome or chromosomal segment;
- normalizing the number of reads or a derivative thereof towards a normalized number of reads;
- obtaining a first score derived from said normalized number of reads and a collection of scores derived from said normalized reads, whereby said first score is derived from the normalized read counts for a target chromosome or chromosomal segment, and said collection of scores is a set of scores derived from the normalized number of reads that were obtained from a set of chromosomes or chromosome segments that include the target chromosomal segment or chromosome;
- calculating a parameter from said scores, whereby said parameter represents a ratio or correlation between said first score and a summary statistic of said collection of scores, whereby the first function of said ratio or correlation is defined as a difference between the first score and a summary statistic of said collection of scores; and whereby the second function of said ratio or correlation is defined as a summary statistic of said collection of scores.

Preferably, said sequences are obtained by low coverage sequencing.

Said normalization preferably occurs on the basis of a set of reference samples, whereby said reference samples are preferably, though not necessary, euploid or essentially euploid for the chromosome or chromosomal segment that corresponds to the target chromosome or chromosomal segment (i.e. the majority of the chromosomes or chromosomal segments in the reference samples that correspond to the target chromosome or chromosomal segment in the test sample are euploid). Such reference set have various sample sizes. A possible sample size can be e.g. 100 samples, such as 50 male and 50 female samples. It will be understood by a skilled person that the reference set can be freely chosen by the user.

By preference, said number of reads is recalibrated to correct for GC content and/or total number of reads obtained from said sample.

By taking into account a set of scores derived of reads of chromosomes or chromosomal segments that include the target chromosome or chromosomal segment, a more robust, sensitive and reliable parameter is obtained as compared to known prior art methods. Other than the known prior art methods, there is no need to make an assumption on the ploidy state of any of the chromosomes in the test sample. In fact, by defining a parameter according to the present teachings, the parameter for the chromosome or region to be analyzed clearly stands out (i.e. is strongly increased/decreased), and does not disappear in the noise (i.e. only moderately or not increased/decreased). Moreover, for screening purpose, sensitivity is key, as it is important to have a reliable and trustworthy result, thereby minimizing the amount of false negatives. In fact, for screening purposes, it may be more important to have high superiority as compared to specificity.

The parameter according to the present teachingsallows robustly detecting and automatically classifying chromosomes, even in noisy data. By taking into account a collection of chromosomes or segments, including the target chromosome or segment, i.e. the majority of information that is available in the dataset, most of the available information is used, coming to a more adequate assessment. For instance, if one would remove e.g. chromosome 1 (the largest chromosome, 7.9% of the genome), a large amount of data would be removed that would not be taken into account, thereby causing a distortion in the assessment.

In particular, the present teachingsis very useful in situations whereby a low number of reads or noisy data is obtained. The inventors found that in the latter situations, the parameter according to the present teachings performed superior compared to other methodologies.

In a preferred embodiment, said scores are obtained on the basis of the genomic representation of the target chromosome or chromosomal segment (or a region thereof) and the genomic representation of all autosomes or chromosomes, thereby including the target chromosome or chromosomal segment.

The parameter is compared to one or more cutoff values. The cutoff values may be determined from any number of suitable ways. Such ways include Bayesian-type likelihood method, sequential probability ratio testing (SPRT), false discovery, confidence interval, receiver operating characteristic (ROC). In a more preferred embodiment, said cutoff value is based on statistical considerations or is empirically determined by testing biological samples. The cutoff value can be validated by means of test data or a validation set and can, if necessary, be amended whenever more data is available.

It is possible that in some variants of the procedure, the cutoff value would be adjusted in accordance with information on the fraction of the cell-free fetal DNA in the maternal plasma sample (also termed fetal fraction or abbreviated as ff or f). In another embodiment, said fetal fraction may serve as an internal control of the quality of the sample. The value of f can be determined from the sequencing dataset in different ways (dependent on the gender of the fetus, or independent from the gender of the fetus), as will be explained further on.

Based on the comparison, a classification of whether a fetal chromosomal aneuploidy exists for the target chromosome or chromosomal section may be determined. In one embodiment, the classification is a definitive yes or no. In another embodiment, a classification may be unclassifiable or uncertain. In yet another embodiment, the classification may be a risk score that is to be interpreted at a later date, for example, by a doctor.

In another embodiment, the comparison of the parameter with one or more cutoff values will result in assessment of the sensitivity of a previous diagnosis. In another or further embodiment, said calculation of a parameter according to the present teachings lead to a verification or improvement of the accuracy of ploidy and/or aneuploidy calls.

In a further preferred method, secondary parameters from the read counts are calculated, which serve as an additional internal control for the usefulness of the parameter, the extend of the aneuploidy (if identified) and/or an indication for the reliability of the parameter, the biological sample or the sequences obtained thereof and thus the final assessment. The value for said secondary parameters can be e.g. a measure or prerequisite of the presence of said aneuploidy and/or a measure of quality of the sample.

In one embodiment, such secondary parameter is calculated as the median of the Z-distribution of the read counts or a derivative thereof, for a target chromosome or a target chromosomal segment measured per bin or an aggregation of bins (i.e. windows). The latter secondary parameters allow assessing if the majority (more than 50%) of the windows in a chromosome is increased or decreased. The latter allows the detection of chromosomal and large subchromosomal aneuploidies. When less than 50% of the windows are affected, the secondary parameters will not be affected (e.g. for smaller CNVs).

In another embodiment, said secondary parameters may be calculated as the median of the absolute value of the Z-scores for the read counts or a derivative thereof, of the remaining chromosomes (that is a collection of chromosomes or segments that exclude the target chromosome or chromosomal segment).

The latter secondary parameters allow the detection of a.o. the presence of technical or biological instabilities and to discriminate these from maternal CNVs. If less than the windows of the other or all chromosomes are affected, this secondary parameter will not be affected. If more than 50% of the windows is affected, this will be derivable from said secondary parameters.

In another embodiment, the present teachings also provide for a quality score (QS). QS allows to assess the overall variation across the genome. A low QS is an indication of a good sample processing and a low level of technical and biological noise. An increase in the QS can indicate two possible reasons. Either an error occurred during the sample processing. In general, the user will be requested to retrieve and test a new biological sample. This is typical for moderately increased QS scores. A strongly increased QS could be an indication of a highly aneuploid sample and the user will be encouraged to do a confirmatory test. Preferably, said QS is determined by calculating the standard deviations of all Z scores for chromosomes or chromosomal segments and optionally by removing the outliers thereof (i.e. the highest and lowest Z scores in this collection).

As an alternative or additional embodiment, the determination of the fetal fraction (see below) also serves as an internal quality control of the sample and the sequences obtained thereof. The quality of a sample may be hampered after retrieval, e.g. by inappropriate conditions during collection, transport or storage. The latter may have an effect on the cell free DNA in the sample, for instance due to rupture of (maternal) white blood cells. As such, the main pool of free floating DNA will become even more enriched for maternal DNA, thereby reducing the percentage of the fetal fraction compared to the total cell free DNA content in the sample. By preference, said fetal fraction will be determined by at least one of the methods described below.

In an embodiment of the present teachings, the parameter will be sufficient to discriminate between the presence and/or absence of an aneuploidy. In a more preferred embodiment of the present teachings; both the parameter as the secondary parameters will be used to come to a decision with regard to the presence or absence of an aneuploidy. Preferably, also said secondary parameters will be compared to predefined threshold values.

By preference, the methodology according to the present teachings is particularly suitable for analyzing aneuploidies linked to segments or deletions given in Table 1, which contains a not-limiting list of chromosome abnormalities that can be potentially identified by methods and kits described herein. In a another or further embodiment, said target chromosomal segment is selected from a bin or a window derived from chromosome X, Y, 6, 7, 8, 13, 14, 15, 16, 18, 21 and/or 22.

In a further or other embodiment, said target chromosome is selected from chromosome X, Y, 6, 7, 8, 13, 14, 15, 16, 18, 21 and/or 22.

**Table 1**

| Chromosome | Abnormality | Disease Association |
|---|---|---|
| X | XO | Turner's Syndrome |
| Y | XXY | Klinefelter syndrome |
| | XYY | Double Y syndrome |
| | XXX | Trisomy X syndrome |
| | XXXX | Four X syndrome |
| | Xp21 deletion | Duchenne's/Becker syndrome, congenital adrenal hypoplasia, chronic granulomatous disease |
| | Xp22 deletion | Steroid sulfatase deficiency |
| | Xp26 deletion | X-linked lymph proliferative disease |
| 1 | 1p Monosomy, trisomy | |
| | 1p36 | 1p36 deletion syndrome |
| | 1q21.1 | 121.1 deletion syndrome; distal 1q21 deletion sydnrome |
| 2 | Monosomy, trisomy 2q | Growth retardation, developmental and mental delay, and minor physical abnormalities |
| | 2p15-16.1 | 2p15-16.1 deletion syndrome |
| | 2q23.1 | 2q23.1 deletion syndrome |

(continued)

| Chromosome | Abnormality | Disease Association |
|---|---|---|
| | 2q37 | 2q37 deletion syndrome |
| 3 | Monosomy, trisomy | |
| | 3p | 3p deletion syndrome |
| | 3q29 | 3q29 deletion syndrome |
| 4 | Monosomy, trisomy | |
| | 4p- | Wolf-Hirschhorn syndrome |
| 5 | 5p | Cri du chat; Lejeune syndrome |
| | 5q Monosomy, trisomy | Myelodysplastic syndrome |
| | 5q35 | 5q35 deletion syndrome |
| 6 | Monosomy, trisomy | |
| | 6p25 | 6p25 deletion syndrome |
| 7 | 7q11.23 deletion | William's syndrome |
| | Monosomy, trisomy | Monosomy 7 syndrome of childhood; myelodysplastic syndrome |
| 8 | 8q24.1 deletion | Langer-Giedion syndrome |
| | 8q22.1 | Nablus mask-like facial syndrome |
| | Monosomy, trisomy | Myelodysplastic syndrome; Warkany syndrome; |
| 9 | Monosomy 9p | Alfi's syndrome |
| | Monosomy 9p, partial trisomy 9p | Rethore syndrome |
| | trisomy | Complete trisomy 9 syndrome; mosaic trisomy 9 syndrome |
| | 9p22 | 9p22 deletion syndrome |
| | 9q34.3 | 9q34.3 deletion syndrome |
| 10 | Monosomy, trisomy | ALL or ANLL |
| | 10p14-p13 | DiGeorge's syndrome type II |
| 11 | 11p- | Aniridia; Wilms tumor |
| | 11p13 | Wagr syndrome |
| | 11p11.2 | Potocki Shaffer syndrome |
| | 11p15 | Beckwith-Wiedemann syndrome |
| | 11q- | Jacobsen syndrome |
| | Monosomy, trisomy | |
| 12 | Monosomy, trisomy | |
| 13 | 13q- | 13q-syndrome; Orbeli syndrome |
| | 13q14 deletion | |
| | Monosomy, trisomy | Patau's syndrome |
| 14 | Monosomy, trisomy | |
| 15 | 15q11-q13 deletion, monosomy | Prader-Willi, Angelman's syndrome |
| | Trisomy | |

(continued)

| Chromosome | Abnormality | Disease Association |
|---|---|---|
| 16 | 16q13.3 deletion | Rubenstein- Taybi |
| | Monosomy, trisomy | |
| 17 | 17p- | 17p syndrome |
| | 17q11.2 deletion | Smith-Magenis |
| | 17q13.3 | Miller-Dieker |
| | Monosomy, trisomy | |
| | 17p11.2-12 trisomy | Charcot-Marie Tooth Syndrome type 1; HNPP |
| 18 | 18p- | 18p partial monosomy syndrome or Grouchy Lamy Thieffry syndrome |
| | Monosomy, trisomy | Edwards Syndrome |
| 19 | Monosomy, trisomy | |
| 20 | 20p- | Trisomy 20p syndrome |
| | 20p11.2-12 deletion | Alagille |
| | 20q- | |
| | Monosomy, trisomy | |
| 21 | Monosomy, trisomy | Down's syndrome |
| 22 | 22q11.2 deletion | DiGeorge's syndrome, velocardiofacial syndrome, conotruncal anomaly face syndrome, autosomal dominant Opitz G/BBB syndrome, Caylor cardiofacial syndrome |
| | Monosomy, trisomy | Complete trisomy 22 syndrome |

II Sequencing, aligning and correction

As mentioned above, only a fraction of the genome is sequenced. In one aspect, even when a pool of nucleic acids in a specimen is sequenced at <100% genomic coverage instead of at several folds of coverage, and among the proportion of sequenced nucleic acid molecules, most of each nucleic acid species is not sequenced or sequenced only once.

This is contrasted from situations where targeted enrichment is performed of a subset of the genome prior to the sequencing reaction, followed by high-coverage sequencing of that subset.

In one embodiment, said sequences are obtained by next generation sequencing. In a further preferred embodiment, said sequencing method is a low coverage, random sequencing method.

In one embodiment, massive parallel short-read sequencing is used. Short sequence tags or reads are generated, e.g. from a certain length between 20bp and 400bp. Alternatively, paired end sequencing could be performed.

In an embodiment, a pre-processing step is available for pre-processing the obtained reads. Such pre-processing option allows filtering low quality reads, thereby preventing them from being mapped. Mapping of low quality reads can require prolonged computer processing capacity, can be incorrect and risks increasing the technical noise in the data, thereby obtaining a less accurate parameter. Such pre-processing is especially valuable when using next-generation sequencing data, which has an overall lower quality or any other circumstance which is linked to an overall lower quality of the reads.

The generated reads can subsequently be aligned to one or more human reference genome sequences. Preferably, the number of aligned reads are counted and/or sorted according to their chromosomal location.

An additional clean-up protocol can be performed, whereby deduplication is performed, e.g. with Picard tools, retaining only uniquely mapped reads. Reads with mismatches and gaps can be removed. Reads that map to blacklisted regions can be excluded. Such blacklisted regions may be taken from a pre-defined list of e.g. common CNVs, collapsed repeats, DAC blacklisted regions as identified in the ENCODE project (i.e. a set of regions in the human genome that have anomalous, unstructured, high signal/read counts in NGS experiments independent of cell line and type of experiment) and the undefined portion of the reference genome. In one embodiment, blacklisted regions are provided to the user. In another embodiment, the user may use or define his or her own set of blacklisted regions.

In a further embodiment, chromosomes are divided into regions of a predefined length, generally referred to as bins. In an embodiment, the bin size is a predefined size provided to the user. In another embodiment, said bin size can be defined by a user, can be uniformly for all chromosomes, can be a specific bin size per chromosome or can vary according to the obtained sequence data. Change of the bin size can have an effect on the final parameter to be defined, either by improving the sensitivity (typically obtained by decreasing the bin size, often at the cost of the specificity) or by improving the specificity (generally by increasing the bin size, often at the cost of the sensitivity). A possible bin size which provides an acceptable specificity and sensitivity is 50 kb.

In a further step, the aligned and filtered reads within a bin are counted, in order to obtain read counts.

The obtained read counts can be corrected for the GC count for the bin. GC bias is known to aggravate genome assembly. Various GC corrections are known in the art (e.g. Benjamini et al., Nucleic Acid Research 2012). In a preferred embodiment, said GC correction will be a LOESS regression. In one embodiment, a user of the methodology according to the present teachingscan be provided with the choice of various possible GC corrections.

In a subsequent step, the genomic representation (GR) of read counts per bin is calculated. Such representation is preferably defined as a ratio or correlation between the GC-corrected read counts for a specific bin and the sum of all GC-corrected read counts.

In an embodiment, said GR is defined as follows:

$$GRi = \frac{GCi}{\sum_k GCk} \cdot 10^7$$

with k over all chromosomal bins

The factor $10^7$ (or 10E7) in the above formula is arbitrary defined, and can be any constant value.

In a final step, the obtained GR per bin are aggregated over a region, whereby said region may be a subregion (window) of a chromosome or the full chromosome. Said window may have a predefined or variable size, which can optionally be chosen by the user. A possible window could have a size of 5 MB or 100 adjacent bins of size 50 kb.

The GR aggregated for a chromosome can be defined by

$$GRi = \frac{\sum_{j \in (bins\ on\ chr)} GCi}{\sum_{k \in (all\ bins)GCk} GCk}$$

In a further embodiment, the genomic representation of a set of reference samples shall be calculated. Said set of reference samples (or also termed reference set) can be predefined or chosen by a user (e.g. selected from his/her own reference samples). By allowing the user the use of an own reference set, a user will be enabled to better capture the recurrent technical variation of his/her environment and its variables (e.g. different wet lab reagents or protocol, different NGS instrument or platform, etc.). In a preferred embodiment, said reference set comprises genomic information of 'healthy' samples that are expected or known to not contain (relevant) aneuploidies. The genomic representation (GR) of the reference set can be defined, either at the genome level and/or at a subregion (chromosome, chromosomal segment, window, or bin). Said reference set may be as small as comprising at least 3 samples, at least 5 samples, or at least between 5 and 25 samples in order to be workable.

Other single molecule sequencing strategies such as that by the Roche 454 platform, the Applied Biosystems SOLiD platform, the the Helicos True Single Molecule DNA sequencing technology, the single molecule, real-time (SMRT™) technology of Pacific Biosciences, and nanopore sequencing technologies like MinION, GridION or PromethION from Oxford Nanopore Technologies could similarly be used in this application.

III Determining scores, parameter and secondary parameters

From the alignments and the obtained read counts or a derivative thereof, optionally corrected for GC content and/or total number of reads obtained from said sample, scores are calculated which eventually lead to a parameter allowing the determination of the presence of an aneuploidy in a sample. Said scores are normalized values derived from the read counts or mathematically modified read counts, whereby normalization occurs in view of the reference set as defined by the user. As such, each score is obtained by means of a comparison with the reference set. It is important to note that the current methodology does not require training of the data or knowledge of the ground truth. The analysis according to the present teachings may use the nature of the reference set and does not require any personal choices or preferences set by the end user. Moreover, it can be readily implemented by a user without the need for access to proprietary databases.

The term first score is used to refer to score linked to the read count for a target chromosome or a chromosomal segment. A collection of scores is a set of scores derived from a set of normalized number of reads that may include the normalized number of reads of said target chromosomal segment or chromosome.

Preferably, said first score represents a Z score or standard score for a target chromosome or chromosomal segment. Preferably, said collection is derived from a set of Z scores obtained from a corresponding set of chromosomes or chromosomal segments that include said target chromosomal segment or chromosome.

In a most preferred embodiment, the first score and the collection of scores are calculated on the basis of the genomic representation of either a target chromosome or chromosomal segment, or all autosomes or chromosomes (or regions thereof) thereby including the target chromosome or chromosome segment.

Such scores can be calculated as follows:

$$Zi = \frac{GRi - \mu\, ref,i}{\sigma\, ref,i}$$

With i a window or a chromosome or a chromosome segment and ref referring to the reference set.

A summary statistic of said collection of scores can e.g. be calculated as the mean or median value of the individual scores. Another summary statistic of said collection of scores can be calculated as the standard deviation or median absolute deviation or mean absolute deviation of the individual scores.

Said parameter p will be calculated as a function of the first score and a derivative (e.g. summary statistic) of the collection of scores. In a preferred embodiment, said parameter will be a ratio or correlation between the first score corrected by the collection of scores (or a derivative thereof) and a derivative of said collection of scores.

In another embodiment, said parameter will be a ratio or correlation between the first score corrected by a summary statistic of a first collection of scores and a summary statistic of a different, second collection of scores, in which both collections of scores include the first score.

In a specifically preferred embodiment, said parameter p is a ratio or correlation between the first score, corrected by a summary statistic of said collection of scores, and a summary statistic of said collection of scores. Preferably, the summary statistic is selected from the mean, median, standard deviation, median absolute deviation or mean absolute deviation. In one embodiment, said both used summary statistics in the function are the same. In another, more preferred embodiment, said summary statistics of the collection of scores differ in the numerator and denominator.

Typically, a suitable embodiment according to the present teachings involve the following steps (after having obtained DNA sequences from a random sequencing process on a biological sample).

- aligning said obtained sequences to a reference genome;
- counting the number of reads on a set of chromosomal segments and/or chromosomes thereby obtaining read counts;
- normalizing said read counts or a derivative thereof into a normalized number of reads;
- obtaining a first score and a collection of scores of said normalized reads, whereby said first score is derived from the normalized reads for a target chromosome or chromosomal segment and said collection of scores is a set of scores derived from a corresponding set of chromosomes or chromosome segments that include said target chromosomal segment or chromosome;
- calculating a parameter p from said first score and said collection of scores, whereby said parameter represents a ratio or correlation between

  * said first score, corrected by a summary statistic of said collection of scores, and
  * a summary statistic of said collection of scores.

A possible parameter p can be calculated as follows:

$$Z\,\text{of}\,Z_i = \frac{Z_i - \underset{j=i,a,b,...}{\text{median}}(Z_j)}{\underset{j=i,a,b,...}{\text{sd}}(Z_j)}$$

Whereby Zi represents the first score and $Z_j$ the collection of scores and whereby i represents the target chromosome or chromosomal section, and whereby j represents a collection chromosomes or chromosomal segments i, a, b, .. that include said target chromosomal segment or chromosome i.

In another embodiment, said parameter p is calculated as

$$Z \text{ of } Z_i = \frac{Z_i - \operatorname*{mean}_{j=i,a,b,\dots}\left(Z_j\right)}{\operatorname*{mad}_{j=i,a,b,\dots}\left(Z_j\right)}$$

Whereby $Z_i$ represents the first score and $Z_j$ the collection of scores and whereby i represents the target chromosome or chromosomal section, and whereby j represents a collection of chromosomes or chromosomal segments i, a, b, .. that includes said target chromosomal segment or chromosome i.

In yet another, most preferred embodiment, said parameter p is calculated as

$$Z \text{ of } Z_i = \frac{Z_i - \operatorname*{median}_{j=i,a,b,\dots}\left(Z_j\right)}{\operatorname*{mad}_{j=i,a,b,\dots}\left(Z_j\right)}$$

Whereby $Z_i$ represents the first score and $Z_j$ the collection of second scores and whereby i represents the target chromosome or chromosomal section, and whereby j represents a collection of chromosomes or chromosomal segments i, a, b, .. that includes said target chromosomal segment or chromosome i.

Said MAD for a data set x_1,x_2,...,x_n may be computed as

$$\text{"MAD"} = 1.4826 \times \text{"median"} \left( |x\_i - \text{"median"} (x)| \right)$$

An alternative MAD that does not use the factor 1.4826 can also be used.

The factor 1.4826 is used to ensure that in case the variable x is normally distributed with a mean $\mu$ and a standard deviation $\sigma$ that the MAD score converges to $\sigma$ for large n. To ensure this, one can derive that the constant factor should equal $1/((\Phi^{-1}(3/4)))$, with $\Phi^{-1}$ is the inverse of the cumulative distribution function for the standard normal distribution.

Apart from the parameter p which will allow the identification of the presence of an aneuploidy, secondary parameters can be calculated which may serve as quality control or provide additional information with regard to one or more aneuploidies present in the sample.

A first secondary parameter which can be calculated allows defining whether chromosomal and large subchromosomal aneuploidies are present in the sample (compared to e.g. smaller aneuploidies). In a preferred embodiment, such parameter is defined by the median of Z scores measured per subregions (e.g. 5 Mb windows) in a target chromosome or target chromosomal section. If more than 50% of these subregions are affected, this will show in the secondary parameter.

In another embodiment, a secondary parameter may be calculated as the median of the absolute value of the Z scores calculated over the remaining chromosomes (that is all chromosomes except the target chromosome or chromosomal segment) per subregion (e.g. 5 Mb windows).

The latter secondary parameter allows the detection of the presence of technical or biological instabilities. If less than half of the windows of the other or all chromosomes are affected, this secondary parameter will not be affected. If more than 50% of the windows is affected, this will be derivable from said secondary parameter.

In another embodiment, the present teachings also provides for a quality score (QS). QS allows to assess the overall variation across the genome. A low QS is an indication of a good sample processing and a low level of technical and biological noise. An increase in the QS can indicate two possible reasons. Either an error occurred during the sample processing. In general, the user will be requested to retrieve and sequence a new biological sample. This is typical for moderately increased QS scores. A strongly increased QS may be an indication of a highly aneuploid sample and the user will be encouraged to do a confirmatory test. Preferably, said QS is determined by calculating the standard deviations of all Z scores for the autosomes or chromosomes and by removing the highest and lowest scoring chromosome.

For instance, samples with a QS exceeding 2 are considered to be of poor quality, and a QS between 1.5 and 2 are of intermediate quality.

IV. Comparison to cutoff value

The parameter p as calculated in the embodiments above shall subsequently be compared with a cutoff parameter or cutoff range for determining whether a change compared to a reference quantity exists (i.e. an imbalance), for example, with regards to the ratio or correlation of amounts of two chromosomal regions (or sets of regions). In one embodiment, the user will be able to define its own cutoff value, either empirically on the basis of experience or previous experiments, or for instance based on standard statistical considerations. If a user would want to increase the sensitivity of the test, the user can lower the thresholds (i.e. bring them closer to 0). If a user would want to increase the specificity of the test, the user can increase the thresholds (i.e. bring them further apart from 0). A user will often need to find a balance between sensitivity and specificity, and this balance is often lab- and application -specific, hence it is convenient if a user can change the threshold values him- or herself.

Based on the comparison with the cutoff value, an aneuploidy may be found present or absent and/or may give an indication on the sensitivity or accuracy of a previous ploidy call.

In an embodiment of the present teachings, comparison of parameter p with a cutoff value is sufficient for determining the presence or absence of an aneuploidy. In another embodiment, said aneuploidy is determined on the basis of a comparison of parameter p with a cutoff value and a comparison of at least one of the secondary parameters, quality score and/or first score with a cutoff value, whereby for each score a corresponding cutoff value is defined or set.

In a preferred embodiment, said presence/absence of an aneuploidy is defined by a comparison of parameter p with a predefined cutoff value, as well as by comparison of all secondary parameters, quality and first scores as described above with their corresponding cutoff values.

The final decision tree may thus be dependent on parameter p alone, or combined with one of the secondary parameters and/or quality score or first score as described above.

In a preferred embodiment, said methodology according to the present teachings comprise the following steps:

- multiplex sequencing of 50 bp single-end reads (performed by end user)
- uploading sequence reads
- mapping of reads to a reference genome
- count number of reads per bin (a bin has a size of 50 kb)
- compute GC content per bin and correct for GC content
- compute Genomic Representation (GR) score per bin. For bin i this equals

$$GRi = \frac{\sum_{j \in (bins\ on\ chr)} GCi}{\sum_{k \in (all\ bins)GCk} GCk}$$

whereby GCi represents the GC corrected number of reads for bin i, and GCk represents the GC corrected number of reads for bin k.
- aggregate the GR values per window (a window consists out of 100 consecutive windows)
- compute a Z score per window or per chromosome, whereby the Z score is based on the GR score per chromosome, compared with the GR scores obtained in a set of reference samples.

$$Z_i = \frac{GR_i - \mu_{Ref,i}}{\sigma_{Ref,i}}$$

with i a chromosome or a window, $\mu_{Ref,i}$ the average or median GR score for the corresponding bins in the set of reference samples and $\sigma_{Ref,I}$ the standard deviation of the GR scores for the corresponding bins in the set of reference samples
- computing of a ZofZ parameter, whereby the ZofZ parameter is based on the Z score, corrected by the median (or mean) of the Z scores of a collection of chromosomes or chromosome segments including target chromosome i and divided by a factor that measures the variability of the Z scores of a collection of chromosomes that includes the target chromosome i (standard deviation or a more robust version thereof, like e.g. the median absolute deviation or mad).
- comparison of the Z score with a threshold value, and the ZofZ parameter with a threshold value, to predict the presence or absence of an aneuploidy.

In a preferred embodiment, a sample will be found normal if the Z score is within the threshold range of -3 and 3, and/or the ZofZ parameter is within a threshold range of -3 and 3. Alternatively, a user can set wider or smaller ranges to shift towards higher specificity or higher sensitivity, respectively.

In a further preferred embodiment, said prediction of the presence or absence of an aneuploidy occurs via a decision tree based on parameter p and secondary parameters.

The present teachings provide a robust method for determining the presence or absence of an aneuploidy in a test sample, with a high sensitivity.

In another or further aspect, the present teachings equally provide a methodology for improving and/or verifying the accuracy of an aneuploidy calling in a test sample obtained from a pregnant female. Said method comprises the calculation of a first score as described above, based on sequences obtained from a biological sample from a pregnant female. These sequences are then processed as described in detail above. The first score provides a first indication of the presence or absence of an aneuploidy in the sample. In particular, said first score may be compared to a cutoff value or cutoff range in order to evaluate whether or not the chance for an aneuploidy exist.

In a second step, a parameter p is calculated as described above. In a most preferred embodiment, parameter p will be calculated as:

$$Z \, of \, Z_i = \frac{Z_i - \underset{j=i,a,b,\dots}{\mathrm{median}}(Z_j)}{\underset{j=i,a,b,\dots}{\mathrm{mad}}(Z_j)}$$

By comparison to a cutoff value or range, parameter p will provide a manner of verifying and/or improving the accuracy of the ploidy calling based on the first score. If the first score indicates the presence of an aneuploidy in said sample, calculation of parameter p allows the verification of this first, (possibly preliminary) diagnosis. Only if both the first score and parameter p comply with the predefined settings and/or boundaries, an assessment of the sample can be made with high certainty. If parameter p contradicts the first findings based on the first score, the latter indicates that these first findings may be incorrect, requiring further analysis. Such further analysis may include retesting (that is performing the analysis anew) or resampling.

Because parameter p provides useful inside information on the manner of behavior of a test chromosome or chromosome of interest in a test sample, thereby improving the accuracy and sensitivity of the diagnosis, unnecessary procedures for the patient may be avoided. When an aneuploidy is detected in a patient on the basis of the present teachings (either via the first score of via parameter p), the patient will be advised to undergo a further analysis procedure, which is often invasive for both fetus and mother. By improving the accuracy of the detection via the present teachings, such procedures are reduced to a minimum, and will only be executed when absolutely required. Hence, the present teachings equally provide a method for reducing the referral to an invasive procedure for determining the presence or absence of a fetal aneuploidy.

As a consequence, the present teachings equally are directed to a method for refining ploidy calls and/or identify miscalls on the basis of a first score as discussed above, by calculating a parameter p as described above.

In a further or other aspect, the present teachings provide a method for assessing the behavior of a target chromosome or a chromosome of interest within a test sample, compared to the behavior of the other chromosomes in said test sample. The behavior of the target chromosome is determined by the calculation of parameter p of the chromosome as described above. In particular, said behavior is to be understood as being similar or distinct in view of the whole population of chromosomes in a test sample.

The present teachings equally pertain to a method for evaluating whether or not a pregnant female should be advised to perform or undergo further invasive testing for assessing an aneuploidy in a fetus. Said methodology comprises the calculation of a first score and parameter p as described above, for a target chromosome within a test sample obtained from a pregnant female. If both said first score and parameter p or either the first score or parameter p are found to be outside a threshold range, the subject will be advised to seek further analysis, for instance in the form of an invasive test such as amniocentesis, chorionic villus sampling and/or cordocentesis. Said threshold range may be chosen by the practitioner. In a preferred embodiment, a first score will be labeled as normal (e.g. no further action required) if found to be within the range of -3 and 3. In a further preferred embodiment, said parameter p will be labeled as normal if found to be within the range of -2.5 and 2.5, more preferably -3 and 3. In a most preferred embodiment, referral for further testing will occur if both the first score and parameter p are outside the range of of -2.5 and 2.5, more preferably -3 and 3 or if parameter p is outside the range of of -2.5 and 2.5, more preferably -3 and 3.

In yet a further aspect, the present teachings provide for a methodology for setting up a non-invasive diagnostic or predictive tool for the prediction of diagnosis of an aneuploidy in samples obtained from a pregnant female comprising both fetal and maternal cfDNA. In a further embodiment, such method comprises the use of an initial reference set, whereby said reference is comprised of a collection of reference samples, to be used to determine the expected genomic representation values for normal samples. Said reference set may be comprised of at least 3 samples, or at least 3, 4, 5,6, 7, 8, 9, 10, 11, 12, 13, 14, 15, 16, 17, 18, 19, 20, 21, 22, 23, 24, 25 samples in order to set up the tool and/or to operate the tool. In one embodiment, the ploidy status of the samples in the reference set is unknown prior to use. In another, more preferred embodiment, said samples in the reference set are devoid of any chromosomal abnormalities.

Said methodology may comprise the analysis of the samples in said reference set, whereby each sample is analyzed on the presence or absence of chromosomal abnormalities. For this analysis, the methodology as described above whereby a first score and a parameter p for a target chromosome in the reference sample is calculated. Additionally (and optionally) also secondary scores as discussed above may be calculated. By preference, all chromosomes within the samples of the reference set will be evaluated. As a reference set for the calculations, the initial reference set is used.

If a test sample on the basis of the calculations is found to comprise chromosomal abnormalities, these test samples may, in a subsequent step, be omitted from the initial reference set, thereby only maintaining those samples which are found to be devoid of any abnormalities.

In a further step, the initial reference set may be further expanded by the addition of these sample data which are analyzed by the tool and which are found to be normal. As such, the reference set is not a static element in the tool, but may help to further increase the sensitivity of the tool by addition of further data throughout the use of the tool.

In contrast to the prior art methodologies, the method of the present teachings allow a straightforward analysis of a test sample, thereby taking into account previously obtained data of a reference set which may be limited in size (even to merely 3 samples), and which does not require any knowledge of the ground truth or influenced by specific choices /preferences of the end user. This is an advantage in view of the prior art.

The methodology of the present teachings are equally devoid of use of a normalizing chromosome within the test sample itself, for performing the analysis with regard to the presence or absence of an aneuploidy. In case the used normalizing chromosome is corrupt itself, such approach may lead to wrong conclusions.


V. Determination of Fetal Fraction


Apart or next to the determination of the presence of an aneuploidy, the present teachings also provide for one or more methodologies for determining the fetal fraction or fetal nucleic acids in a sample which is a mixture of fetal and maternal nucleic acids. In general, the fetal fraction within the sample will be so low that it cannot be easily determined. In general, the fetal fraction in samples will vary from 4 to 20% across different samples, with an average of about 10% of the total genome fraction.

The present teachings provide for two different methodologies of defining the fetal fraction in a sample, depending on the nature of the pregnancy.

A first methodology is independent of the type of pregnancy or sex of the fetus. Such methodology is based on the presence of polymorphisms in the maternal (and fetal) DNA of the sample. More specifically, it is based on predefine stretches of DNA that can be present in the fetal DNA and absent in the maternal DNA. Polymorphic sites that are contained in the target nucleic acids include without limitation single nucleotide polymorphisms (SNPs), tandem SNPs, small-scale multi-base deletions or insertions, called IN-DELS (also called deletion insertion polymorphisms or DIPs), Multi-Nucleotide Polymorphisms (MNPs), Copy Number Variations (CNVs) and Short Tandem Repeats (STRs). In a most preferred embodiment, said polymorphisms are CNVs. In a first step, the sample is sequenced as described above and reads are mapped against a reference genome.

In a subsequent step, the number of sequences that align to each of a predefined set of polymorphisms is counted. Optionally this can be achieved by mapping the obtained sequence reads to each of said predefined polymorphisms. Said predefined set of polymorphisms is to be understood as a collection of polymorphisms which have been identified and are deemed to be of relevance for the determination of the fetal fraction.

Said set of polymorphisms are preferably benign polymorphisms with frequent occurrence in population. In a preferred embodiment, said set comprises CNVs. Such CNVs may be variable in size, in a preferred embodiment, said CNVs have a length between 10 kb to 1Mb, more preferably between 10 kb and 100 kb. Alternatively, said CNVs have a length between 2 bp and 10 Mb. Herein kb refers to kilobasepairs (i.e. 1,000 basepairs) and Mb refers to megabasepairs (i.e. 1,000,000 basepairs)

In a further embodiment, said set of polymorphisms comprises additional data which is linked to the polymorphisms within said set. In a preferred embodiment, said data comprises one or more attributes of each polymorphism. Said attributes may comprise, but are not limiting to a correction factor for each polymorphism, whereby said correction factor provides a link between read counts for said polymorphism and the actual fetal fraction. The latter allows for a correction of the obtained reads corresponding to a polymorphism within a sample thereby obtaining an estimate of the fetal fraction or the actual fetal fraction.

Said attributes may comprises a cutoff value per polymorphism which allows identifying whether said polymorphism, if present in a sample, qualifies as informative polymorphism.

In a preferred embodiment, said attributes are determined using a set of samples for which the fetal fraction is known. These samples could be e.g. male pregnancies (for which the fetal fraction can be determined using the reads coming from the X or Y chromosome, as described further), or alternatively, samples for which the fetal fraction was determined using orthogonal methods (based on epigenetics or targeted sequencing or digital PCR).

In an embodiment, said set of polymorphisms and attributes are predefined. In another embodiment, said set of polymorphisms and attributes may be defined by the user.

In a subsequent step, the obtained read count - or a derivative thereof - for each polymorphism is used to identify whether the particular polymorphism is informative in the sample. In general, such informative polymorphisms are those polymorphisms which have a lower read number than a certain cut-off, in which the cut-off may correspond to the theoretically expected number of reads given the total read number for the sample, or a derivative thereof. It is deemed that the lower amount of observed read counts for such informative polymorphisms is due to their presence in the fetal genome and not in the maternal genome. Preferably, the number of reads - or a derivative thereof - should be less than half of the theoretically expected number of reads, as this would mean that the polymorphism is not present in 1, 2 or more copies in the maternal genome and hence only present in the fetal genome. Based on these informative polymorphisms, an estimation of the fetal fraction can be made by assuming that the obtained read count is directly correlated with the fetal fraction. In a preferred embodiment, the fetal fraction is calculated by first correcting the obtained read count for each informative polymorphism using its polymorphism specific attributes, and subsequently taking the median or average of the corrected read counts across all informative polymorphisms. A sample should have at least one informative polymorphism in order to be able estimate the fetal fraction. The fetal fraction of samples for which no informative polymorphism was identified can be estimated using alternative methods, provided that the sample is derived from a male fetus (see below).

In one embodiment, the fraction of fetal nucleic acids in the mixture of fetal and maternal nucleic acids is calculated for each of the informative polymorphism. In a first step, the expected count for the informative polymorphism is computed based on the normalized counts (normalized towards e.g. 10.000.000) obtained from said sample. Subsequently, based on the expected counts (i.e. the number of reads that one would expect for the polymorphism, given the total number of reads obtained for the sample, and optionally corrected with a polymorphism specific attribute), an estimation of the fetal fraction of the test sample is derived.

In an embodiment, this estimation can be computed for each informative polymorphism, using the formula 2 x 100 x observed counts for the informative polymorphism / expected counts for the informative polymorphism.

The expected count is the number of reads that one would expect for the polymorphism, given the total number of reads obtained for the sample, and optionally corrected with a polymorphism specific attribute. This polymorphism specific attribute or factor can be derived for each polymorphism in said set of polymorphisms using a set of samples for which the fetal fraction is known using alternative methods (e.g. for male fetuses using read counts on chromosome X or Y).

In an embodiment, the actual fetal fraction can be calculated as follows:

$$\text{Actual fetal fraction} = \text{estimated fetal fraction} \times \text{factor.}$$

The percent fetal fraction is calculated for at least 1, at least 2, at least 3, at least 4, at least 5, at least 6, at least 7, at least 8, at least 9, at least 10, at least 11, at least 12, at least 13, at least 14, at least 15, at least 16, at least 37, at least 18, at least 19, at least 20, at least 25, at least 30, at least 35, at least 40 or more informative polymorphisms. In an embodiment, the fetal fraction will be determined by the average or median fetal fraction as determined for each individual informative polymorphisms. In one embodiment, the fetal fraction is the average or median fetal fraction determined for at least 1, 2 or 3 informative polymorphisms.

In general, determination of the fetal fraction in a sample from a pregnant female is obtained by obtaining the read counts from one or more polymorphisms in a sample, and determining whether a polymorphism is informative based on these read counts and polymorphism specific attributes, whereby the read count of each informative polymorphism is related to the estimated fetal fraction.

In an embodiment of the present teachings, said fetal fraction in a sample is determined as follows:

- receiving the sequences of at least a portion of the nucleic acid molecules contained in a biological sample obtained from said pregnant female;

- counting the number of sequences that align to a predefined set of polymorphisms

- comparing the obtained number of sequences with the expected number of sequences for each polymorphic site to identify the informative polymorphic site(s) for the sample;

- calculating from the obtained number of sequences for said informative polymorphic site(s) an amount, whereby said amount is an indication for the fetal fraction.

Said amount is calculated using linear scaling based on informative polymorphism-specific attributes.

In one embodiment, said sequences are obtained by next generation sequencing. In a further preferred embodiment, said sequencing method is a random low coverage sequencing method.

By preference, said polymorphisms are copy number variations with a size between 100 bp and 1 Mb, or between 1 kb and 1 Mb, or between 2 bp and 10 Mb.

Said fetal fraction may serve as an internal quality control of the sample and thus accounts for another secondary parameter obtained from said sample.

In another embodiment of the present teachings, a method for determining the fetal fraction is based on cases in which a male pregnancy (i.e. the pregnant woman carries a male fetus) was identified. If a male pregnancy has been detected, the fetal fraction can be determined on the basis of reads aligned to the Y chromosome. The X and Y chromosome typically have regions that are similar between X and Y chromosome, called Pseudo-Autosomal-Regions (PAR). As the Y chromosome is small in size, the influence of these PAR regions is strong, as only a minor amount of the reads will be attributed to specific regions within the Y chromosome. The influence of PAR on the X chromosome is of lesser importance due to the size of the X chromosome.

In a first step, those regions that are unique for the X or Y chromosome (outside the PAR regions) are defined. Reads directed against these unique X and/or Y chromosome regions are counted and the fetal fraction is determined on the basis of the unique X and/or Y chromosome regions (or a derivative of these reads, such as normalised reads).

Based on the X-chromosomes, fetal fraction can be computed as twice the difference at the 50kb bin level between the median number of reads mapping to the autosomes and the median number of reads mapping to chromosome X, divided by the median number of reads mapping to the autosomes. This can be written as the following formula:

$$\mathrm{FF}_{\mathrm{X}} = 2 \times \left| 1 - \frac{\underset{\mathrm{on}\,X}{\mathrm{median}}(\mathrm{GC} - \mathrm{corr\ counts})}{\underset{\mathrm{on\ autosomes}}{\mathrm{median}}(\mathrm{GC} - \mathrm{corr\ counts})} \right|$$

Secondly, fetal fraction can also be estimated based on the Y-chromosome as all reads that map to chromosome Y should in theory originate from the fetal DNA. Chromosome Y-based fetal fraction is defined as twice the median number of GC-corrected reads mapping to Y over the median number of GC-corrected reads mapping to the autosomes, or in a formula:

$$\mathrm{FF}_{\mathrm{Y}} = 2 \times \frac{\underset{\mathrm{over}\,Y}{\mathrm{median}}(\mathrm{GC} - \mathrm{corr\ counts})}{\underset{\mathrm{on\ autosomes}}{\mathrm{median}}(\mathrm{GC} - \mathrm{corr\ counts})}$$

The present teachings equally provide for a computer program product comprising a computer readable medium encoded with a plurality of instructions for controlling a computing system to perform an operation of determining or estimating the fetal fraction in a biological sample obtained from a pregnant female subject according to the present teachings. Specifically, the operation comprises the steps of:

- receiving the sequences of at least a portion of the nucleic acid molecules contained in a biological sample obtained from said pregnant female;

- counting the number of sequences that align to a predefined set of polymorphisms

- comparing the obtained number of sequences with the expected number of sequences to identify the informative polymorphic site(s) for the sample; and

- calculating from the obtained number of sequences for said informative polymorphic site(s) an amount, whereby said amount is an indication for the fetal fraction.

VI Gender determination

Gender determination can occur by determining regions which are informative or indicative for male pregnancies. These regions can be defined by looking into a dataset comprising sequencing data from male datasets. Regions that are statistically indicative for male pregnancies are subsequently withhold.

The reads for one or more informative regions - which typically reside on the Y chromosome - are obtained from the analyzed sample and compared to a predefined threshold value. If the total number of reads across all selected regions in a test sample is above a first threshold value, it is most possibly a male pregnancy. On the other hand, if the total number of reads across all selected regions in a test sample is below a second threshold value, it is most possibly a female pregnancy. If the total number of reads across all selected regions in a test sample is in between the first and the second threshold value, the gender is undetermined (this could be the case for vanishing twins). By proper selection of the regions, the first and the second threshold values, the method would be less sensitive towards bias resulting from vanishing twins (especially vanishing boys).

VII Toolbox and kit

By preference, the methodologies as described above are all computer implemented. To that purpose, the present teachings equally relate to a computer program product comprising a computer readable medium encoded with a plurality of instructions for controlling a computing system to perform an operation for performing prenatal diagnosis of a fetal aneuploidy and/or screening for fetal aneuploidies and/or determination of the fetal fraction in a biological sample obtained from a pregnant female subject, wherein the biological sample includes nucleic acid molecules.

With regard to the determination of the presence or absence of an aneuploidy in a sample, the operation comprises the steps of:

- receiving the sequences of at least a portion the nucleic acid molecules contained in a biological sample obtained from said pregnant female;
- aligning said obtained sequences to a reference genome;
- counting the number of reads on a set of chromosomal segments and/or chromosomes thereby obtaining read counts;
- normalizing said read counts or a derivative thereof into a normalized number of reads;
- obtaining a first score of said normalized reads and a collection of scores of said normalized reads, whereby said first score is derived from the normalized reads for a target chromosome or chromosomal segment and whereby said collection of scores is a set of scores derived from the normalized number of reads for a set of chromosomes or chromosomal segments that include said target chromosomal segment or chromosome;
- calculating a parameter p from said first score and said collection of scores.

In a preferred embodiment, said parameter whereby said parameter represents a ratio or correlation between

    * a first score, corrected by a summary statistics of said collection of scores, and
    * a summary statistics of the collection of scores.

With regard to the determination of the fetal fraction within a biological sample, said operation comprises the steps of:

- receiving the sequences of at least a portion of the nucleic acid molecules contained in a biological sample obtained from said pregnant female;
- counting the number of sequences that align to a predefined set of polymorphisms
- comparing the obtained number of sequences with the expected number of sequences to identify the informative polymorphic site(s) for the sample; and
- calculating from the obtained number of sequences for said informative polymorphic site(s) an amount, whereby said amount is an indication for the fetal fraction.

In another embodiment, said fetal fraction can be determined in case of a male pregnancy on the basis of the Y chromosome.

Said operations comprise the steps of

- receiving the sequences of at least a portion of the nucleic acid molecules contained in a biological sample obtained from said pregnant female;
- determining the gender of said fetus; whereby if said fetus is male:

        * aligning said obtained sequences to a reference database;
        * identifying and counting reads specifically located in non-PAR regions of the X and/or Y chromosome;
        * calculating from said read counts an amount, whereby said amount is an indication for the fetal fraction.

Said operations can be performed by a user or practitioner in an environment remote from the location of sample collection and/or the wet lab procedure, being the extraction of the nucleic acids from the biologic sample and the sequencing.

Said operations can be provided to the user by means of adapted software to be installed on a computer, or can be stored into the cloud.

After having performed the required or desired operation, the practitioner or user will be provided with a report or score, whereby said report or score provides information on the feature that has been analyzed. Preferably, report will comprise a link to a patient or sample ID that has been analyzed. Said report or score may provide information on the presence or absence of an aneuploidy in a sample, whereby said information is obtained on the basis of a parameter which has been calculated by the above mentioned methodology. The report may equally provide information on the nature of the aneuploidy (if detected, e.g. large or small chromosomal aberrations) and/or on the quality of the sample that has been analyzed.

Alternatively, said practitioner or user may be provided with information on the fetal fraction, whereby said fetal fraction has been determined by one of methodologies of the present teachings.

In another embodiment, said practitioner or user may, by virtue of the report, be provided with gender information of the fetus.

It shall be understood by a person skilled in the art that above-mentioned information may be presented to a practitioner in one report.

By preference, above mentioned operations are part of a digital platform which enables molecular analyzing of a sample by means of various computer implemented operations.

In particular, the present teachings also comprises a visualization tool, which enables the user or practitioner to visualize the obtained results as well as the raw data that has been imputed in the system. In an embodiment, said visualizations comprises a window per chromosome, depicting the chromosome that has been analyzed, showing the reads per region or a score derived thereof and the scores and/or parameter that has been calculated. By showing to the practitioner or user the calculated scores and parameter together with the visual depiction of the read counts, a user may perform an additional control or assessment of the obtained results. By allowing the user to look at the data, users will be able to define improved decision rules and thresholds.

Moreover, an additional control is added, as the visual data per chromosome enables the user to evaluate for every chromosome if the automated classification is correct. This adds an additional safety parameter.

In a preferred embodiment, said platform and visualization tool is provided with algorithms which take into account the fact that certain regions give more reads (due to a recurrent technical bias that makes some regions of the genome always over- or under-represented). Correction measures may be provided for this overrepresentation by making a comparison with a reference set (that is ideally processed using the same or similar protocol) and plotting e.g. Z scores or alternative scores that represent the unlikeliness of certain observations under the assumption of euploidy. Standard visualization tools only display read count, and do not allow to correct for the recurrent technical bias.

Finally, based on the link between the obtained scores and/or parameter and the visual data per chromosome a user or practitioner may decide to alter the threshold/cutoff value that is used to define the presence of an aneuploidy. As such, the user may decide to aim for a higher sensitivity (e.g. being less stringent on the decrease/increase of the parameter or scores) or higher specificity (e.g. by being more stringent on the increase/decrease of parameter or scores).

The platform may be provided with other features, which provide for an accurate analysis of the molecular data obtained from the biological sample.

As previously mentioned, the methodology and platform allows a certain degree of liberty to the user. Apart from defining own cutoff values and thresholds, the user may also define own reference sets of genomes, to be used to calculate the scores and/or other information such as fetal fraction, gender determination etc. By using their own reference set, a user may be allowed to better capture the recurrent technical variation of the lab (different wet lab reagents and protocol, different NGS instrument and platform, different operator, different ...) and hence have a more suitable reference data set to that particular lab. In order to ensure the robustness of a new reference set, methodologies are provided to remove outliers from the reference set. E.g. if 100 reference samples are used in a reference set, there will be 100 reference results for each 50 kb bin. If a certain predefined percentage of outliers is removed (e.g. 5% of the results), the reference set can be made more robust to variation in the reference set.

In an embodiment, a method for performing CNV calling is provided. With the term 'CNV calling' it is meant a methodology that determines the boundaries of a CNV of a CNV or a segmental aneuploidy. Said boundaries are to be understood as the approximate chromosomal coordinates.

Subsequently, these boundaries are used to cross-reference with CNV reference genome databases which contain previously observed CNVs (optionally annotated, e.g. benign or pathogenic). CNV calling can be performed by various methodologies. Some of them were developed in the array-CGH field (where one or adjacent set of aCGH probe(s) can be considered as the equivalent of a bin), others are more specific for NGS data.

In another embodiment, said platform allows CNV quantification. With the term CNV quantification is understood the determination of the absolute number of copies (or the expected range) of the observed CNV. The latter will allow determining if a CNV is rather maternal (very high value) or rather fetal (very low value). By preference, said CNV quantification is done after CNV calling. In another, more preferred embodiment, said CNV calling takes into account knowledge on the cell-free fraction.

In an embodiment, said platform allows CNV signature recognition. With the term 'CNV signature recognition' a methodology for determining whether a specific combination of CNVs (and their quantity) is present in a sample is understood. By preference, CNV signature recognition is performed after CNV calling and CNV quantification.

All methodologies mentioned above employ use of one or more CNV reference genome databases which comprise known CNVs. The methodologies mentioned above can be based on the aligning of sequences obtained from a biological sample against said one or more CNV reference databases, or alternatively align the sequences obtained from a biological sample against a reference genome and subsequently identify the reads that were aligned to specific regions of interest (i.e. the regions identified as CNVs in the CNV reference databases). Coupling to such reference databases allow the identification of CNVs that are (likely) pathogenic and thus the (clinical) accuracy is increased: if a CNV is observed, and it contains a (likely) pathogenic region, it could be very relevant and requiring follow up of the patient.

The platform according to the present teachings will allow a large degree of liberty to the user or practitioner. By preference, said platform will be compatible with various data formats such as fastq, fastq.gz, bcl and bam files. In a further embodiment, said platform is able to receive data formats which are directly streamed from the sequencing platform that is used (e.g. NGS instrument). The latter strongly reduces the waiting time for data upload as the upload happens simultaneously with the sequencing reaction. As such, the total time-to-result will be optimized, which is beneficial for the user.

In an embodiment, said platform is compatible with sequencing data from various sources, including SMRT (single molecule real time) sequencing data. Algorithms can be present that enable the processing of SMRT data (as e.g. PacBio) and deduce epigenetic information (e.g. methylation or other modifications) from said SMRT data. The latter again allows identification of parameters that indicate aneuploidy detection, or could aid in the determination of the fetal fraction, or determine quality metrics.

The platform according to the present teachings is inherently compatible with many different types of NGS library preparation kits and protocols and NGS sequencing platform. This is an advantage as a user will not have to invest in dedicated NGS sequencing platform or NGS library preparation kits that are specific for a specific application, but instead a user can use its preferred platform and kit. Moreover, this allows a user a certain degree of flexibility in material to be used. If newer or cheaper instruments or kits become available, a user will be allowed an easy change.

As mentioned before, the current methodology is compatible with cell-free DNA extracted from various sorts of biological samples, including blood, saliva, blastocoel fluid and urine. Using urine or saliva instead of blood would represent a truly non-invasive sample type and allows for e.g. home-testing and shipment of the sample to the test lab. This is obviously an additional advantage compared to other sample obtaining methods such as drawing blood.

B. Detection of a chromosomal aberrancy in a sample

The present teachings describe a methodology for determining whether a subject has tumor-derived cell-free DNA in his or her peripheral blood, for confirming a cancer diagnosis, for aiding in the classification of a cancer, for assessing the treatment response, for monitoring the subject, for identifying the presence of a cancer and/or an increased risk of a cancer in a subject, said subject is preferably a mammal.

In a first aspect, the method for determining whether a subject has tumor-derived cell-free DNA in his or her peripheral blood, for confirming a cancer diagnosis, for aiding in the classification of a cancer, for assessing the treatment response, for monitoring the subject, for identifying the presence of a cancer and/or an increased risk of a cancer in a subject is based on the determination of a parameter from the nucleic acid content of a biological sample. The biological sample may be plasma, urine, serum, blastocoel fluid, cerebrospinal fluid or any other suitable sample. For example, the nucleic acid molecules may be fragments from chromosomes.

At least a portion of a plurality of the nucleic acid molecules contained in the biological sample is randomly sequenced to obtain a number of sequences. The portion sequenced represents a fraction of the human genome and may be isolated from the sample by conventional means (e.g. cell-free DNA extraction means and preparation of a NGS library). In one embodiment, the nucleic acid molecules are fragments of respective chromosomes. One end (e.g. 50 basepairs (bp)), both ends, or the entire fragment may be sequenced. A subset of the nucleic acid molecules in the sample may be sequenced, and this subset is randomly chosen, as will be described in more detail later.

In one embodiment, the random sequencing is done using massively parallel sequencing. Massively parallel sequencing, such as that achievable on the HiSeq2500, HiSeq3000, HiSeq4000, HiSeq X, MiSeq, MiSeqDx, NextSeq500, NextSeq550 flowcell, the 454 platform (Roche), Illumina Genome Analyzer (or Solexa platform) or PGM or Proton platform (IonTorrent) or GeneRead (Qiagen) or SOLiD System (Applied Biosystems) or the Helicos True Single Molecule DNA sequencing technology, the single molecule, real-time (SMRT™) technology of Pacific Biosciences, and nanopore sequencing as in MinION, PrmethION, GridION (Oxford Nanopore technologies), allow the sequencing of many nucleic acid molecules isolated from a specimen at high orders of multiplexing in a parallel fashion. Each of these platforms sequences clonally expanded or even non-amplified single molecules of nucleic acid fragments. Clonal expansion can be achieved via bridge amplification, emulsion PCR, or Wildfire technology.

As a high number of sequencing reads, in the order of hundred thousand to millions or even possibly hundreds of millions or billions, are generated from each sample in each run, the resultant sequenced reads form a representative profile of the mix of nucleic acid species in the original specimen. For example, the haplotype, transcriptome and methylation profiles of the sequenced reads resemble those of the original specimen. Due to the large sampling of sequences from each specimen, the number of identical sequences, such as that generated from the sequencing of a nucleic acid pool at several folds of coverage or high redundancy, is also a good quantitative representation of the count of a particular nucleic acid species or locus in the original sample.

Based on the sequencing (e.g. data from the sequencing), a first score of a target chromosome or chromosomal segment is determined. The first score is determined from sequences identified as originating from (i.e. aligning) to the target chromosome or segment. For example, a bioinformatics procedure may then be used to locate each of these DNA sequences to the human genome or a reference genome. It is possible that a proportion of such sequences will be discarded from subsequent analysis because they are present in the repeat regions of the human genome, or in regions subjected to inter-individual variations, e.g. copy number variations. A score of the target chromosome or chromosomal segment and of one or more other chromosomes may thus be determined.

Based on the sequencing, a collection of scores of one or more chromosomes or chromosomal segments is determined from sequences identified as originating from (i.e. aligning to) a set of one of more chromosomes. In one embodiment, said set contains all of the other chromosomes besides the first one (i.e. the one being tested). In another embodiment, said set contains just a single other chromosome. In a most preferred embodiment, said set contains chromosomes or chromosomal segments and includes the target chromosome or chromosomal segment.

There are a number of ways of determining a score. By preference, said score is based on the read counts obtained from sequencing. Said read counts can include, but are not limiting to counting the number of reads, the number of sequenced nucleotides (basepairs) or the accumulated lengths of sequenced nucleotides (basepairs) originating from particular chromosome(s) or chromosomal segments such as bins or windows or clinically relevant chromosome portions.

Rules may be imposed on the results of the sequencing to determine what gets counted. In one aspect, a read count may be obtained based on a proportion of the sequenced output. For example, sequencing output corresponding to nucleic acid fragments of a specified size range could be selected.

In one embodiment, said score is the raw read count for a certain chromosome or chromosomal segment.

In a preferred embodiment, said read counts are subjected to mathematical functions or operations in order to derive said score of said read counts. Such operations include but are not limiting to statistical operations, regression models standard calculations (sum, subtraction, multiplying and division), whereby said standard calculations are preferably based on one or more obtained read counts.

In a preferred embodiment, said first score is a normalized value derived from the read counts or mathematically modified read counts. In a further preferred embodiment, said score is a Z score or standard score relating to the read counts of a certain chromosome, chromosomal segment or the mathematically amended counts, in which the Z score quantifies how much the number of reads of a certain sequence differs from the number of reads that were obtained from the same sequence in a set of reference samples. It is known to a person skilled in the art how such a Z score can be calculated.

In a preferred embodiment, a parameter is determined based on a first score (corresponding to the target chromosome or chromosomal segment) and a collection of scores. The parameter preferably represents a relative score between the first score and a summary statistic of the collection of scores. The parameter may be, for example, a correlation or ratio of the first score to a summary statistic of the collection of scores. In one aspect, each score could be an argument to a function or separate functions, where a ratio or correlation may be then taken of these separate functions. The parameter may be, for example, a ratio or correlation of the first score to a summary statistic of scores present in the collection. In one aspect, each score could be an argument to a function or separate functions, where a ratio or correlation may be then taken of these separate functions.

In a preferred embodiment, the parameter may be obtained by a ratio or correlation between:

- a first function whereby the first score and the collection of scores are the arguments;

- a second function whereby the collection of scores is the argument.

In a more preferred embodiment, said first function is defined as a difference, preferably the difference between the first score and a summary statistic of the collection of scores, whereby said summary statistic is preferably selected from the mean, median, standard deviation or median absolute deviation (mad) or mean absolute deviation.

In a further preferred embodiment, said second function is defined as a variability summary statistic of the collection of scores, whereby said summary statistic may be an average or a measure of variability and is preferably selected from the mean, median, standard deviation or median absolute deviation (mad) or mean absolute deviation.

Typically, a suitable embodiment according to the present teachings involve the following steps (after having obtained DNA sequences from a random, low-coverage sequencing process on a biological sample).

- aligning sequences to a reference genome;

- obtaining the read counts per chromosome or chromosomal segment;

- normalizing the number of reads or a derivative thereof towards a normalized number of reads;

- obtaining a first score derived from said normalized number of reads and a collection of scores derived from said normalized reads, whereby said first score is derived from the normalized read counts for a target chromosome or chromosomal segment, and said collection of scores is a set of scores derived from the normalized number of reads that were obtained from a set of chromosomes or chromosome segments that include the target chromosomal segment or chromosome;

- calculating a parameter from said scores, whereby said parameter represents a ratio or correlation between said first score and a summary statistic of said collection of scores, whereby the first function of said ratio or correlation is defined as a difference between the first score and a summary statistic of said collection of scores; and whereby the second function of said ratio or correlation is defined as a summary statistic of said collection of scores. Preferably, said sequences are obtained by low coverage sequencing.

Said normalization preferably occurs on the basis of a set of reference samples, whereby said reference samples are preferably, though not necessary, euploid or essentially euploid for the chromosome or chromosomal segment that corresponds to the target chromosome or chromosomal segment (i.e. the majority of the chromosomes or chromosomal segments in the reference samples that correspond to the target chromosome or chromosomal segment in the test sample are euploid). Such reference set have various sample sizes. A possible sample size can be e.g. 100 samples, such as 50 male and 50 female samples. It will be understood by a skilled person that the reference set can be freely chosen by the user.

By preference, said number of reads is recalibrated to correct for GC content and/or total number of reads obtained from said sample

By taking into account a set of scores derived of reads of chromosomes or chromosomal segments that include the target chromosome or chromosomal segment for calculation the collection of scores a more robust, sensitive and reliable parameter is obtained as compared to known prior art methods. Other than the known prior art methods, there is no need to make an assumption on the ploidy state of any of the chromosomes in the test sample. Even if multiple aneuploidies would be present in the test sample or a lot of technical or biological noise is present (e.g. coming from the presence of cancer or CNVs), the current parameter p still offers a valuable tool, whereas the methods known in the art may fail in those situations (Vandenberghe et al., "Non-invasive detection of genomic imbalances in Hodgkin/Reed-Sternberg cells in early and advanced stage Hodgkin's lymphoma by sequencing of circulating cell-free DNA: a technical proof-of-principle study", 2015). In fact, by defining a parameter according to the present teachings, the parameter for the chromosome or region to be analyzed clearly stands out (i.e. is strongly increased/decreased), and does not disappear in the noise (i.e. only moderately or not increased/decreased). Moreover, for screening purpose, sensitivity is key, as it is important to have a reliable and trustworthy result, thereby minimizing the amount of false negatives. In fact, for screening purposes, it may be more important to have high sensitivity as compared to specificity.

The parameter according to the present teachings allow robustly detecting and automatically classifying chromosomes, even in noisy data. By taking into account a collection of chromosomes or segments, including the target chromosome or segment i.e. the majority of information that is available in the dataset, most of the available information is used, coming to a more adequate assessment. For instance, if one would remove e.g. chromosome 1 (the largest chromosome, 7.9% of the genome), a large amount of data would be removed that would not be taken into account, thereby causing a distortion in the assessment.

In particular, the present teachings are very useful in situations whereby a low number of reads or noisy data is obtained. The inventors found that in the latter situations, the parameter according to the present teachings performed superior compared to other methodologies.

In a preferred embodiment, said scores are obtained on the basis of the genomic representation of the target chromosome or chromosomal segment (or a region thereof) and the genomic representation of all autosomes or other chromosomes, thereby including the target chromosome or chromosomal segment..

The parameter is compared to one or more cutoff values. The cutoff values may be determined from any number of suitable ways. Such ways include Bayesian-type likelihood method, sequential probability ratio testing (SPRT), false discovery, confidence interval, receiver operating characteristic (ROC). In a more preferred embodiment, said cutoff value is based on statistical consideration or is empirically determined by testing biological samples. The cutoff value can be validated by means of test data or a validation set and can, if necessary, be amended whenever more data is available.

Based on the comparison, a classification of whether a chromosomal aneuploidy exists for the target chromosome is determined. In one embodiment, the classification is a definitive yes or no. In another embodiment, a classification may be unclassifiable or uncertain. In yet another embodiment, the classification may be a score that is to be interpreted at a later date, for example, by a doctor. In another embodiment, the classification may occur on a genome-wide level. In yet another embodiment, the classification may be a score that determines the likelihood for the presence of genome-wide instability or the presence of a predefined CNV signature (i.e. a defined combination of CNVs or subchromosomal or chromosomal copy number aberrations).

In a further preferred method, secondary parameters from the read counts are calculated, which serve as an additional internal control for the usefulness of the parameter, the extend of the aneuploidy (if identified) and/or an indication for the reliability of the parameter, the biological sample or the sequences obtained thereof and thus the final assessment. Said secondary parameters can be a prerequisite of the presence of said aneuploidy and/or a measure of quality of the sample as well as a measure for genome-wide instability.

In one embodiment, such secondary parameter is calculated as the median of the Z-distribution of the read counts or a derivative thereof, for a target chromosome or a target chromosomal segment measured per bin or an aggregation of bins (i.e. windows). The latter secondary parameters allow assessing if the majority (more than 50%) of the windows in a chromosome is increased or decreased. The latter allows the detection of chromosomal and large subchromosomal aneuploidies. When less than 50% of the windows are affected, the secondary parameters will not be affected (e.g. for smaller CNVs).

In another embodiment, said secondary parameters may be calculated as the median of the absolute value of the Z-scores for the read counts or a derivative thereof, of the remaining chromosomes (that is a collection of chromosomes or segments that exclude the target chromosome or segment).

The latter secondary parameters allow the detection of e.g. the presence of technical or biological instabilities (cf. malignancies, cancer) and to discriminate these from maternal CNVs. If less than the windows of the other or all chromosomes are affected, these secondary parameters will not be affected. If more than 50% of the windows is affected, this will be derivable from said secondary parameters.

In another embodiment, the present teachings also provide for a quality score (QS). QS allows to assess the overall variation across the genome. A low QS is an indication of a good sample processing and a low level of technical and biological noise. An increase in the QS can indicate two possible reasons. Either an error occurred during the sample processing. In general, the user will be requested to retrieve and test a new biological sample. This is typical for moderately increased QS scores. A strongly increased QS could be an indication of a highly aneuploid sample and the user will be encouraged to do a confirmatory test. Preferably, said QS is determined by calculating the standard deviations of all Z scores for chromosomes or chromosomal segments and optionally by removing the outliers thereof (i.e. the highest and lowest Z-scores in this collection).

In an embodiment of the present teachings, the parameter p will be sufficient to discriminate between the presence and/or absence of an aneuploidy. In a more preferred embodiment of the present teachings; both the parameter as the secondary parameters will be used to come to a decision with regard to the presence or absence of an aneuploidy. Preferably, also said secondary parameters will be compared to predefined threshold values.

In a preferred embodiment, said target chromosome or chromosomal segment comprise whole chromosomes amplifications and/or deletions of which are known to be associated with a cancer (e.g., as described herein). In certain embodiments said target chromosomes or chromosome segments comprise chromosome segment amplifications or deletions of which known to be associated with one or more cancers. In certain embodiments the chromosome segments comprise substantially whole chromosome arms (e.g., as described herein). In certain embodiments the chromosome segments comprise whole chromosome aneuploidies. In certain embodiments the whole chromosome aneuploidies comprise a loss, while in certain other embodiments the whole chromosome aneuploidies comprise a gain (e.g., a gain or a loss as shown in Table 1). In certain embodiments the chromosome segments of interest are substantially arm-level segments comprising a p arm or a q arm of any one or more of chromosomes 1-22, X and Y. In certain embodiments the aneuploidies comprise an amplification of a substantial arm level segment of a chromosome or a deletion of a substantial arm level segment of a chromosome. In certain embodiments the chromosomal segments of interest substantially comprise one or more arms selected from the group consisting of 1q, 3q, 4p, 4q, 5p, 5q, 6p, 6q, 7p, 7q, 8p, 8q, 9p, 9q, 10p, 10q, 12p, 12q, 13q, 14q, 16p, 17p, 17q, 18p, 18q, 19p, 19q, 20p, 20q, 21q, and/or 22q. In certain embodiments the aneuploidies comprise an amplification of one or more arms selected from the group consisting of 1q, 3q, 4p, 4q, 5p, 5q, 6p, 6q, 7p, 7q, 8p, 8q, 9p, 9q, 10p, 10q, 12p, 12q, 13q, 14q, 16p, 17p, 17q, 18p, 18q, 19p, 19q, 20p, 20q, 21q, 22q. In certain embodiments the aneuploidies comprise a deletion of one or more arms selected from the group consisting of 1p, 3p, 4p, 4q, 5q, 6q, 8p, 8q, 9p, 9q, 10p, 10q, 11p, 11q, 13q, 14q, 15q, 16q, 17p, 17q, 18p, 18q, 19p, 19q, 22q. In certain embodiments the chromosomal segments of interest are segments that comprise a region and/or a gene shown in Table 4 and/or Table 6 and/or Table 5 and/or Table 7. In certain embodiments the aneuploidies comprise an amplification of a region and/or a gene shown in Table 3 and/or Table 5. In certain embodiments the aneuploidies comprise a deletion of a region and/or a gene shown in Table 5 and/or Table 7. In certain embodiments the chromosome segments of interest are segments known to contain one or more oncogenes and/or one or more tumor suppressor genes. In certain embodiments the aneuploidies comprise an amplification of one or more regions selected from the group consisting of 20Q13, 19q12, 1q21-1q23, 8p11-p12, and the ErbB2. In certain embodiments the aneuploidies comprise an amplification of one or more regions comprising a gene selected from the group consisting of MYC, ERBB2 (EGFR), CCND1 (Cyclin D1), FGFR1, FGFR2, HRAS, KRAS, MYB, MDM2, CCNE, NRAS, MET, ERBB1, CDK4, MYCB, ERBB2, AKT2, MDM2, BRAF, ARAF, CRAF, PIK3CA, AKT1, PTEN, STK11, MAP2K1, ALK, ROS1, CTNNB1, TP53, SMAD4, FBX7, FGFR3, NOTCH1, ERBB4 and CDK4, and the like. In certain embodiments the cancer is a cancer selected from the group consisting of leukemia, ALL, brain cancer, breast cancer, colorectal cancer, dedifferentiated liposarcoma, esophageal adenocarcinoma, esophageal squamous cell cancer, GIST, glioma, HCC, hepatocellular cancer, lung cancer, lung NSC, lung SC, medulloblastoma, melanoma, MPD, myeloproliferative disorder, cervical cancer, ovarian cancer, prostate cancer, and renal cancer.

In certain embodiments the biological sample comprise a sample selected from the group consisting of whole blood, a blood fraction, saliva/oral fluid, urine, a tissue biopsy, pleural fluid, pericardial fluid, cerebral spinal fluid, and peritoneal fluid.

In certain embodiments the detection of aneuploidies or genome-wide instability or CNV signatures or microsatellite instability (MSI) indicates a positive result and said method further comprises prescribing, initiating, and/or altering treatment of a human subject from whom the test sample was taken. In certain embodiments prescribing, initiating, and/or altering treatment of a human subject from whom the test sample was taken comprises prescribing and/or performing further diagnostics to determine the presence and/or severity of a cancer. In certain embodiments the further diagnostics comprise screening a sample from said subject for a biomarker of a cancer, and/or imaging said subject for a cancer. In certain embodiments when said method indicates the presence of neoplastic cells in said mammal, treating said mammal, or causing said mammal to be treated, to remove and/or to inhibit the growth or proliferation of said neoplastic cells. In certain embodiments treating the mammal comprises surgically removing the neoplastic (e.g., tumor) cells. In certain embodiments treating the mammal comprises performing radiotherapy or causing radiotherapy to be performed on said mammal to kill the neoplastic cells. In certain embodiments treating the mammal comprises administering or causing to be administered to said mammal anti-cancer drugs like Receptor Tyrosine Kinase (RTK) inhibitors, kinase inhibitors, CTLA4 inhibitors, PD1 inhibitors, PDL1 inhibitors, immunotherapy, tumor-targeting T-cell therapies, chimeric antigen receptor (CAR) T-cell therapy, cancer vaccines (e.g., matuzumab, erbitux, vectibix, nimotuzumab, matuzumab, panitumumab, fluorouracil, capecitabine, 5-trifluoromethyl-2'-deoxyuridine, methotrexate, raltitrexed, pemetrexed, cytosine arabinoside, 6-mercaptopurine, azathioprine, 6-thioguanine, pentostatin, fludarabine, cladribine, floxuridine, cyclophosphamide, neosar, ifosfamide, thiotepa, 1,3-bis(2-chloroethyl)-1-nitosourea, 1,-(2-chloroethyl)-3-cyclohexyl-1 nitrosourea, hexamethylmelamine, busulfan, procarbazine, dacarbazine, chlorambucil, melphalan, cisplatin, carboplatin, oxaliplatin, bendamustine, carmustine, chloromethine, dacarbazine, fotemustine, lomustine, mannosulfan, nedaplatin, nimustine, prednimustine, ranimustine, satraplatin, semustine, streptozocin, temozolomide, treosulfan, triaziquone, triethylene melamine, thiotepa, triplatin tetranitrate, trofosfamide, uramustine, doxorubicin, daunorubicin, mitoxantrone, etoposide, topotecan, teniposide, irinotecan, camptosar, camptothecin, belotecan, rubitecan, vincristine, vinblastine, vinorelbine, vindesine, paclitaxel, docetaxel, abraxane, ixabepilone, larotaxel, ortataxel, tesetaxel, vinflunine, imatinib mesylate, sunitinib malate, sorafenib tosylate, nilotinib hydrochloride monohydrate/, tasigna, semaxanib, vandetanib, vatalanib, vemurafenib, dabrafenib, trametinib, ipilimumab, pembrolizumab, nivolumab, retinoic acid, a retinoic acid derivative, and the like).

Methods of monitoring a treatment of a subject for a cancer are also provided. In various embodiments the methods comprise performing a method for determining whether a subject has tumor-derived cell-free DNA in his or her peripheral blood, for confirming a cancer diagnosis, for aiding in the classification of a cancer, for assessing the treatment response, for monitoring the subject, for identifying the presence of a cancer and/or an increased risk of a cancer in a mammal as described herein on a sample from the subject or receiving the results of such a method performed on the sample before or during the treatment; and; performing the method again on a second sample from the subject or receiving the results of such a method performed on the second sample at a later time during or after the treatment; where a reduced number or severity of aneuploidy (e.g., a reduced aneuploidy frequency and/or a decrease or absence of certain aneuploidies) or a change in the CNV signature in the second measurement (e.g., as compared to the first measurement) can be an indicator of a positive course of treatment and the same or increased number or severity of aneuploidy or no or an adverse change in the CNV signature in the second measurement (e.g., as compared to the first measurement) can be an indicator of a negative course of treatment and, when said indicator is negative, adjusting said treatment regimen to a more aggressive treatment regimen and/or to a palliative treatment regimen.

**Table** 2 Illustrative specific, recurrent chromosome gains and losses in human cancer (see, e.g., Gordon et al. (2012) Nature Rev. Genetics. 13: 189-203).

| Chromosome | Gains (cancer type) | Losses (cancer type) |
| --- | --- | --- |
| 1 | Multiple myeloma, Adenocarcinoma (breast) | Adenocarcinoma (kidney) |
| 2 | Hepatoblastoma, Ewing's sarcoma | |
| 3 | Multiple myeloma, Diffuse large B-cell lymphoma | Melanoma, Adenocarcinoma (kidney) |
| 4 | Acute lymphoblastic leukaemia | Adenocarcinoma (kidney) |
| 5 | Multiple myeloma, Adenoca rci noma (kidney) | |
| 6 | Acute lymphoblastic leukaemia, Wilms' tumour | Adenocarcinoma (kidney) |
| 7 | Adenocarcinoma (kidney) | Acute myeloid leukaemia Juvenile myelomonocytic leukaemia |
| 8 | Acute myeloid leukaemia, Chronic myeloid leukaemia, Ewing's sarcoma | Adenocarcinoma (kidney) |
| 9 | Multiple myeloma, Polycythaemia vera | |
| 10 | Acute lymphoblastic leukaemia, Adenoca rci noma (uterus) | Astrocytoma, Multiple myeloma |
| 11 | Multiple myeloma | |
| 12 | Chronic lymphocytic leukaemia, Wilms' tumor | Multiple myeloma |
| 13 | Acute myeloid leukaemia, Wilms tumor | Multiple myeloma |
| 14 | Acute lymphoblastic leukaemia | |
| 15 | Acute lymphoblastic leukaemia | |
| 16 | Adenocarcinoma (kidney) | Multiple myeloma |
| 17 | Adenoca rci noma (kidney) Acute lymphoblastic leukaemia | |
| 18 | Acute lymphoblastic leukaemia, Wilms' tumour | Adenocarcinoma (kidney) |
| 19 | Multiple myeloma, Chronic myeloid leukaemia | Adenocarcinoma (Breast) Meningioma |
| 20 | Hepatoblastoma, Adenocarcinoma (kidney) | |
| 21 | Acute lymphoblastic leukaemia, Acute megakaryoblastic leukaemia | |
| X | Acute lymphoblastic leukaemia | |
| Y | Follicular lymphoma | |

In various embodiments, the method described herein can be used to detect and/or quantify whole chromosome aneuploidies that are associated with cancer generally, and/or that are associated with particular cancers. Thus, for example, in certain embodiments, detection and/or quantification of whole chromosome aneuploidies characterized by the gains or losses shown in Table 2 are contemplated.

Multiple studies have reported patterns of arm-level copy number variations across large numbers of cancer specimens (Lin et al. Cancer Res 68, 664-673 (2008); George et al. PLoS ONE 2, e255 (2007); Demichelis et al. Genes Chromosomes Cancer 48: 366-380 (2009); Beroukhim et al. Nature. 463(7283): 899-905 [2010]). It has additionally been observed that the frequency of arm-level copy number variations decreases with the length of chromosome arms. Adjusted for this trend, the majority of chromosome arms exhibit strong evidence of preferential gain or loss, but rarely both, across multiple cancer lineages (see, e.g., Beroukhim et al. Nature. 463(7283): 899-905 [2010]).

Accordingly, in one embodiment, methods described herein are used to determine arm level CNVs (CNVs comprising one chromosomal arm or substantially one chromosomal arm) in a sample. The CNVs can be determined in a test sample comprising a constitutional (germline) nucleic acid and the arm level CNVs can be identified in those constitutional nucleic acids. In certain embodiments arm level CNVs are identified (if present) in a sample comprising a mixture of nucleic acids (e.g., nucleic acids derived from normal and nucleic acids derived from neoplastic cells). In certain embodiments the sample is derived from a subject that is suspected or is known to have cancer e.g. carcinoma, sarcoma, lymphoma, leukemia, germ cell tumors, blastoma, and the like. In one embodiment, the sample is a plasma sample derived (processed) from peripheral blood that may comprise a mixture of cfDNA derived from normal and cancerous cells. In another embodiment, the biological sample that is used to determine whether a CNV is present is derived from a cells that, if a cancer is present, comprise a mixture of cancerous and non-cancerous cells from other biological tissues including, but not limited to biological fluids such as serum, sweat, tears, sputum, urine, sputum, ear flow, lymph, saliva, cerebrospinal fluid, ravages, bone marrow suspension, vaginal flow, transcervical lavage, brain fluid, ascites, milk, secretions of the respiratory, intestinal and genitourinary tracts, and leukophoresis samples, or in tissue biopsies, swabs, or smears. In other embodiments, the biological sample is a stool (fecal) sample.

In various embodiments the CNVs identified as indicative of the presence of a cancer or an increased risk for a cancer include, but are not limited to the arm level CNVs listed in Table 3. As illustrated in Table 3 certain CNVs that comprise a substantial arm-level gain are indicative of the presence of a cancer or an increased risk for a certain cancers. Thus, for example, a gain in 1q is indicative of the presence or increased risk for acute lymphoblastic leukemia (ALL), breast cancer, GIST, HCC, lung NSC, medulloblastoma, melanoma, MPD, ovarian cancer, and/or prostate cancer. A gain in 3q is indicative of the presence or increased risk for Esophageal Squamous cancer, Lung SC, and/or MPD. A gain in 7q is indicative of the presence or increased risk for colorectal cancer, glioma, HCC, lung NSC, medulloblastoma, melanoma, prostate cancer, and/or renal cancer. A gain in 7p is indicative of the presence or increased risk for breast cancer, colorectal cancer, esophageal adenocarcinoma, glioma, HCC, Lung NSC, medulloblastoma, melanoma, and/or renal cancer. A gain in 20q is indicative of the presence or increased risk for breast cancer, colorectal cancer, dedifferentiated liposarcoma, esophageal adenocarcinoma, esophageal squamous, glioma cancer, HCC, lung NSC, melanoma, ovarian cancer, and/or renal cancer, and so forth.

Similarly as illustrated in Table 3 certain CNVs that comprise a substantial arm-level loss are indicative of the presence of and/or an increased risk for certain cancers. Thus, for example, a loss in 1p is indicative of the presence or increased risk for gastrointestinal stromal tumor. A loss in 4q is indicative of the presence or increased risk for colorectal cancer, esophageal adenocarcinoma, lung sc, melanoma, ovarian cancer, and/or renal cancer. A loss in 17p is indicative of the presence or increased risk for breast cancer, colorectal cancer, esophageal adenocarcinoma, HCC, lung NSC, lung SC, and/or ovarian cancer, and the like.

**Table 3 Significant arm-level chromosomal segment copy number alterations in each of 16 cancer subtypes**

| Arm | Cancer Types Significantly Gained In | Cancer Types Significantly Lost In | Oncogene/Tumor Suppressor Gene |
|---|---|---|---|
| 1p | - | GIST | |
| 1q | ALL, Breast, GIST, HCC, Lung NSC, Medulloblastoma, Melanoma, MPD, Ovarian, Prostate | - | |
| 3p | - | Esophageal Squamous, Lung NSC, Lung SC, Renal | VHL |
| 3q | Esophageal Squamous, Lung SC, MPD | - | |
| 4p | ALL | Breast, Esophageal Adenocarcinoma, Renal | |
| 4q | ALL | Colorectal, Esophageal Adenocarcinoma, Lung SC, Melanoma, Ovarian, Renal | |

(continued)

| | | Cancer Types | Cancer Types | Oncogene/Tumor |
|---|---|---|---|---|
| | 5p | Esophageal Squamous, HCC, Lung NSC, Lung SC, Renal | - | TERT |
| | 5q | HCC, Renal | Esophageal Adenocarcinoma, Lung NSC | APC |
| | 6p | ALL, HCC, Lung NSC, Melanoma | - | |
| | 6q | ALL | Melanoma, Renal | |
| | 7p | Breast, Colorectal, Esophageal Adenocarcinoma, Glioma, HCC, Lung NSC, Medulloblastoma, Melanoma, Renal | - | EGFR |
| | 7 q | Colorectal, Glioma, HCC, Lung NSC, Medulloblastoma, Melanoma, Prostate, Renal | - | BRAF, MET |
| | 8p | ALL, MPD | Breast, HCC, Lung NSC, Medulloblastoma, Prostate, Renal | |
| | 8q | ALL, Breast, Colorectal, Esophageal Adenocarcinoma, Esophageal Squamous, HCC, Lung NSC, MPD, Ovarian, Prostate | Medulloblastoma | MYC |
| | 9p | MPD | ALL, Breast, Esophageal Adenocarcinoma, Lung NSC, Melanoma, Ovarian, Renal | CDKN2A/B |
| | 9q | ALL, MPD | Lung NSC, Melanoma, Ovarian, Renal | |
| | 10p | ALL | Glioma, Lung SC, Melanoma | |
| | 10q | ALL | (Glioma, Lung SC, Medulloblastoma, Melanoma | PTEN |
| | 11p | - | Medulloblastoma | WT1 |
| | 11q | - | Dedifferentiated Liposarcoma, Medulloblastoma, Melanoma | ATM |
| | 12p | Colorectal, Renal | - | KRAS |
| | 12q | Renal | - | |
| | 113q | Colorectal | Breast, Dedifferentiated Liposarcoma, Glioma, Lung NSC, Ovarian | RB1/BRCA2 |

(continued)

| | | Cancer Types | Cancer Types | Oncogene/Tumor |
|---|---|---|---|---|
| | 14q | ALL, Lung NSC, Lung SC, Prostate | GIST, Melanoma, Renal | |
| | 15q | - | GIST, Lung NSC, Lung SC, (Ovarian | |
| | 16p | Breast | - | |
| | 16q | - | Breast, HCC, Medulloblastoma, Ovarian, Prostate | |
| | 17p | ALL | Breast, Colorectal, Esophageal Adenocarcinoma, HCC, Lung NSC, Lung SC, Ovarian | TP53 |
| | 17q | ALL, HCC, Lung NSC, Medulloblastoma | Breast, Ovarian | ERBB2, NF1/BRCA1 |
| | 18p | ALL, Medulloblastoma | Colorectal, Lung NSC | |
| | 18q | ALL, Medulloblastoma | Colorectal, Esophageal Adenocarcinoma, Lung NSC | SMAD2, SMAD4 |
| | 19p | Glioma | Esophageal Adenocarcinoma, Lung NSC, Melanoma, (Ovarian | |
| | 19q | Glioma, Lung SC | Esophageal Adenocarcinoma, Lung NSC | |
| | 20p | Breast, Colorectal, Esophageal Adenocarcinoma, Esophageal Squamous, GIST, Glioma, HCC, Lung NSC, Melanoma, Renal | - | |
| | 20q | Breast, Colorectal, Ded ifferentiated Liposarcoma, Esophageal Adenocarcinoma, Esophageal Squamous, Glioma, HCC, Lung NSC, Melanoma, Ovarian, Renal | - | |
| | 21q | ALL, GIST, MPD | - | |
| | 22q | Melanoma | Breast, Colorectal, Dedifferentiated Liposarcoma, Esophageal Adenocarcinoma, GIST, Lung NSC, Lung SC, Ovarian, Prostate | NF2 |

The examples of associations between arm level copy number variations are intended to be illustrative and not limiting. Other arm level copy number variations and their cancer associations are known to those of skill in the art.

Other copy number variations that do not span a significant portion of a chromosome or chromosome arm, such as CNVs of 1 kb to 1Mb, or 1 kb to 10 Mb, or 100 kb to 10 Mb, or 1 kb to 50 Mb, or 2 bp to 10 Mb or 2 bp to 50 Mb could equally be informative for the detection or confirmation of the presence of tumor-derived cell-free DNA.

As indicated above, in certain embodiment, the method described herein can be used to determine the presence or absence of a chromosomal amplification. In some embodiments, the chromosomal amplification is the gain of one or more entire chromosomes. In other embodiments, the chromosomal amplification is the gain of one or more segments of a chromosome. In yet other embodiments, the chromosomal amplification is the gain of two or more segments of two or more chromosomes. In various embodiments, the chromosomal amplification can involve the gain of one or more oncogenes.

Dominantly acting genes associated with human solid tumors typically exert their effect by overexpression or altered expression. Gene amplification is a common mechanism leading to upregulation of gene expression. Evidence from cytogenetic studies indicates that significant amplification occurs in over 50% of human breast cancers. Most notably, the amplification of the proto-oncogene human epidermal growth factor receptor 2 (HER2) located on chromosome 17 (17(17q21-q22)), results in overexpression of HER2 receptors on the cell surface leading to excessive and dysregulated signaling in breast cancer and other malignancies (Park et al., Clinical Breast Cancer 8:392-401 [2008]). A variety of oncogenes have been found to be amplified in other human malignancies. Examples of the amplification of cellular oncogenes in human tumors include amplifications of: c-myc in promyelocytic leukemia cell line HL60, and in small-cell lung carcinoma cell lines, N-myc in primary neuroblastomas (stages III and IV), neuroblastoma cell lines, retinoblastoma cell line and primary tumors, and small-cell lung carcinoma lines and tumors, L-myc in small-cell lung carcinoma cell lines and tumors, c-myb in acute myeloid leukemia and in colon carcinoma cell lines, c-erbb in epidermoid carcinoma cell, and primary gliomas, c-K-ras-2 or KRAS in primary carcinomas of lung, colon, bladder, and rectum, N-ras or NRAS in mammary carcinoma cell line (Varmus H., Ann Rev Genetics 18: 553-612 (1984) [cited in Watson et al., Molecular Biology of the Gene (4th ed.; Benjamin/Cummings Publishing Co. 1987)].

Amplifications of oncogenes are a common cause of many types of cancer, as is the case with P70-S6 Kinase 1 amplification and breast cancer. In such cases the genetic amplification occurs in a somatic cell and affects only the genome of the cancer cells themselves, not the entire organism, much less any subsequent offspring. Other examples of oncogenes that are amplified in human cancers include MYC, ERBB2 (EFGR), CCND1 (Cyclin D1), FGFR1 and FGFR2 in breast cancer, MYC and ERBB2 in cervical cancer, HRAS, KRAS, NRAS, and MYB in colorectal cancer, MYC, CCND1 and MDM2 in esophageal cancer, CCNE, KRAS and MET in gastric cancer, ERBB1, and CDK4 in glioblastoma, CCND1, ERBB1, and MYC in head and neck cancer, CCND1 in hepatocellular cancer, MYCB in neuroblastoma, MYC, ERBB2 and AKT2 in ovarian cancer, MDM2 and CDK4 in sarcoma, NRAS in melanoma and MYC in small cell lung cancer. In one embodiment, the present method can be used to determine the presence or absence of amplification of an oncogene associated with a cancer. In some embodiments, the amplified oncogene is associated with breast cancer, cervical cancer, colorectal cancer, esophageal cancer, gastric cancer, glioblastoma, head and neck cancer, hepatocellular cancer, neuroblastoma, ovarian cancer, melanoma, prostate cancer, sarcoma, and small cell lung cancer.

In one embodiment, the present method can be used to determine the presence or absence of a chromosomal deletion. In some embodiments, the chromosomal deletion is the loss of one or more entire chromosomes. In other embodiments, the chromosomal deletion is the loss of one or more segments of a chromosome. In yet other embodiments, the chromosomal deletion is the loss of two or more segments of two or more chromosomes. The chromosomal deletion can involve the loss of one or more tumor suppressor genes.

Chromosomal deletions involving tumor suppressor genes are believed to play an important role in the development and progression of solid tumors. The retinoblastoma tumor suppressor gene (Rb-1), located in chromosome 13q14, is the most extensively characterized tumor suppressor gene. The Rb-1 gene product, a 105 kDa nuclear phosphoprotein, apparently plays an important role in cell cycle regulation (Howe et al., Proc Natl Acad Sci (USA) 87:5883-5887 [1990]). Altered or lost expression of the Rb protein is caused by inactivation of both gene alleles either through a point mutation or a chromosomal deletion. Rbi gene alterations have been found to be present not only in retinoblastomas but also in other malignancies such as osteosarcomas, small cell lung cancer (Rygaard et al., Cancer Res 50: 5312-5317 [1990)]) and breast cancer. Restriction fragment length polymorphism (RFLP) studies have indicated that such tumor types have frequently lost heterozygosity at 13q suggesting that one of the Rb-1 gene alleles has been lost due to a gross chromosomal deletion (Bowcock et al., Am J Hum Genet, 46: 12 [1990]). Chromosome 1 abnormalities including duplications, deletions and unbalanced translocations involving chromosome 6 and other partner chromosomes indicate that regions of chromosome 1, in particular 1q21-1q32 and 1p11-13, might harbor oncogenes or tumor suppressor genes that are pathogenetically relevant to both chronic and advanced phases of myeloproliferative neoplasms (Caramazza et al., Eur J Hematol 84:191-200 [2010]). Myeloproliferative neoplasms are also associated with deletions of chromosome 5. Complete loss or interstitial deletions of chromosome 5 are the most common karyotypic abnormality in myelodysplastic syndromes (MDSs). Isolated del(5q)/5q-MDS patients have a more favorable prognosis than those with additional karyotypic defects, who tend to develop myeloproliferative neoplasms (MPNs) and acute myeloid leukemia. The frequency of unbalanced chromosome 5 deletions has led to the idea that 5q harbors one or more tumor-suppressor genes that have fundamental roles in the growth control of hematopoietic stem/progenitor cells (HSCs/HPCs). Cytogenetic mapping of commonly deleted regions (CDRs) centered on 5q31 and 5q32 identified candidate tumor-suppressor genes, including the ribosomal subunit RPS14, the transcription factor Egr1/Krox20 and the cytoskeletal remodeling protein, alpha-catenin (Eisenmann et al., Oncogene 28:3429-3441 [2009]). Cytogenetic and allelotyping studies of fresh tumors and tumor cell lines have shown that allelic loss from several distinct regions on chromosome 3p, including 3p25, 3p21-22, 3p21.3, 3p12-13 and 3p14, are the earliest and most frequent genomic abnormalities involved in a wide spectrum of major epithelial cancers of lung, breast, kidney, head and neck, ovary, cervix, colon, pancreas, esophagus, bladder and other organs. Several tumor suppressor genes have been mapped to the chromosome 3p region, and are thought that interstitial deletions or promoter hypermethylation precede the loss of the 3p or the entire chromosome 3 in the development of carcinomas (Angeloni D., Briefings Functional Genomics 6:19-39 [2007]).

Newborns and children with Down syndrome (DS) often present with congenital transient leukemia and have an increased risk of acute myeloid leukemia and acute lymphoblastic leukemia. Chromosome 21, harboring about 300 genes, may be involved in numerous structural aberrations, e.g., translocations, deletions, and amplifications, in leukemias, lymphomas, and solid tumors. Moreover, genes located on chromosome 21 have been identified that play an important role in tumorigenesis. Somatic numerical as well as structural chromosome 21 aberrations are associated with leukemias, and specific genes including RUNX1, TMPRSS2, and TFF, which are located in 21q, play a role in tumorigenesis (Fonatsch C Gene Chromosomes Cancer 49:497-508 [2010]).

In view of the foregoing, in various embodiments the method described herein can be used to determine the segment CNVs that are known to comprise one or more oncogenes or tumor suppressor genes, and/or that are known to be associated with a cancer or an increased risk of cancer. In certain embodiments, the CNVs can be determined in a test sample comprising a constitutional (germline) nucleic acid and the segment can be identified in those constitutional nucleic acids. In certain embodiments segment CNVs are identified (if present) in a sample comprising a mixture of nucleic acids (e.g., nucleic acids derived from normal and nucleic acids derived from neoplastic cells). In certain embodiments the sample is derived from a subject that is suspected or is known to have cancer e.g. carcinoma, sarcoma, lymphoma, leukemia, germ cell tumors, blastoma, and the like. In one embodiment, the sample is a plasma sample derived (processed) from peripheral blood that may comprise a mixture of cfDNA derived from normal and cancerous cells. In another embodiment, the biological sample that is used to determine whether a CNV is present is derived from a cells that, if a cancer is present, comprises a mixture of cancerous and non-cancerous cells from other biological tissues including, but not limited to biological fluids such as serum, sweat, tears, sputum, urine, sputum, ear flow, lymph, saliva, cerebrospinal fluid, ravages, bone marrow suspension, vaginal flow, transcervical lavage, brain fluid, ascites, milk, secretions of the respiratory, intestinal and genitourinary tracts, and leukophoresis samples, or in tissue biopsies, swabs, or smears. In other embodiments, the biological sample is a stool (fecal) sample.

The CNVs used to determine presence of a cancer and/or increased risk for a cancer can comprise amplification or deletions.

In various embodiments the CNVs identified as indicative of the presence of a cancer or an increased risk for a cancer include one or more of the amplifications shown in Table 4.

**Table 4 Illustrative, but non-limiting chromosomal segments characterized by amplifications that are associated with cancers.**

| Peak region | Length (Mb) | Cancer types identified in this analysis but not prior publications |
|---|---|---|
| chr1: 119996566- | 0.228 | Breast, Lung SC, Melanoma |
| 1120303234 | | |
| chr1: 148661965- | 0.35 | Breast, Dedifferentiated liposarcoma, |
| 1149063439 | | Esophageal adenocarcinoma, |
| | | Hepatocellular, Lung SC, Melanoma, |
| | | Ovarian, Prostate, Renal |
| chr1: 1-5160566 | 4.416 | Esophageal adenocarcinoma, Ovarian |
| chr1: 158317017- | 1.627 | Dedifferentiated liposarcoma, Esophageal |
| 1159953843 | | adenocarcinoma, Prostate, Renal |

(continued)

| Peak region | Length (Mb) | Cancer types identified in this analysis but not prior publications |
|---|---|---|
| chr1: 169549478-1170484405 | 0.889 | Colorectal, Dedifferentiated liposarcoma, Prostate, Renal |
| chr1: 201678483-1203358272 | 1.471 | Prostate |
| chr1: 241364021-1247249719 | 5.678 | Lung NSC, Melanoma, Ovarian |
| chr1: 39907605-140263248 | 0.319 | Acute lymphoblastic leukemia, Breast, Lung NSC, Lung SC |
| chr1: 58658784-160221344 | 1.544 | Breast, Dedifferentiated liposarcoma, Lung SC |
| chr3: 170024984-1173604597 | 3.496 | Breast, Esophageal adenocarcinoma, Glioma |
| chr3: 178149984-1199501827 | 21.123 | Esophageal squamous, Lung NSC |
| chr3: 86250885-195164178 | 8.795 | Lung SC, Melanoma |
| chr4: 54471680-55980061 | 1.449 | Lung NSC |
| chr5: 1212750-1378766 | 0.115 | Dedifferentiated liposarcoma |
| chr5: 174477192-180857866 | 6.124 | Breast, Lung NSC |
| chr5: 45312870-149697231 | 4.206 | Lung SC |
| chr6: 1-23628840 | 23.516 | Esophageal adenocarcinoma |
| chr6: 135561194-1135665525 | 0.092 | Breast, Esophageal adenocarcinoma |
| chr6: 43556800-44361368 | 0.72 | Esophageal adenocarcinoma, Hepatocellular, Ovarian |
| chr6: 63255006-65243766 | 1.988 | Esophageal adenocarcinoma, Lung NSC |
| chr7: 115981465-116676953 | 0.69 | Esophageal adenocarcinoma, Lung NSC, Melanoma, Ovarian |
| chr7: 54899301-55275419 | 0.363 | Esophageal adenocarcinoma, Esophageal squamous |
| chr7: 89924533-98997268 | 9.068 | Breast, Esophageal adenocarcinoma, Esophageal squamous, Ovarian |

(continued)

| Peak region | Length (Mb) | Cancer types identified in this analysis but not prior publications |
|---|---|---|
| chr8: 101163387- 103693879 | 2.516 | Lung NSC, Melanoma, Ovarian |
| chr8: 116186189- 120600761 | 4.4 | Breast, Hepatocellular, Lung NSC, Ovarian |
| chr8: 128774432- 128849112 | 0.009 | Esophageal adenocarcinoma, Esophageal squamous, Hepatocellular, Lung SC, Medulloblastoma, Myeloproliferative disorder, Ovarian |
| chr8: 140458177- 146274826 | 5.784 | Lung NSC, Medulloblastoma, Melanoma, (Ovarian |
| chr8: 38252951- 38460772 | 0.167 | Colorectal, Esophageal adenocarcinoma, Esophageal squamous |
| chr8: 42006632- 42404492 | 0.257 | Esophageal adenocarcinoma, Lung NSC, Lung SC, Ovarian, Prostate |
| chr8: 81242335- 81979194 | 0.717 | Breast, Melanoma |
| chr9: 137859478- 140273252 | 2.29 | Colorectal, Dedifferentiated liposarcoma |
| chr10: 74560456- 82020637 | 7.455 | Breast, Ovarian, Prostate |
| chr11: 101433436- 102134907 | 0.683 | Lung NSC, Lung SC |
| chr11: 32027116- 37799354 | 5.744 | Breast, Dedifferentiated liposarcoma, Lung NSC, Lung SC |
| chr11: 69098089- 69278404 | 0.161 | Dedifferentiated liposarcoma, Esophageal adenocarcinoma, Hepatocellular, Lung SC, Ovarian |
| chr11: 76699529- 78005085 | 1.286 | Dedifferentiated liposarcoma, Esophageal adenocarcinoma, Lung SC, Ovarian |
| chr12: 1-1311104 | 1.271 | Lung NSC |
| chr12: 25189655- 25352305 | 0.112 | Acute lymphoblastic leukemia, Esophageal adenocarcinoma, Esophageal squamous, Ovarian |
| chr12: 30999223- 32594050 | 1.577 | Acute lymphoblastic leukemia, Colorectal, Esophageal adenocarcinoma, Esophageal squamous, Lung NSC, Lung SC |
| chr12: 38788913- | 3.779 | Breast, Colorectal, Dedifferentiated |

(continued)

| Peak region | Length (Mb) | Cancer types identified in this analysis but not prior publications |
|---|---|---|
| 42596599 | | liposarcoma, Esophageal squamous, Lung NSC, Lung SC |
| chr12: 56419524- 56488685 | 0.021 | Dedifferentiated liposarcoma, Melanoma, Renal |
| chr12: 64461446- 64607139 | 0.041 | Dedifferentiated liposarcoma, Renal |
| chr12: 66458200- 66543552 | 0.058 | Dedifferentiated liposarcoma, Esophageal squamous, Renal |
| chr12: 67440273- 67566002 | 0.067 | Breast, Dedifferentiated liposarcoma, Esophageal squamous, Melanoma, Renal |
| chr12: 68249634- 68327233 | 0.06 | Breast, Dedifferentiated liposarcoma, Esophageal squamous, Renal |
| chr12: 70849987- 70966467 | 0.036 | Dedifferentiated liposarcoma, Renal |
| chr12: 72596017- 73080626 | 0.23 | Renal |
| chr12: 76852527- 77064746 | 0.158 | Dedifferentiated liposarcoma |
| chr12: 85072329- 85674601 | 0.272 | Dedifferentiated liposarcoma |
| chr12: 95089777- 95350380 | 0.161 | Dedifferentiated liposarcoma |
| chr13: 108477140- 110084607 | 1.6 | Breast, Esophageal adenocarcinoma, Lung NSC, Lung SC |
| chr13: 1-40829685 | 22.732 | Acute lymphoblastic leukemia, Esophageal adenocarcinoma |
| chr13: 89500014- 93206506 | 3.597 | Breast, Esophageal adenocarcinoma, Medulloblastoma |
| chr14: 106074644- 106368585 | 0.203 | Esophageal squamous |
| chr14: 1-23145193 | 3.635 | Acute lymphoblastic leukemia, Esophageal squamous, Hepatocellular, Lung SC |
| chr14: 35708407- 36097605 | 0.383 | Breast, Esophageal adenocarcinoma, Esophageal squamous, Hepatocellular, Prostate |
| chr15: 96891354- | 0.778 | Breast, Colorectal, Esophageal |

| Peak region | Length (Mb) | Cancer types identified in this analysis but not prior publications |
|---|---|---|
| 97698742 | | adenocarcinoma, Lung NSC, |
| | | Medulloblastoma, Melanoma |
| chr17: 18837023- | 0.815 | Breast, Hepatocellular |
| 19933105 | | |
| chr17: 22479313- | 0.382 | Breast, Lung NSC |
| 22877776 | | |
| chr17: 24112056- | 0.114 | Breast, Lung NSC |
| 24310787 | | |
| chr17: 35067383- | 0.149 | Colorectal, Esophageal adenocarcinoma, |
| 35272328 | | Esophageal squamous |
| chr17: 44673157- | 0.351 | Melanoma |
| 45060263 | | |
| chr17: 55144989- | 0.31 | Lung NSC, Medulloblastoma, Melanoma, |
| 55540417 | | Ovarian |
| chr17: 62318152- | 1.519 | Breast, Lung NSC, Melanoma, Ovarian |
| 63890591 | | |
| chr17: 70767943- | 0.537 | Breast, Lung NSC, Melanoma, Ovarian |
| 71305641 | | |
| chr18: 17749667- | 5.029 | Colorectal, Esophageal adenocarcinoma, |
| 22797232 | | Ovarian |
| chr19: 34975531- | 0.096 | Breast, Esophageal adenocarcinoma, |
| 35098303 | | Esophageal squamous |
| chr19: 43177306- | 2.17 | Lung NSC, Ovarian |
| 45393020 | | |
| chr19: 59066340- | 0.321 | Breast, Lung NSC, Ovarian |
| 59471027 | | |
| chr2: 15977811- | 0.056 | Lung SC |
| 16073001 | | |
| chr20: 29526118- | 0.246 | Ovarian |
| 29834552 | | |
| chr20: 51603033- | 0.371 | Hepatocellular, Lung NSC, Ovarian |
| 51989829 | | |
| chr20: 61329497- | 0.935 | Hepatocellular, Lung NSC |
| 62435964 | | |
| chr22: 19172385- | 0.487 | Colorectal, Melanoma, Ovarian |
| 19746441 | | |
| chrX: 152729030- | 1.748 | Breast, Lung NSC, Renal |

(continued)

| Peak region | Length (Mb) | Cancer types identified in this analysis but not prior publications |
|---|---|---|
| 154913754 | | |
| chrX: 66436234- | 0.267 | Ovarian, Prostate |

In certain embodiments in combination with the amplifications described above (herein), or separately, the CNVs identified as indicative of the presence of a cancer or an increased risk for a cancer include one or more of the deletions shown in Table 5.

**Table 5 Illustrative but not limiting chromsomal segments characterized by deletions that are associated with cancers.**

| Peak region | Length (Mb) | Cancer types identified in this analysis but not prior publications |
|---|---|---|
| chr1: 110339388-119426489 | 1p13.2 | Acute lymphoblastic leukemia, |
| | | Esophageal adenocarcinoma, Lung NSC, |
| | | Lung SC, Melanoma, Ovarian, Prostate |
| chr1: 223876038-247249719 | 1q43 | Acute lymphoblastic leukemia, Breast, |
| | | Lung SC, Melanoma, Prostate |
| chr1: 26377344-27532551 | 1p36.11 | Breast, Esophageal adenocarcinoma, |
| | | Esophageal squamous, Lung NSC, Lung |
| | | SC, Medulloblastoma, Myeloproliferative |
| | | disorder, Ovarian, Prostate |
| chr1: 3756302-6867390 | 1p36.31 | Acute lymphoblastic leukemia, Breast, |
| | | Esophageal squamous, Hepatocellular, |
| | | Lung NSC, Lung SC, Medulloblastoma, |
| | | Myeloproliferative disorder, Ovarian, |
| | | Prostate, Renal |
| chr1: 71284749-74440273 | 1p31.1 | Breast, Esophageal adenocarcinoma, |
| | | Glioma, Hepatocellular, Lung NSC, Lung |
| | | SC, Melanoma, Ovarian, Renal |
| chr2: 1-15244284 | 2p25.3 | Lung NSC, Ovarian |
| chr2: 138479322-143365272 | 2q22.1 | Breast, Colorectal, Esophageal |
| | | adenocarcinoma, Esophageal squamous, |
| | | Hepatocellular, Lung NSC, Ovarian, |
| | | Prostate, Renal |
| chr2: 204533830-206266883 | 2q33.2 | Esophageal adenocarcinoma, |
| | | Hepatocellular, Lung NSC, |
| | | Medulloblastoma, Renal |
| chr2: 241477619-242951149 | 2q37.3 | Breast, Dedifferentiated liposarcoma, |
| | | Esophageal adenocarcinoma, Esophageal |
| | | squamous, Hepatocellular, Lung NSC, |

(continued)

| Peak region | Length (Mb) | Cancer types identified in this analysis but not prior publications |
|---|---|---|
| | | Lung SC, Medulloblastoma, Melanoma, |
| | | Ovarian, Renal |
| chr3: 116900556-120107320 | 3q13.31 | Dedifferentiated liposarcoma, |
| | | Esophageal adenocarcinoma, Hepatocellular, |
| | | Lung NSC, Melanoma, Myeloproliferative |
| | | disorder, Prostate |
| chr3: 1-2121282 | 3p26.3 | Colorectal, Dedifferentiated liposarcoma, |
| | | Esophageal adenocarcinoma, Lung NSC, |
| | | Melanoma, Myeloproliferative disorder |
| | 3q26.31 | Acute lymphoblastic leukemia, |
| chr3: 175446835-178263192 | | Dedifferentiated liposarcoma, Esophageal |
| | | adenocarcinoma, Lung NSC, Melanoma, |
| | | Myeloproliferative disorder, Prostate |
| chr3: | 3p14.2 | Breast, Colorectal, Dedifferentiated |
| 58626894-61524607 | | liposarcoma, Esophageal adenocarcinoma, |
| | | Esophageal squamous, Hepatocellular, |
| | | Lung NSC, Lung SC, Medulloblastoma, |
| | | Melanoma, Myeloproliferative disorder, |
| | | Ovarian, Prostate, Renal |
| chr4: 1-435793 | 4p16.3 | Myeloproliferative disorder |
| chr4: 186684565-191273063 | 4q35.2 | Breast, Esophageal adenocarcinoma, |
| | | Esophageal squamous, Lung NSC, |
| | | Medulloblastoma, Melanoma, Prostate, |
| | | Renal |
| chr4: 91089383-93486891 | 4q22.1 | Acute lymphoblastic leukemia, Esophageal |
| | | adenocarcinoma, Hepatocellular, Lung |
| | | NSC, Renal |
| chr5: 177541057-180857866 | 5q35.3 | Breast, Lung NSC, Myeloproliferative |
| | | disorder, Ovarian |
| chr5: 57754754-59053198 | 5q11.2 | Breast, Colorectal, Dedifferentiated |
| | | liposarcoma, Esophageal adenocarcinoma, |
| | | Esophageal squamous, Lung SC, |
| | | Melanoma, Myeloproliferative disorder, |
| | | Ovarian, Prostate |
| chr5: 85837489-133480433 | 5q21.1 | Colorectal, Dedifferentiated liposarcoma, |
| | | Lung NSC, Lung SC, Myeloproliferative |
| | | disorder, Ovarian |

(continued)

| Peak region | Length (Mb) | Cancer types identified in this analysis but not prior publications |
|---|---|---|
| chr6: 101000242-121511318 | 6q22.1 | Colorectal, Lung NSC, Lung SC |
| | | |
| chr6: 1543157-2570302 | 6p25.3 | Colorectal, Dedifferentiated liposarcoma, |
| | | Esophageal adenocarcinoma, Lung NSC, |
| | | Lung SC, Ovarian, Prostate |
| chr6: 161612277-163134099 | 6q26 | Colorectal, Esophageal adenocarcinoma, |
| | | Esophageal squamous, Lung NSC, Lung |
| | | SC, Ovarian, Prostate |
| chr6: 76630464-105342994 | 6q16.1 | Colorectal, Hepatocellular, Lung NSC |
| | | |
| chr7: 141592807-142264966 | 7q34 | Breast, Colorectal, Esophageal |
| | | adenocarcinoma, Esophageal squamous, |
| | | Hepatocellular, Lung NSC, Ovarian, |
| | | Prostate, Renal |
| chr7: 144118814-148066271 | 7q35 | Breast, Esophageal adenocarcinoma, |
| | | Esophageal squamous, Lung NSC, |
| | | Melanoma, Myeloproliferative disorder, |
| | | Ovarian |
| chr7: 156893473-158821424 | 7q36.3 | Breast, Esophageal adenocarcinoma, |
| | | Esophageal squamous, Lung NSC, |
| | | Melanoma, Myeloproliferative disorder, |
| | | Ovarian, Prostate |
| chr7: 3046420-4279470 | 7p22.2 | Melanoma, Myeloproliferative disorder, |
| | | Ovarian |
| chr7: 65877239-79629882 | 7q21.11 | Breast, Medulloblastoma, Melanoma, |
| | | Myeloproliferative disorder, Ovarian |
| chr8: 1-392555 | 8p23.3 | Acute lymphoblastic leukemia, Breast, |
| | | Myeloproliferative disorder |
| chr8: 2053441-6259545 | 8p23.2 | Acute lymphoblastic leukemia, |
| | | Dedifferentiated liposarcoma, Esophageal |
| | | adenocarcinoma, Esophageal squamous, |
| | | Hepatocellular, Lung NSC, |
| | | Myeloproliferative disorder |
| chr8: 22125332-30139123 | 8p21.2 | Acute lymphoblastic leukemia, |
| | | Dedifferentiated liposarcoma, |
| | | Hepatocellular, Myeloproliferative |
| | | disorder, Ovarian, Renal |

(continued)

| Peak region | Length (Mb) | Cancer types identified in this analysis but not prior publications |
|---|---|---|
| chr8: 39008109-41238710 | 8p11.22 | Acute lymphoblastic leukemia, Breast, |
| | | Dedifferentiated liposarcoma, Esophageal |
| | | squamous, Hepatocellular, Lung NSC, |
| | | Myeloproliferative disorder, Renal |
| chr8: 42971602-72924037 | 8q11.22 | Breast, Dedifferentiated liposarcoma, |
| | | Esophageal squamous, Hepatocellular, |
| | | Lung NSC, Myeloproliferative disorder, |
| | | Renal |
| chr9: 1-708871 | 9p24.3 | Acute lymphoblastic leukemia, Breast, |
| | | Lung NSC, Myeloproliferative disorder, |
| | | Ovarian, Prostate |
| chr9: 21489625-22474701 | 9p21.3 | Colorectal, Esophageal adenocarcinoma, |
| | | Esophageal squamous, Myeloproliferative |
| | | disorder, Ovarian |
| chr9: 36365710-37139941 | 9p13.2 | Myeloproliferative disorder |
| | | |
| chr9: 7161607-12713130 | 9p24.1 | Acute lymphoblastic leukemia, Breast, |
| | | Colorectal, Esophageal adenocarcinoma, |
| | | Hepatocellular, Lung SC, |
| | | Medulloblastoma, Melanoma, |
| | | Myeloproliferative disorder, Ovarian, |
| | | Prostate, Renal |
| chr10: 1-1042949 | 10p15.3 | Colorectal, Lung NSC, Lung SC, Ovarian, |
| | | Prostate, Renal |
| chr10: 129812260-135374737 | 10q26.3 | Breast, Colorectal, Glioma, Lung NSC, |
| | | Lung SC, Melanoma, Ovarian, Renal |
| chr10: 52313829-53768264 | 10q11.23 | Colorectal, Lung NSC, Lung SC, Ovarian, |
| | | Renal |
| chr10: 89467202-90419015 | 10q23.31 | Breast, Lung SC, Ovarian, Renal |
| | | |
| chr11: 107086196-116175885 | 11q23.1 | Esophageal adenocarcinoma, |
| | | Medulloblastoma, Renal |
| chr11: 1-1391954 | 11p15.5 | Breast, Dedifferentiated liposarcoma, |
| | | Esophageal adenocarcinoma, Lung NSC, |
| | | Medulloblastoma, Ovarian |
| chr11: 130280899-134452384 | 11q25 | Esophageal adenocarcinoma, Esophageal |
| | | squamous, Hepatocellular, Lung NSC, |

(continued)

| Peak region | Length (Mb) | Cancer types identified in this analysis but not prior publications |
|---|---|---|
| | | Medulloblastoma, Renal |
| chr11: 82612034-85091467 | 111q14.1 | Melanoma, Renal |
| | | |
| chr12: 11410696-12118386 | 12p13.2 | Breast, Hepatocellular, Myeloproliferative |
| | | disorder, Prostate |
| | 12q24.33 | Dedifferentiated liposarcoma, Lung NSC, |
| chr12: 131913408-132349534 | | Myeloproliferative disorder |
| chr12: 97551177-99047626 | 12q23.1 | Breast, Colorectal, Esophageal squamous, |
| | | Lung NSC, Myeloproliferative disorder |
| chr13: 111767404-114142980 | 13q34 | Breast, Hepatocellular, Lung NSC |
| | | |
| chr13: 1-23902184 | 13q12.11 | Breast, Lung SC, Ovarian |
| chr13: 46362859-48209064 | 13q14.2 | Hepatocellular, Lung SC, |
| | | Myeloproliferative disorder, Prostate |
| chr13: 92308911-94031607 | 13q31.3 | Breast, Hepatocellular, Lung NSC, Renal |
| | | |
| chr14: 1-29140968 | 14q11.2 | Acute lymphoblastic leukemia, Esophageal |
| | | adenocarcinoma, Myeloproliferative |
| | | disorder |
| chr14: 65275722-67085224 | 14q23.3 | Dedifferentiated liposarcoma, |
| | | Myeloproliferative disorder |
| chr14: 80741860-106368585 | 14q32.12 | Acute lymphoblastic leukemia, |
| | | Dedifferentiated liposarcoma, Melanoma, |
| | | Myeloproliferative disorder |
| chr15: 1-24740084 | 15q11.2 | Acute lymphoblastic leukemia, Breast, |
| | | Esophageal adenocarcinoma, Lung NSC, |
| | | Myeloproliferative disorder, Ovarian |
| chr15: 35140533-43473382 | 15q15.1 | Esophageal adenocarcinoma, Lung NSC, |
| | | Myeloproliferative disorder |
| chr16: 1-359092 | 16p13.3 | Esophageal adenocarcinoma, |
| | | Hepatocellular, Lung NSC, Renal |
| chr16: 31854743-53525739 | 16q11.2 | Breast, Hepatocellular, Lung NSC, |
| | | Melanoma, Renal |
| chr16: 5062786-7709383 | 16p13.3 | Hepatocellular, Lung NSC, |
| | | Medulloblastoma, Melanoma, |
| | | Myeloproliferative disorder, Ovarian, |
| | | Renal |

(continued)

| Peak region | Length (Mb) | Cancer types identified in this analysis but not prior publications |
|---|---|---|
| | 16q23.1 | Breast, Colorectal, Esophageal |
| chr16: 76685816-78205652 | | adenocarcinoma, Hepatocellular, Lung |
| | | NSC, Lung SC, Medulloblastoma, Renal |
| chr16: 80759878-82408573 | 16q23.3 | Colorectal, Hepatocellular, Renal |
| | | |
| chr16: 88436931-88827254 | 16q24.3 | Colorectal, Hepatocellular, Lung NSC, |
| | | Prostate, Renal |
| chr17: 10675416-12635879 | 17p12 | Lung NSC, Lung SC, Myeloproliferative |
| | | disorder |
| chr17: 26185485-27216066 | 17q11.2 | Breast, Colorectal, Dedifferentiated |
| | | liposarcoma, Lung NSC, Lung SC, |
| | | Melanoma, Myeloproliferative disorder, |
| | | Ovarian |
| chr17: 37319013-37988602 | 17q21.2 | Breast, Colorectal, Dedifferentiated |
| | | liposarcoma, Lung SC, Melanoma, |
| | | Myeloproliferative disorder, Ovarian |
| chr17: 7471230-7717938 | 17p13.1 | Lung SC, Myeloproliferative disorder |
| | | |
| chr17: 78087533-78774742 | 17q25.3 | Colorectal, Myeloproliferative disorder |
| | | |
| chr18: 1-587750 | 18p11.32 | Myeloproliferative disorder |
| chr18: 46172638-49935241 | 18q21.2 | Esophageal adenocarcinoma, Lung NSC |
| | | |
| chr18: 75796373-76117153 | 18q23 | Colorectal, Esophageal adenocarcinoma, |
| | | Esophageal squamous, Ovarian, Prostate |
| chr19: 1-526082 | 19p13.3 | Hepatocellular, Lung NSC, Renal |
| chr19: 21788507-34401877 | 19p12 | Hepatocellular, Lung NSC, Renal |
| | | |
| chr19: 52031294-53331283 | 19q13.32 | Breast, Hepatocellular, Lung NSC, |
| | | Medulloblastoma, Ovarian, Renal |
| chr19: 63402921-63811651 | 19q13.43 | Breast, Colorectal, Dedifferentiated |
| | | liposarcoma, Hepatocellular, Lung NSC, |
| | | Medulloblastoma, Ovarian, Renal |
| chr20: 1-325978 | 20p13 | Breast, Dedifferentiated liposarcoma, Lung |
| | | NSC |
| chr20: 14210829-15988895 | 20p12.1 | Esophageal adenocarcinoma, Lung NSC, |
| | | Medulloblastoma, Melanoma, |

(continued)

| Peak region | Length (Mb) | Cancer types identified in this analysis but not prior publications |
|---|---|---|
| | | Myeloproliferative disorder, Prostate, |
| | | Renal |
| chr21: 38584860-42033506 | 21q22.2 | Breast |
| | | |
| chr22: 20517661-21169423 | 22q11.22 | Acute lymphoblastic leukemia, Esophageal |
| | | adenocarcinoma |
| chr22: 45488286-49691432 | 22q13.33 | Breast, Hepatocellular, Lung NSC, Lung |
| | | SC |
| chrX: 1-3243111 | Xp22.33 | Esophageal adenocarcinoma, Lung NSC, |
| | | Lung SC |
| chrX: 31041721-34564697 | Xp21.2 | Acute lymphoblastic leukemia, Esophageal |
| | | adenocarcinoma, Glioma |

The aneuploidies identified as characteristic of various cancers (e.g., the aneuploidies identified in Tables 4 and 5) may contain genes known to be implicated in cancer etiologies (e.g., tumor suppressors, oncogenes, etc.). These aneuploidies can also be probed to identify relevant but previously unknown genes.

Table 6 illustrates target genes known to be within the identified amplified segment and predicted genes, and Table 6 illustrates target genes known to be within the identified deleted segment and predicted genes.

**Table 6 Illustrative, but non-limiting chromosomal segments and genes known or predicted to be present in regions characterized by amplification in various cancers**

| Chromosome and band | Peak region | # genes | Known target | Grail top target |
|---|---|---|---|---|
| 8q24.21 | chr8: 128774432-128849112 | 1 | MFC | MYC |
| | | | | |
| 11q13.2 | chr11: 69098089-69278404 | 3 | CCND1 | ORAOV1 |
| | | | | |
| 17q12 | chr17: 35067383-35272328 | 6 | ERBB2 | ERBB2, |
| | | | | C17orf37 |
| 12q14.1 | chr12: 56419524-56488685 | 7 | CDK4 | TSPAN31 |
| | | | | |
| 14q13.3 | chr14: 35708407-36097605 | 3 | NKX2-1 | NKX2-1 |
| | | | | |
| 12q15 | chr12: 67440273-67566002 | 1 | MDM2 | MDM2 |
| | | | | |
| 7p11.2 | chr7: 54899301-55275419 | 1 | EGFR | EGFR |
| | | | | |
| 1q21.2 | chr1: 148661965-149063439 | 9 | MCL1± | MCL1 |
| | | | | |

(continued)

| Chromosome and band | Peak region | # genes | Known target | Grail top target |
|---|---|---|---|---|
| 8p12 | chr8: 38252951-38460772 | 3 | FGFR1 | FGFR1 |
| 12p12.1 | chr12: 25189655-25352305 | 2 | KRAS | KRAS |
| 19q12 | chr19: 34975531-35098303 | 1 | CCNE1 | CCNE1 |
| 22q11.21 | chr22: 19172385-19746441 | 11 | CRKL | CRKL |
| 12q15 | chr12: 68249634-68327233 | 2 | | LRRC10 |
| 12q14.3 | chr12: 64461446-64607139 | 1 | HMGA2 | HMGA2 |
| Xq28 | chrX: 152729030-154913754 | 53 | | SPRY3 |
| 5p15.33 | chr5: 1212750-1378766 | 3 | TERT | TERT |
| 3q26.2 | chr3: 170024984-173604597 | 22 | PRKCI | |
| 15q26.3 | chr15: 96891354-97698742 | 4 | IGF1R | IGF1R |
| 20q13.2 | chr20: 51603033-51989829 | 1 | | ZNF217 |
| 8p11.21 | chr8: 42006632-42404492 | 6 | | PLAT |
| 1p34.2 | chr1: 39907605-40263248 | 7 | MYCL1 | MYCL1 |
| 17q21.33 | chr17: 44673157-45060263 | 4 | | NGFR, PHB |
| 2p24.3 | chr2: 15977811-16073001 | 1 | MYCN | MYCN |
| 7q21.3 | chr7: 89924533-98997268 | 62 | CDK6 | CDK6 |
| 13q34 | chr13: 108477140-110084607 | 4 | | IRS2 |
| 11q14.1 | chr11: 76699529-78005085 | 14 | | GAB2 |

(continued)

| Chromosome and band | Peak region | # genes | Known target | Grail top target |
|---|---|---|---|---|
| 20q13.33 | chr20: 61329497-62435964 | 38 | | BIRC7 |
| 17q23.1 | chr17: 55144989-55540417 | 5 | | RPS6KB1 |
| 1p12 | chr1: 119996566-120303234 | 5 | | REG4 |
| 8q21.13 | chr8: 81242335-81979194 | 3 | | ZNF704, ZBTB10 |
| 6p21.1 | chr6: 43556800-44361368 | 18 | | VEGFA |
| 5p11 | chr5: 45312870-49697231 | 0 | | |
| 120q11.21 | chr20: 29526118-29834552 | 5 | BCL2L1‡ | BCL2L1, ID1 |
| 6q23.3 | chr6: 135561194-135665525 | | MYB** | hsa-mir-548a-2 |
| 1q44 | chr1: 241364021-247249719 | 71 | | AKT3 |
| 5q35.3 | chr5: 174477192-180857866 | 92 | | FLT4 |
| 7q31.2 | chr7: 115981465-116676953 | 3 | MET | MET |
| 18q11.2 | chr18: 17749667-22797232 | 21 | | CABLES1 |
| 17q25.1 | chr17: 70767943-71305641 | 13 | | GRB2, ITGB4 |
| 1p32.1 | chr1: 58658784-60221344 | 7 | JUN | JUN |
| 17q11.2 | chr17: 24112056-24310787 | 5 | | DHRS13, FLOT2, ERAL1, PHF12 |
| 17p11.2 | chr17: 18837023-19933105 | 12 | | MAPK7 |
| 8q24.11 | chr8: 116186189-120600761 | 13 | | NOV |

(continued)

| Chromosome and band | Peak region | # genes | Known target | Grail top target |
|---|---|---|---|---|
| 12q15 | chr12: 66458200-66543552 | 0 | | |
| 19q13.2 | chr19: 43177306-45393020 | 60 | | LGALS7, DYRK1B |
| 11q22.2 | chrll: 101433436-102134907 | 8 | BIRC2, YAP1 | BIRC2 |
| 4q12 | chr4: 54471680-55980061 | 7 | PDGFRA, KIT | KDR, KIT |
| 12p11.21 | chr12: 30999223-32594050 | 9 | | DDX11, FAM60A |
| 3q28 | chr3: 178149984-199501827 | 143 | PIK3CA | PIK3CA |
| 1p36.33 | chr1: 1-5160566 | 77 | | TP73 |
| 17q24.2 | chr17: 62318152-63890591 | 12 | | BPTF |
| 1q23.3 | chr1: 158317017-159953843 | 52 | | PEA15 |
| 1q24.3 | chr1: 169549478-170484405 | 6 | | BAT2D1, MYOC |
| 8q22.3 | chr8: 101163387-103693879 | 14 | | RRM2B |
| 13q31.3 | chr13: 89500014-93206506 | 3 | | GPC5 |
| 12q21.1 | chr12: 70849987-70966467 | 0 | | |
| 12p13.33 | chr12: 1-1311104 | 10 | | WNK1 |
| 12q21.2 | chr12: 76852527-77064746 | 0 | | |
| 1q32.1 | chrl: 201678483-203358272 | 21 | MDM4 | MDM4 |
| 19q13.42 | chr19: 59066340-59471027 | 19 | | PRKCG, TSEN34 |
| 12q12 | chr12: 38788913-42596599 | 12 | | ADAMTS20 |
| 12q23.1 | chr12: 95089777-95350380 | 2 | | ELK3 |

(continued)

| Chromosome and band | Peak region | # genes | Known target | Grail top target |
|---|---|---|---|---|
| 12q21.32 | chr12: 85072329-85674601 | 0 | | |
| 10q22.3 | chr10: 74560456-82020637 | 46 | | SFTPA1B |
| 3p11.1 | chr3: 86250885-95164178 | 8 | | POU1F1 |
| 17q11.1 | chr17: 22479313-22877776 | 1 | | WSB1 |
| 8q24.3 | chr8: 140458177-146274826 | 97 | | PTP4A3, MAFA, PARP10 |
| Xq12 | chrX: 66436234-67090514 | 1 | AR | AR |
| 6q12 | chr6: 63255006-65243766 | 3 | | PTP4A1 |
| 14q11.2 | chr14: 1-23145193 | 95 | | BCL2L2 |
| 9q34.3 | chr9: 137859478-140273252 | 76 | | NRARP, MRPL41, TRAF2, LHX3 |
| 6p24.1 | chr6: 1-23628840 | 95 | | E2F3 |
| 13q12.2 | chr13: 1-40829685 | 110 | | FOXO1 |
| 12q21.1 | chr12: 72596017-73080626 | 0 | | |
| 14q32.33 | chr14: 106074644-106368585 | 0 | | |
| 11p13 | chr11: 32027116-37799354 | 35 | | WT1 |

**Table 7 Illustrative, but non-limiting chromosomal segments and genes known or predicted to be present in regions characterized by amplification in various cancers**

| Chromosome and band | Peak region | # genes | Known target | Grail top target |
|---|---|---|---|---|
| 9p21.3 | chr9: 21489625-22474701 | 5 | CDKN2A/B | CDKN2A |
| 3p14.2 | chr3: 58626894-61524607 | 2 | FHIT§ | FHIT |

(continued)

| Chromosome and band | Peak region | # genes | Known target | Grail top target |
|---|---|---|---|---|
| 16q23.1 | chr16: 76685816-78205652 | 2 | WWOX§ | WWOX |
| 9p24.1 | chr9: 7161607-12713130 | 3 | PTPRD§ | PTPRD |
| 20p12.1 | chr20: 14210829-15988895 | 2 | MACROD2§ | FLRT3 |
| 6q26 | chr6: 161612277-163134099 | 1 | PARK2§ | PARK2 |
| 13q14.2 | chr13: 46362859-48209064 | 8 | RB1 | RB1 |
| 2q22.1 | chr2: 138479322-143365272 | 3 | LRP1B§ | LRP1B |
| 4q35.2 | chr4: 186684565-191273063 | 15 | | FRG2, TUBB4Q |
| 5q11.2 | chr5: 57754754-59053198 | 5 | PDE4D§ | PLK2, PDE4D |
| 16p13.3 | chr16: 5062786-7709383 | 2 | A2BP1§ | A2BP1 |
| 7q34 | chr7: 141592807-142264966 | 3 | TRB@{circumflex over ()} | PRSS1 |
| 2q37.3 | chr2: 241477619-242951149 | 19 | | TMEM 16G, ING5 |
| 19p13.3 | chr19: 1-526082 | 10 | | GZMM, THEG, PPAP2C, C 19orf20 |
| 10q23.31 | chr 10: 89467202-90419015 | 4 | PTEN | PTEN |
| 8p23.2 | chr8: 2053441-6259545 | 1 | CSMD1§ | CSMD1 |
| 1p36.31 | chr1: 3756302-6867390 | 23 | | DFFB, ZBTB48, AJAP1 |
| 4q22.1 | chr4: 91089383-93486891 | 2 | | MGC48628 |
| 18q23 | chr18: 75796373-76117153 | 4 | | PARD6G |

(continued)

| Chromosome and band | Peak region | # genes | Known target | Grail top target |
|---|---|---|---|---|
| 6p25.3 | chr6: 1543157-2570302 | 2 | | FOXC1 |
| 19q13.43 | chr19: 63402921-63811651 | 17 | | ZNF324 |
| Xp21.2 | chrX: 31041721-34564697 | 2 | DMD§ | DMD |
| 11q25 | chr11: 130280899-134452384 | 12 | OPCML§, HNT§ | HNT |
| 13q12.11 | chr13: 1-23902184 | 29 | | LATS2 |
| 22q13.33 | chr22 : 45488286-49691432 | 38 | | TUBGCP6 |
| 15q11.2 | chr15: 1-24740084 | 20 | | A26B1 |
| 22q11.22 | chr22 : 20517661-21169423 | 3 | | VPREB1 |
| 10q26.3 | chr10: 129812260-135374737 | 35 | | MGMT, SYCE1 |
| 12p13.2 | chr12: 11410696-12118386 | 2 | ETV6$ | ETV6 |
| 8p23.3 | chr8: 1-392555 | 2 | | ZNF596 |
| 1p36.11 | chr1: 26377344-27532551 | 24 | | SFN |
| 11p15.5 | chr11: 1-1391954 | 49 | | RASSF7 |
| 17q11.2 | chr17: 26185485-27216066 | 10 | NF1 | NF1 |
| 11q23.1 | chr11: 107086196-116175885 | 61 | ATM | CADM1 |
| 9p24.3 | chr9: 1-708871 | 5 | | FOXD4 |
| 10q11.23 | chr10: 52313829-53768264 | 4 | PRKG1§ | DKK1, PRKG1 |
| 15q15.1 | chr15: 35140533-43473382 | 109 | | TUBGCP4 |
| 1p13.2 | chr1: 110339388-119426489 | 81 | | MAGI3 |
| Xp22.33 | chrX: 1-3243111 | 21 | | SHOX |
| 3p26.3 | chr3: 1-2121282 | 2 | | CHL1 |
| 9p13.2 | chr9: 36365710-37139941 | 2 | PAX5 | MELK |

(continued)

| Chromosome and band | Peak region | # genes | Known target | Grail top target |
|---|---|---|---|---|
| 17p13.1 | chr17: 7471230-7717938 | 10 | TP53 | ATP1B2 |
| 12q24.33 | chr12: 131913408-132349534 | 7 | | CHFR |
| 7q36.3 | chr7: 156893473-158821424 | 7 | PTPRN2§ | NCAPG2 |
| 6q16.1 | chr6: 76630464-105342994 | 76 | | FUT9, |
| | | | | C6orf165, |
| | | | | C6orf162, |
| | | | | GJA10 |
| 5q21.1 | chr5: 85837489-133480433 | 142 | APC | APC |
| 8p11.22 | chr8: 39008109-41238710 | 7 | | C8orf4, |
| | | | | ZMAT4 |
| 19q13.32 | chr 19: 52031294-53331283 | 25 | | BBC3 |
| 10p15.3 | chr10: 1-1042949 | 4 | | TUBB8 |
| 1p31.1 | chr1: 71284749-74440273 | 4 | NEGR1§ | NEGR1 |
| 13q31.3 | chr13: 92308911-94031607 | 2 | GPC6§ | GPC6, |
| | | | | DCT |
| 16q11.2 | chr16: 31854743-53525739 | 37 | | RBL2 |
| 20p13 | chr20: 1-325978 | 10 | | SOX12 |
| 5q35.3 | chr5: 177541057-180857866 | 43 | | SCGB3A1 |
| 1q43 | chr1: 223876038-247249719 | 173 | RYR2§ | FH, |
| | | | | ZNF678 |
| 16p13.3 | chr16: 1-359092 | 16 | | HBZ |
| 17q21.2 | chr17: 37319013-37988602 | 22 | | CNP |
| 2p25.3 | chr2: 1-15244284 | 51 | | MYT1L |
| 3q13.31 | chr3: 116900556-120107320 | 1 | | LSAMP |
| 7q21.11 | chr7: 65877239-79629882 | 73 | MAGI2§ | CLDN4 |

(continued)

| Chromosome and band | Peak region | # genes | Known target | Grail top target |
|---|---|---|---|---|
| 7q35 | chr7: 144118814-148066271 | 3 | CNTNAP2§ | CNTNAP2 |
| 14q32.12 | chr14: 80741860-106368585 | 154 | | PRIMA1 |
| 16q24.3 | chr16: 88436931-88827254 | 9 | | C16orf3 |
| 3q26.31 | chr3: 175446835-178263192 | 1 | NAALADL2§ | NAALADL2 |
| 17q25.3 | chr17: 78087533-78774742 | 8 | | ZNF750 |
| 19p12 | chr19: 21788507-34401877 | 12 | | ZNF492, ZNF99 |
| 12q23.1 | chr12: 97551177-99047626 | 3 | ANKS1B§ | ANKS1B |
| 4p16.3 | chr4: 1-435793 | 4 | | ZNF141 |
| 18p11.32 | chr18: 1-587750 | 4 | | COLEC12 |
| 2q33.2 | chr2: 204533830-206266883 | 1 | PARD3B§ | PARD3B |
| 8p21.2 | chr8: 22125332-30139123 | 63 | | DPYSL2, STMN4 |
| 8q11.22 | chr8: 42971602-72924037 | 86 | SNTG1§ | FLJ23356, ST18, RB1CC1 |
| 16q23.3 | chr16: 80759878-82408573 | 2 | CDH13§ | CDH13 |
| 11q14.1 | chr11: 82612034-85091467 | 6 | DLG2§ | CCDC89, CCDC90B, TMEM126A |
| 14q23.3 | chr14: 65275722-67085224 | 7 | | GPHN, MPP5 |
| 7p22.2 | chr7: 3046420-4279470 | 1 | SDK1§ | SDK1 |
| 13q34 | chr13: 111767404-114142980 | 25 | | TUBGCP3 |
| 17p12 | chr17: 10675416-12635879 | 5 | MAP2K4 | MAP2K4, ZNF18 |

(continued)

| Chromosome and band | Peak region | # genes | Known target | Grail top target |
|---|---|---|---|---|
| 21q22.2 | chr21: 38584860-42033506 | 19 | DSCAM§, TMPRSS2/ERG$ | DSCAM |
| 18q21.2 | chr18: 46172638-49935241 | 7 | SMAD4, DCC§ | DCC |
| 6q22.1 | chr6: 101000242-121511318 | 87 | | GTF3C6, TUBE1, ROS1 |
| 14q11.2 | chr14: 1-29140968 | 140 | | ZNF219, NDRG2 |

Although the examples herein concern humans and the language is primarily directed to human concerns, the concepts described herein are applicable to genomes from any plant or animal.

In various embodiments, it is contemplated to use the method identified herein to identify CNVs of segments comprising the amplified regions or genes identified in Table 6 and/or to use the methods identified herein to identify CNVs of segments comprising the deleted regions or genes identified in Table 7. In other embodiments, it is contemplated to use the method identified herein to screen for the presence of CNVs of segments that were not previously linked to cancer or are not described in Table 6 or 7.

In one embodiment, the methods described herein provide a means to assess the association between gene amplification and the extent of tumor evolution. Correlation between amplification and/or deletion and stage or grade of a cancer may be prognostically important because such information may contribute to the definition of a genetically based tumor grade that would better predict the future course of disease with more advanced tumors having the worst prognosis. In addition, information about early amplification and/or deletion events may be useful in associating those events as predictors of subsequent disease progression.

Gene amplification and deletions as identified by the method can be associated with other known parameters such as tumor grade, histology, Brd/Urd labeling index, hormonal status, nodal involvement, tumor size, survival duration and other tumor properties available from epidemiological and biostatistical studies. For example, tumor DNA to be tested by the method could include atypical hyperplasia, ductal carcinoma in situ, stage I-III cancer and metastatic lymph nodes in order to permit the identification of associations between amplifications and deletions and stage. The associations made may make possible effective therapeutic intervention. For example, consistently amplified regions may contain an overexpressed gene, the product of which may be able to be attacked therapeutically (for example, the growth factor receptor tyrosine kinase, p185HER2).

In various embodiments, the method described herein can be used to identify amplification and/or deletion events that are associated with drug resistance by determining the copy number variation of nucleic acid sequences from primary cancers to those of cells that have metastasized to other sites. If gene amplification and/or deletion is a manifestation of karyotypic instability that allows rapid development of drug resistance, more amplification and/or deletion in primary tumors from chemoresistant patients than in tumors in chemosensitive patients would be expected. For example, if amplification of specific genes is responsible for the development of drug resistance, regions surrounding those genes would be expected to be amplified consistently in tumor cells from pleural effusions of chemoresistant patients but not in the primary tumors. Discovery of associations between gene amplification and/or deletion and the development of drug resistance may allow the identification of patients that will or will not benefit from adjuvant therapy.

In other embodiments, the method described herein can be used to identify the presence of genome-wide instability and/or microsatellite instability and/or specific combinations of Copy Number Variations (which could span whole chromosomes, chromosome arms, or smaller DNA segments).

In yet another embodiment, the method described herein can be used to identify the tumor origin, i.e. the primary tissue or organ where the tumor originated from prior to becoming metastatic.

Both complete and partial chromosomal aneuploidies as well as smaller Copy Number Variations of DNA segments that could be associated with the formation, and progression of cancer can be determined according to the present method.

II Sequencing, aligning and correction

As mentioned above, only a fraction of the genome is sequenced. In one aspect, even when a pool of nucleic acids in a specimen is sequenced at <100% genomic coverage instead of at several folds of coverage, and among the proportion of sequenced nucleic acid molecules, most of each nucleic acid species is not sequenced or sequenced only once.

This is contrasted from situations where targeted enrichment is performed of a subset of the genome prior to the sequencing reaction, followed by high-coverage sequencing of that subset.

In one embodiment, massive parallel short-read sequencing is used.. Short sequence tags or reads are generated, e.g. from a certain length between 20bp and 400bp. Alternatively, paired end sequencing could be performed.

In an embodiment, a pre-processing step is available for pre-processing the obtained reads. Such pre-processing option allows filtering low quality reads, thereby preventing them from being mapped. Mapping of low quality reads can require prolonged computer processing capacity, can be incorrect and risks increasing the technical noise in the data, thereby obtaining a less accurate parameter. Such pre-processing is especially valuable when using next-generation sequencing data, which has an overall lower quality or any other circumstance which is linked to an overall lower quality of the reads.

The generated reads can subsequently be aligned to one or more human reference genome sequences. Preferably, the number of aligned reads are counted and/or sorted according to their chromosomal location.

An additional clean-up protocol can be performed, whereby deduplication is performed, e.g. with Picard tools, retaining only uniquely mapped reads. Reads with mismatches and gaps can be removed. Reads that map to blacklisted regions can be excluded. Such blacklisted regions may be taken from a pre-defined list of e.g. common CNVs, collapsed repeats, DAC blacklisted regions as identified in the ENCODE project (i.e. a set of regions in the human genome that have anomalous, unstructured, high signal/read counts in NGS experiments independent of cell line and type of experiment) and the undefined segment of the reference genome. In one embodiment, blacklisted regions are provided to the user. In another embodiment, the user may use or define his or her own set of blacklisted regions.

In a further embodiment, chromosomes are divided into regions of a predefined length, generally referred to as bins. In an embodiment, the bin size is a predefined size provided to the user. In another embodiment, said bin size can be defined by a user, can be uniformly for all chromosomes or can be a specific bin size per chromosome or can vary according to the obtained sequence data. Change of the bin size can have an effect on the final parameter to be defined, either by improving the sensitivity (typically obtained by decreasing the bin size, often at the cost of the specificity) or by improving the specificity (generally by increasing the bin size, often at the cost of the sensitivity). A possible bin size which provides an acceptable specificity and sensitivity is 50 kb.

In a further step, the aligned and filtered reads within a bin are counted, in order to obtain read counts.

The obtained read counts can be corrected for the GC count for the bin. GC bias is known to aggravate genome assembly. Various GC corrections are known in the art (e.g. Benjamini et al., Nucleic Acid Research 2012). In a preferred embodiment, said GC correction will be a LOESS regression. In one embodiment, a user of the methodology according to the present teachings can be provided with the choice of various possible GC corrections.

In a subsequent step, the genomic representation (GR) of read counts per bin is calculated. Such representation is preferably defined a ratio or correlation between the GC-corrected read counts for a specific bin and the sum of all GC-corrected read counts.

In an embodiment, said GR is defined as follows:

$$GRi = \frac{GCi}{\sum_k GCk} \cdot 10^7$$

with k over all chromosomal bins (whereby the factor 107 in the above formula is arbitrary defined, and can be any constant value)

In a final step, the obtained GR per bin are aggregated over a region, whereby said region may be a subregion (window) of a chromosome or the full chromosome. Said window may have a predefined or variable size, which can optionally be chosen by the user. A possible window could have a size of 5 MB or 100 adjacent bins of size 50 kb.

The GR aggregated for a chromosome can be defined by

$$GRi = \frac{\sum_{j \in (bins\ on\ chr)} GCi}{\sum_{k \in (all\ bins)GCk} GCk}$$

In a further embodiment, the genomic representation of a set of reference samples shall be calculated. Said set of reference samples (or also termed reference set) can be predefined or chosen by a user (e.g. selected from his/her own reference samples). By allowing the user the use of an own reference set, a user will be enabled to better capture the recurrent technical variation of his/her environment and its variables (e.g. different wet lab reagents or protocol, different NGS instrument or platform, etc.) In a preferred embodiment, said reference set comprises genomic information of 'healthy' samples, that are known to not contain (relevant) aneuploidies. The genomic representation (GR) of the reference set can be defined, either at the genome level and/or at a subregion (chromosome, chromosomal segment, window, or bin).

Other single molecule sequencing strategies such as that by the Roche 454 platform, the Applied Biosystems SOLiD platform, the the Helicos True Single Molecule DNA sequencing technology, the single molecule, real-time (SMRT™) technology of Pacific Biosciences, and nanopore sequencing technologies like MinION, GridION or PromethION from Oxford Nanopore Technologies could similarly be used in this application.

III Determining scores, parameter and secondary parameters

From the alignments and the obtained read counts or a derivative thereof, optionally corrected for GC content and/or total number of reads obtained from said sample, scores are calculated which eventually lead to a parameter allowing the determination of the presence of an aneuploidy in a sample. Said scores are normalized values derived from the read counts or mathematically modified read counts, whereby normalization occurs in view of the reference set. As such, each score is obtained by means of a comparison with the reference set. The term first score is used to refer to score linked to the read count for a target chromosome or a chromosomal segment. A collection of scores is a set of scores derived from a set of normalized number of reads that may include the normalized number of reads of said target chromosomal segment or chromosome.

Preferably, said first score represents a Z score or standard score for a target chromosome or chromosomal segment. Preferably, said collection is derived from a set of Z scores obtained from a corresponding set of chromosomes or chromosomal segments that include said target chromosomal segment or chromosome.

Such scores can be calculated as follows:

$$Zi = \frac{GRi - \mu\,ref,i}{\sigma\,ref,i}$$

With i a window or a chromosome or a chromosome segment.

A summary statistic of said collection of scores can e.g. be calculated as the mean or median value of the individual scores.

Another summary statistic of said collection of scores can be calculated as the standard deviation or median absolute deviation or mean absolute deviation of the individual scores.

Optionally but not necessarily, the same collection of scores is used for both types of calculations.

Said parameter p will be calculated as a function of the first score and a derivative (e.g. summary statistic) of the collection of scores. In a preferred embodiment, said parameter p will be a ratio or correlation between the first score corrected by the collection of scores (or a derivative thereof) and a derivative of said collection of scores.

In another embodiment, said parameter will be a ratio or correlation between the first score corrected by a summary statistic of a first collection of scores and a summary statistic of a different, second collection of scores, in which both collections of scores include the first score.

In a specifically preferred embodiment, said parameter p is a ratio or correlation between the first score, corrected by a summary statistic of said collection of scores, and a summary statistic of said collection of scores. Preferably, the summary statistic is selected from the mean, median, standard deviation, median absolute deviation or mean absolute deviation. In one embodiment, said both used summary statistics in the function are the same. In another, more preferred embodiment, said summary statistics of the collection of scores differ in the numerator and denominator.

Typically, a suitable embodiment according to the present teachings involve the following steps (after having obtained DNA sequences from a random sequencing process on a biological sample).

- aligning said obtained sequences to a reference genome;

- counting the number of reads on a set of chromosomal segments and/or chromosomes thereby obtaining read counts;

- normalizing said read counts or a derivative thereof into a normalized number of reads;

- obtaining a first score and a collection of scores of said normalized reads, whereby said first score is derived from the normalized reads for a target chromosome or chromosomal segment and said collection of scores is a set of scores derived from a corresponding set of chromosomes or chromosome segments that include said target chromosomal segment or chromosome;

- calculating a parameter p from said first score and said collection of scores, whereby said parameter represents a ratio or correlation between

  * said first score, corrected by a summary statistic of said collection of scores, and

  * a summary statistic of said collection of scores.

A possible parameter p can be calculated as follows:

$$Z \text{ of } Z_i = \frac{Z_i - \underset{j=i,a,b,\ldots}{\text{median}}(Z_j)}{\underset{j=i,a,b,\ldots}{\text{sd}}(Z_j)}$$

Whereby Zi represents the first score and Z j the collection of scores and whereby i represents the target chromosome or chromosomal section, and whereby j represents a collection chromosomes or chromosomal segments i, a, b, .. that includes said target chromosomal segment or chromosome i.
In another embodiment, said parameter p is calculated as

$$Z \text{ of } Z_i = \frac{Z_i - \underset{j=i,a,b,\ldots}{\text{mean}}(Z_j)}{\underset{j=i,a,b,\ldots}{\text{mad}}(Z_j)}$$

Whereby Zi represents the first score and Z j the collection of scores and whereby i represents the target chromosome or chromosomal section, and whereby j represents a collection of chromosomes or chromosomal segments i, a, b, .. that includes said target chromosomal segment or chromosome i.
In yet another, most preferred embodiment, said parameter p is calculated as

$$Z \text{ of } Z_i = \frac{Z_i - \underset{j=i,a,b,\ldots}{\text{median}}(Z_j)}{\underset{j=i,a,b,\ldots}{\text{mad}}(Z_j)}$$

Whereby Zi represents the first score and Z j the collection of scores and whereby i represents the target chromosome or chromosomal section, and whereby j represents a collection of chromosomes or chromosomal segments i, a, b, .. that includes said target chromosomal segment or chromosome i.
Said MAD (median absolute deviation) for a data set x_1,x_2,...,x_n is known in the art and may be computed as

$$\text{"MAD"}=1.4826 \times \text{"median"} (|x\_i\text{-"median"} (x)|)$$

An alternative MAD that does not use the factor 1.4826 can also be used.
The factor 1.4826 is used to ensure that in case the variable x is normally distributed with a mean $\mu$ and a standard deviation $\sigma$ that the MAD score converges to $\sigma$ for large n. To ensure this, one can derive that the constant factor should equal $1/((\Phi^{-1} (3/4)))$, with $\Phi^{-1}$ is the inverse of the cumulative distribution function for the standard normal distribution.
Apart from the parameter p which will allow the identification of the presence of aneuploidies, secondary parameters can be calculated which may serve as quality control or provide additional information with regard to one or more aneuploidies present in the sample.

A first secondary parameter which can be calculated allows defining whether chromosomal and large subchromosomal aneuploidies are present in the sample (compared to e.g. smaller aneuploidies). In a preferred embodiment, such parameter is defined by the median of the Z scores measured per subregions (e.g. 5 Mb windows) in a target chromosome. If more than 50% of these subregions are affected, this will show in the secondary parameters.

In another embodiment, a secondary parameter may be calculated as the median of the absolute value of the Z scores calculated over the remaining chromosomes (that is all chromosomes except the target chromosome or chromosomal segment) per subregion (e.g. 5 Mb windows).The latter secondary parameter allows the detection of the presence of technical or biological instabilities (cf. malignancies, cancer). If less than half of the windows of the other or all autosomes or chromosomes are affected, this secondary parameter will not be affected. If more than 50% of the windows is affected, this will be derivable from said secondary parameters.

In another embodiment, the present teachings also provide for a quality score (QS). QS allows to assess the overall variation across the genome. A low QS is an indication of a good sample processing and a low level of technical and biological noise. An increase in the QS can indicate two possible reasons. Either an error occurred during the sample processing. In general, the user will be requested to retrieve and sequence a new biological sample. This is typical for moderately increased QS scores. A strongly increased QS is an indication of a highly aneuploid or genome-wide instable sample and the user may be encouraged to do a confirmatory test to further assess whether the subject is developing cancer. Preferably, said QS is determined by calculating the standard deviations of all Z distributions for the chromosomes and by removing the highest and lowest scoring chromosome.

For instance, samples with a QS exceeding 2 are considered to be of poor quality, or at increased risk for cancer and a QS between 1.5 and 2 are of intermediate quality.

IV. Comparison to cutoff value

The parameter p as calculated in the embodiments above shall subsequently be compared with a cutoff parameter for determining whether a change compared to a reference quantity exists (i.e. an imbalance), for example, with regards to the ratio or correlation of amounts of two chromosomal regions (or sets of regions). The presence of an aneuploidy and/or an increased number of said aneuploidy is an indicator of the presence and/or increased risk of a cancer. In one embodiment, the user will be able to define its own cutoff value, either empirically on the basis of experience or previous experiments, or for instance based on standard statistical considerations. If a user would want to increase the sensitivity of the test, the user can lower the thresholds (i.e. bring them closer to 0). If a user would want to increase the specificity of the test, the user can increase the thresholds (i.e. bring them further apart from 0). A user will often need to find a balance between sensitivity and specificity, and this balance is often lab- and application -specific, hence it is convenient if a user can change the threshold values him- or herself.

Based on the comparison with the cutoff value, an aneuploidy may be found present or absent. Such presence is indicative for the presence and/or increased risk of a cancer.

In an embodiment of the present teachings, comparison of parameter p with a cutoff value is sufficient for determining the presence or absence of an aneuploidy. In another embodiment, said aneuploidy is determined on the basis of a comparison of parameter p with a cutoff value and a comparison of at least one of the secondary parameters, quality score and/or first score with a cutoff value, whereby for each score a corresponding cutoff value is defined or set.

In a preferred embodiment, said presence/absence of an aneuploidy is defined by a comparison of parameter p with a predefined cutoff value, as well as by comparison of all secondary parameters, quality and first scores as described above with their corresponding cutoff values.

The final decision tree may thus be dependent on parameter p alone, or combined with one of the secondary parameters and/or quality score or first score as described above.

In a preferred embodiment, said methodology according to the present teachings comprise the following steps:

- multiplex sequencing of 50 bp single-end reads (performed by end user)

- uploading sequence reads

- mapping of reads to a reference genome

- count number of reads per bin (a bin has a size of 50 kb)

- compute GC content per bin and correct for GC content

- compute Genomic Representation (GR) score per bin. For bin i this equals

$$GRi = \frac{\sum_{j \in (bins\ on\ chr)} GCi}{\sum_{k \in (all\ bins)GCk} GCk}$$

- aggregate the GR values per window (a window consists out of 100 consecutive windows)

- compute a Z score per window or per chromosome, whereby the Z score is based on the GR score per chromosome, compared with the GR scores obtained in a set of reference samples.

$$Z_i = \frac{GR_i - \mu_{\mathrm{Ref},i}}{\sigma_{\mathrm{Ref},i}}$$

with i a chromosome or a window, $\mu$ Ref,i the average or median GR score for the corresponding bins in the set of reference samples and $\square$ Ref,I the standard deviation of the GR scores for the corresponding bins in the set of reference samples.

- computing of a ZofZ parameter, whereby the ZofZ parameter is based on the Z score, corrected by the median (or mean) of the Z scores of a collection of chromosomes including target chromosome i and divided by a factor that measures the variability of the Z scores of a collection of chromosomes that includes the target chromosome i (standard deviation or a more robust version thereof, like e.g. the median absolute deviation or mad).

- comparison of the Z score with a threshold value, and the ZofZ parameter with a threshold value, to predict the presence or absence of an aneuploidy.

In a further preferred embodiment, said prediction of the presence or absence of an aneuploidy occurs via a decision tree based on parameter p and secondary parameters.

V Toolbox and kit

By preference, the methodologies as described above are all computer implemented. To that purpose, the present teachings equally relates to a computer program product comprising a computer readable medium encoded with a plurality of instructions for controlling a computing system to perform an operation for performing analysis of a chromosomal or subchromosomal aneuploidy in a biological sample obtained from a subject, wherein the biological sample includes nucleic acid molecules.
With regard to the determination of the presence or absence of aneuploidy or genome-wide instability in a sample, the operation comprises the steps of:

- receiving the sequences of at least a portion the nucleic acid molecules contained in a biological sample obtained from said pregnant female;

- aligning said obtained sequences to a reference genome;

- counting the number of reads on a set of chromosomal segments and/or chromosomes thereby obtaining read counts;

- normalizing said read counts or a derivative thereof into a normalized number of reads;

- obtaining a first score of said normalized reads and a collection of scores of said normalized reads, whereby said first score is derived from the normalized reads for a target chromosome or chromosomal segment and whereby said collection of scores is a set of scores derived from the normalized number of reads for a set of chromosomes or chromosomal segments that include said target chromosomal segment or chromosome;

- calculating a parameter p from said first score and said collection of scores.

In a preferred embodiment, said parameter whereby said parameter represents a ratio or correlation between

* a first score, corrected by a summary statistics of said collection of scores, and

* a summary statistics of the collection of scores.

Said operations can be performed by a user or practitioner in an environment remote from the location of sample collection and/or the wet lab procedure, being the extraction of the nucleic acids from the biologic sample and the sequencing.

Said operations can be provided to the user by means of adapted software to be installed on a computer, or can be stored into the cloud.

After having performed the required or desired operation, the practitioner or user will be provided with a report or score, whereby said report or score provides information on the feature that has been analyzed. Preferably, report will comprise a link to a patient or sample ID that has been analyzed. Said report or score may provide information on the presence or absence of an aneuploidy in a sample, whereby said information is obtained on the basis of a parameter which has been calculated by the above mentioned methodology. The report may equally provide information on the nature of the aneuploidy (if detected, e.g. large or small chromosomal aberrations) and/or on the quality of the sample that has been analyzed.

It shall be understood by a person skilled in the art that above-mentioned information may be presented to a practitioner in one report.

By preference, above mentioned operations are part of a digital platform which enables molecular analyzing of a sample by means of various computer implemented operations.

In particular, the present teachings also comprise a visualization tool, which enables the user or practitioner to visualize the obtained results as well as the raw data that has been imputed in the system. In an embodiment, said visualizations comprises a window per chromosome, depicting the chromosome that has been analyzed, showing the reads per region and the scores and/or parameter that has been calculated. By showing to the practitioner or user the calculated scores and parameter together with the visual depiction of the read counts, a user may perform an additional control or assessment of the obtained results. By allowing the user to look at the data, users will be able to define improved decision rules and thresholds.

Moreover, an additional control is added, as the visual data per chromosome enables the user to evaluate for every chromosome if the automated classification is correct. This adds an additional safety parameter.

In a preferred embodiment, said platform and visualization tool is provided with algorithms which take into account the fact that certain regions give more reads (due to a recurrent technical bias that makes some regions of the genome always over- or under-represented). Correction measures may be provided for this overrepresentation by making a comparison with a reference set (that is ideally processed using the same or similar protocol) and plotting e.g. Z scores or alternative scores that represent the unlikeliness of certain observations under the assumption of euploidy. Standard visualization tools only display read count, and do not allow correcting for the recurrent technical bias.

Finally, based on the link between the obtained scores and/or parameter and the visual data per chromosome a user or practitioner may decide to alter the threshold/cutoff value that is used to define the presence of an aneuploidy. As such, the user may decide to aim for a higher sensitivity (e.g. being less stringent on the decrease/increase of the parameter or scores) or higher specificity (e.g. by being more stringent on the increase/decrease of parameter or scores).

The platform may be provided with other features, which provide for an accurate analysis of the molecular data obtained from the biological sample.

The platform according to the present teachings is inherently compatible with many different types of NGS library preparation kits and protocols and NGS sequencing platform. This is an advantage as a user will not have to invest in dedicated NGS sequencing platform or NGS library preparation kits that are specific for a specific application, but instead a user can use its preferred platform and kit. Moreover, this allows a user a certain degree of flexibility in material to be used. If newer or cheaper instruments or kits become available, a user will be allowed an easy change.

As mentioned before, the current methodology is compatible with cell-free DNA extracted from various sorts of biological samples, including blood, saliva and urine. Using urine or saliva instead of blood would represent a truly non-invasive sample type and allows for e.g. home-testing and shipment of the sample to the test lab. This is obviously an additional advantage compared to other sample obtaining methods such as drawing blood.

The present teachings are further described by the following non-limiting examples which further illustrate the invention, and are not intended to, nor should they be interpreted to, limit the scope of the invention.

**Examples**

<u>General</u>

<u>1. Preparation and sequencing of the sample</u>

1. Blood collection, plasma separation and cell-free DNA extraction

One tube (10 ml) of maternal blood is collected in Streck tubes and stored at 4°C. The blood is collected via a standard phlebotomy procedure.
The plasma (+/- 5 ml) is separated maximum 72 hours after blood sampling by the standard dual centrifugation method:

- The blood sample is centrifuged at 2000xg for 20 minutes (this may be done at room temperature), without using the brake.
- The plasma is then transferred to either three 1.5ml low binding tubes, or to a single 5 ml low binding tube. A second centrifugation at 13,000xg is done for 2 minutes (this may be done at room temperature).
- The plasma is transferred to sterile 1.5 ml or 5 ml low binding tubes for storage at -20°C prior to cell-free DNA (cfDNA) extraction.

Optionally, the buffy coat layer can be stored for future testing. Maternal genomic DNA from the buffy coat layer can be investigated to confirm or exclude maternal abnormalities.
The cell-free DNA is extracted from the plasma using the QIAamp Circulating Nucleic Acid Kit (Qiagen) according to the manufacturer's recommendations, with a final elution volume of 60 $\mu$l. The DNA samples are stored at -20°C when not used immediately for library preparation.

<u>2. cfDNA quantification</u>

The extracted cfDNA is quantified using a Qubit fluorometer. The cell-free DNA concentration is usually around 0.1-1 ng/$\mu$l.

<u>3. Library preparation</u>

25 $\mu$l of the extracted cfDNA is used as starting material for library preparation. During library preparation, the DNA samples are adapted for next-generation sequencing. Adaptors are added to the ends of the DNA fragments.
The sequencing libraries are prepared using the TruSeq ChIP library preparation kit (Illumina) with some adaptation of the manufacturer's protocol by reducing the reagent volumes to allow the generation of sequencing libraries using low starting amounts of DNA.
The library preparation protocol may be summarized as follows:
(Note: The bead-based size selection for removal of large DNA fragments and removal of small DNA fragments described in the protocol is NOT used.)

<u>End repair of the DNA fragments:</u>

1. Add 5 $\mu$l Resuspension Buffer and 20 $\mu$l End Repair Mix to the 25 $\mu$l starting material (total = 50 $\mu$l)
The bead-based size selection for removal of large DNA fragments and removal of small DNA fragments described in the protocol is NOT used.
2. Incubate 30 minutes at 30°C
3. Add 80 $\mu$l undiluted AMPure beads to the 50 $\mu$l sample mixture after end repair.
4. Wash the beads twice with 190 $\mu$l 80% EtOH
5. Resuspend the dried pellet with 10 $\mu$l Resuspension Buffer. Transfer 9 $\mu$l of the supernatant to a new tube.

<u>Adenylation of the 3' ends</u>

1. Add 6.25 $\mu$l A-tailing Mix

2. Heat 30 minutes at 37°C + 5 minutes at 70°C

Ligation of the indexed paired-end adaptors to the DNA

1. Adaptors are diluted 1/2 with Resuspension Buffer => add 2,5 µl to sample
2. Add 1.25µl Ligation Mix (no Resuspension Buffer). Incubate 30 minutes at 30°C
3. Add 2.5 µl Stop ligation buffer
4. Add 21 µl AMPure beads for clean-up
5. Wash the beads twice with 190 µl 80% EtOH
6. Resuspend the dried pellet in 27 µl Resuspension Buffer. Transfer 25 µl of the supernatant to a new tube.
7. Add 25 µl AMPure beads for clean-up
8. Wash the beads twice with 190 µl 80% EtOH
9. Resuspend the dried pellet in 12.5 µl Resuspension Buffer. Transfer 10 µl of the supernatant to a new tube.

Enrich DNA fragments

1. Prepare the PCR mix by mixing 2,5 µl PCR Primer Cocktail and 12,5 µl PCR Master Mix for each sample.
2. PCR conditions:

98°C for 30 seconds
15 cycles of:

98°C for 10 seconds
60°C for 30 seconds
72°C for 30 seconds
72°C for 5 minutes

hold at 4°C

3. Add 25 µl of AMPure beads for clean up
4. Wash the beads twice with 190 µl 80% EtOH
5. Resuspend the dried pellet in 32.5 µl Resuspension Buffer. Transfer 30 µl of the supernatant to a new tube.
6. Use 2 µl of the sample for Qubit quantification and 2µl for fragment analysis (see next section)

4. Library quality check

Proper cell-free DNA isolation and NGS library preparation are tested by analyzing every library on the Fragment Analyzer (Advanced Analytical Technologies Inc., Germany) prior to sequencing, to assess:

- the size distribution (confirm suitable size profile using concentration, peak ratio, peak height, ...),
- the quality of the library. Samples containing high molecular weight fragments will be classified as not eligible for sequencing (indicates contamination with maternal genomic DNA).

Typical libraries demonstrate a narrow size distribution with a peak at about 300-350 bp.
Additionally a Qubit quantification step is performed so that the enrichment reaction is done with the appropriate amount of input DNA material. DNA concentration is usually around 15-30 ng/µl.

5. Normalize and pool libraries

Samples are indexed during library preparation and up to 24 samples are normalized and pooled in equal volumes for multiplex sequencing across both lanes of an Illumina HiSeq2500 flow cell.

6. NGS run

Sequencing is performed on the HiSeq 2500 (Illumina) in Rapid Run mode producing 50bp single end reads.

Detection of an aneuploidy in a biological sample: validation of the methodology

* Mapping and filtering of the mapped reads

The 50bp single end sequence reads of a test sample are mapped to the reference genome GRCh37.75 with BWA-backtrack. With Picard tools duplicated reads are removed and based on the mapping quality reads mapping to multiple locations are discarded. Also reads mapping sub-optimally to multiple locations are removed. To reduce sample variability, we retain only those reads that match perfectly with the reference genome (i.e., no mismatches and no gaps are allowed). Finally, also reads that fall in an in-house curated list of blacklisted regions are removed. These blacklisted regions comprise common polymorphic CNVs, collapsed repeats, DAC blacklisted regions generated for the ENCODE project and the undefined portion of the reference genome (i.e., the Ns).

* Computing genomic representation

The reference genome is divided in bins of 50kb and the number of reads of the test sample is counted per bin. These read counts are corrected according to the GC contents of the bins with locally weighted scatterplot smoothing (Loess regression). These GC-corrected read counts are then divided by the total sum of all autosomal GC-corrected read counts and multiplied by $10^7$. This is defined as the genomic representations (GR) per bin. On these per-bin GR values a sliding window is applied and the sum of these GR values is computed for all consecutive 100 bins. The windows are each time shifted with 1 bin (i.e., 50 kb). In this way a GR value is obtained per 5Mb window. Similarly, for each autosome also the sum of the per-bin GR values is calculated, to obtain a GR value for each autosome in the test sample.

* Comparison with a reference set

In a reference set of 100 *normal* samples (50 male and 50 female pregnancies) the GR values are computed for all autosomes and for all 5Mb windows as described above. For each autosome and 5Mb window the mean $\mu$ and the standard deviation $\sigma$ of the GR scores are computed over all 100 reference samples. This allows computing a *Z-score* for each window and each autosome $i$ in a test sample, defined as

$$Z_i = \frac{GR_i - \mu_i}{\sigma_i}$$

where $GR_i$ is the GR value in the test sample for window or autosome i and $\mu_i$, $\sigma_i$ the average and standard deviation, respectively, of the GR scores measured in the 100 reference samples for window or autosome *i*.

Based on the 22 Z-scores of the autosomes in a test sample, a *ZZ2 score* is computed for each autosome as

$$ZZ2 = \frac{Z_i - \underset{j=1,\dots,22}{\mathrm{median}}(Z_j)}{\underset{j=1,\dots,22}{\mathrm{sd}}(Z_j)}$$

where the Z-score $Z_i$ of autosome i in the test sample is compared with the median and the standard deviation (sd) of the 22 Z-scores obtained for all 22 autosomes in the test sample.
Alternatively, a *ZofZ-score* is computed as

$$Zof Z_i = \frac{Z_i - \underset{j=1,\dots,22}{\mathrm{median}}(Z_j)}{\underset{j=1,\dots,22}{\mathrm{mad}}(Z_j)}$$

where the Z-score $Z_i$ of chromosome *i* in the test sample is compared with the median and the median absolute deviation (mad) of the 22 Z-scores obtained for all 22 autosomes in the test sample. Said MAD for a data set x_1,x_2,...,x_n is known in the art and may be computed as

$$\text{"MAD"}=1.4826 \times \text{"median"} \left(|x\_i-\text{"median"}(x)|\right)$$

An alternative MAD that does not use the factor 1.4826 can also be used.

The factor 1.4826 is used to ensure that in case the variable x is normally distributed with a mean $\mu$ and a standard deviation $\sigma$ that the MAD score converges to $\sigma$ for large n. To ensure this, one can derive that the constant factor should equal $1/((\Phi^{\wedge}(-1)(3/4)))$, with $\Phi^{\wedge}(-1)$ is the inverse of the cumulative distribution function for the standard normal distribution.

The ZZ2 and ZofZ-scores quantify the deviation of the Z-score of the target autosome from all Z-scores observed in the test sample. This robust version of the Z-of-Z scores does not make any assumptions on the aneuploidy state of the autosome under consideration and the other autosomes.

Based on the Z-scores computed for all 5Mb windows in the test sample, the *BM score* of each autosome *i* is computed as the median of the Z-scores over all windows of the target autosome:

$$BM_i = \operatorname*{median}_{j=\text{window on i}} \left(Z_j\right)$$

where the median of the Z-scores is computed over all windows j on autosome *i*. This BM-score reflects the size of the aberration: aneuploidies will result in large BM values, while smaller, segmental CNVs will have less influence on the median of the Z-scores and result in smaller BM-scores.

To distinguish high, aberration-related BM scores from augmented BM values due to noise in the data set, the OM score for an autosome *i* is computed as the median of the Z-scores of all windows of the other autosomes:

$$OM_i = \operatorname*{median}_{j=\text{window not on i}} \left|Z_j\right|$$

where the median is computed over all, absolute Z-scores obtained for 5Mb windows *j* not located on autosome *i*.

Finally, for each test sample a *quality score (QS)* is calculated as

$$QS = \operatorname*{sd}_{j}\left(Z_j\right)$$

with *j* over all autosomes expect for the 2 autosomes with the highest and the lowest Z-score. This score will identify test samples of poor quality that will result in unreliable aneuploidy calling. A highly elevated QS-score can also hint at DNA samples containing at least a fraction of DNA originating from a tumor.

For each of the above calculated parameters, a threshold value can be defined. Based on standard statistical considerations, one could choose a threshold value of 2, 2.5 or 3. In the context of the Z-score, this means that the chance that the test result is normal (i.e. the obtained GR score is similar as the GR scores for the same region in the reference set) is very unlikely. In order to make a test more specific, one could increase the threshold value. In order to make a test more sensitive, one could decrease the threshold value. These threshold values can be defined for each of the parameters, and can differ for each of the parameters. It is for instance conceivable that threshold values for BM and OM are set to 1, while for Z score and ZZ-score they are set to 3. Also negative threshold values can be used.

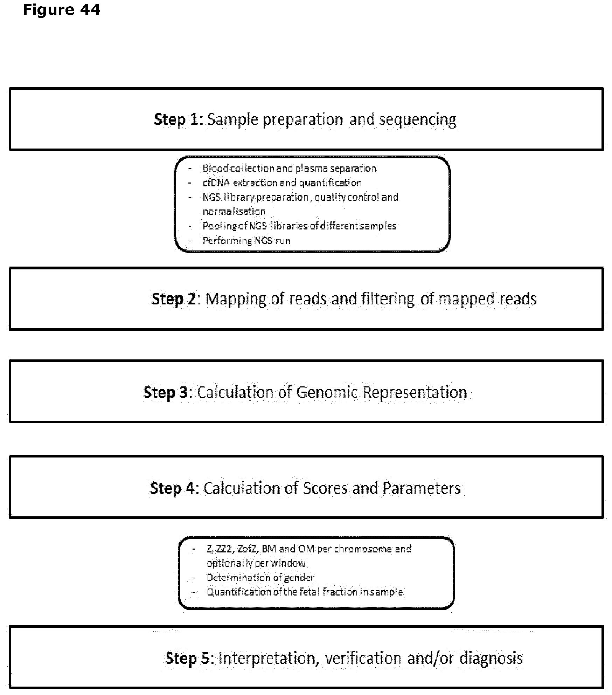

Figure 44 depicts a schematic overview of the performed steps as explained above.

## Example 1

1.1 ZZ2 computation for chromosome 21 for sample A

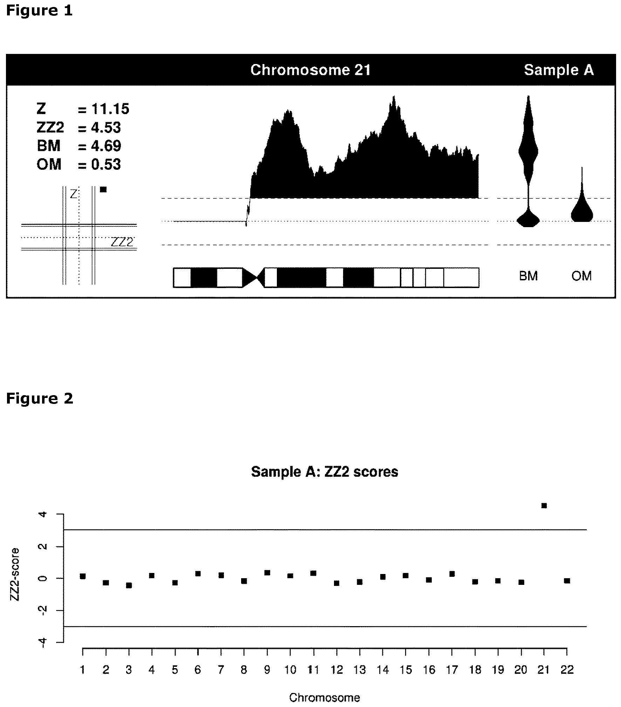

For sample A, the median of the Z-scores for all chromosomes equals -0.1641 and the standard deviation equals 2.494. For chromosome 21, we have a Z-score of 11.147 and this results in a ZZ2 score of 4.5347, above the threshold of 3. (Figure 1)

This automated classification as an abnormal chromosome based on ZZ2>3, is also confirmed by visual inspection of the plot. This sample was tested with an invasive test and the trisomy was confirmed.

None of the other chromosomes in sample A have elevated ZZ2 scores (figure 2).

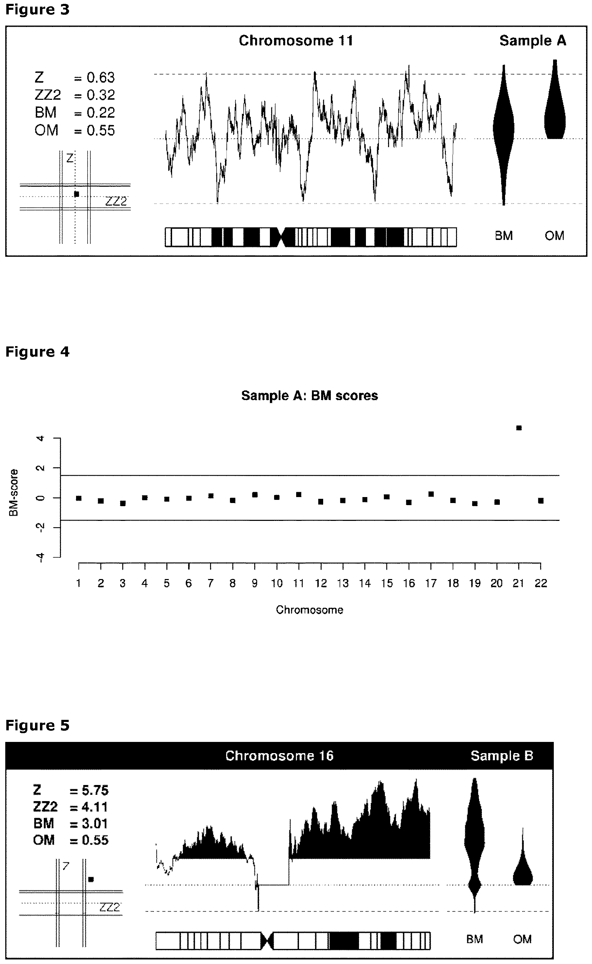

The plots of the Z-scores of these other chromosomes are also not indicative for aneuploidy. For example, on chromosome 11 we have a ZZ2 score 0.319 and this results in the plot as shown in figure 3.

The plot in figure 4 shows the BM scores of all autosomes. The latter confirms that the chromosome 21 is strongly aberrant, while other potential autosomal aberrations will span less than half of the chromosome.

1.2 ZofZ computation for chromosome 21 for sample A

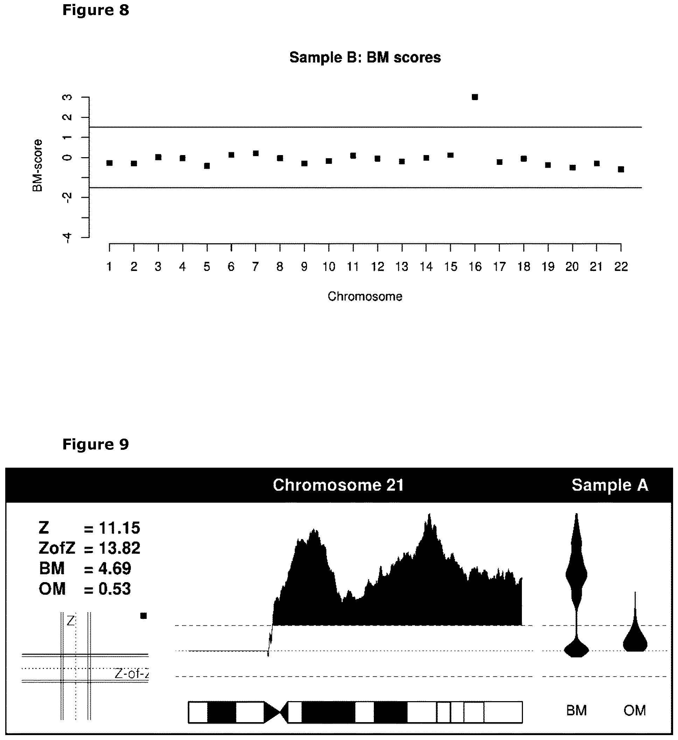

For sample A, the median of the Z-scores for all chromosomes equals -0.164 and the median absolute deviation equals 0.819. For chromosome 21, we have a Z-score of 11.147 and this results in a ZofZ-score of 13.817, far above the threshold of 3. If we plot the Z-scores, this clearly hints at a trisomy 21 (figure 9). This sample was tested with an invasive test and the trisomy was confirmed.

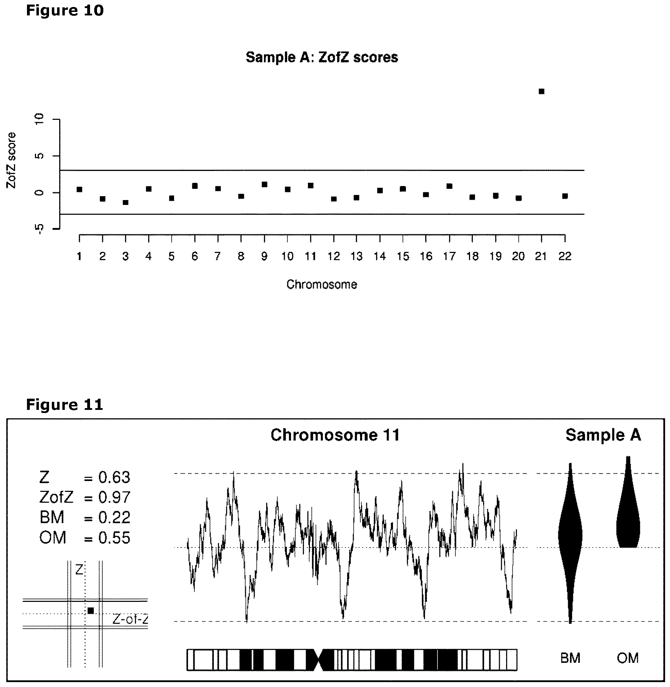

Based on these ZofZ parameters and a threshold of 3, none of the other chromosomes would be called aneuploidy (figure 10).

The plots of the Z-scores of these other chromosomes are also not indicative for aneuploidy. For example, on chromosome 11 we have a ZofZ- parameters 0.973 and this results in the plot as shown in figure 11.

1.3 ZZ2 computation for chromosome 16 for sample B

In sample B the median of the Z-scores for all chromosomes equals -0.2651 and the standard deviation equals 1.464. For chromosome 16, we have a Z-score of 5.754 which results in a ZZ2 parameter of 4.111, which is above the threshold of 3.

Indeed, if the Z-scores are plotted (figure 5), this clearly hints at a trisomy 16. This sample was tested with an invasive test and the trisomy was confirmed.

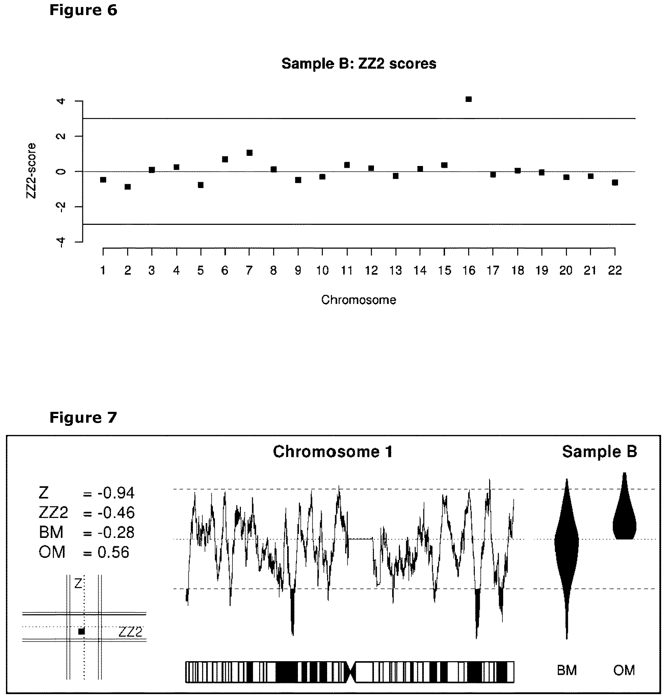

None of the other chromosomes in sample B have elevated ZZ2- parameters (figure 6).

The plots of the Z-scores of these other chromosomes are also not indicative for aneuploidy. For example, on chromosome 1 we have a ZZ2 parameter -0.459 which results in the plot shown in figure 7.

The plot of the BM scores of all autosomes as shown in figure 8 confirms that chromosome 16 is strongly aberrant, while other, potential autosomal aberrations will span less than half of the chromosome.

1.4 ZofZ computation for chromosome 16 for sample B

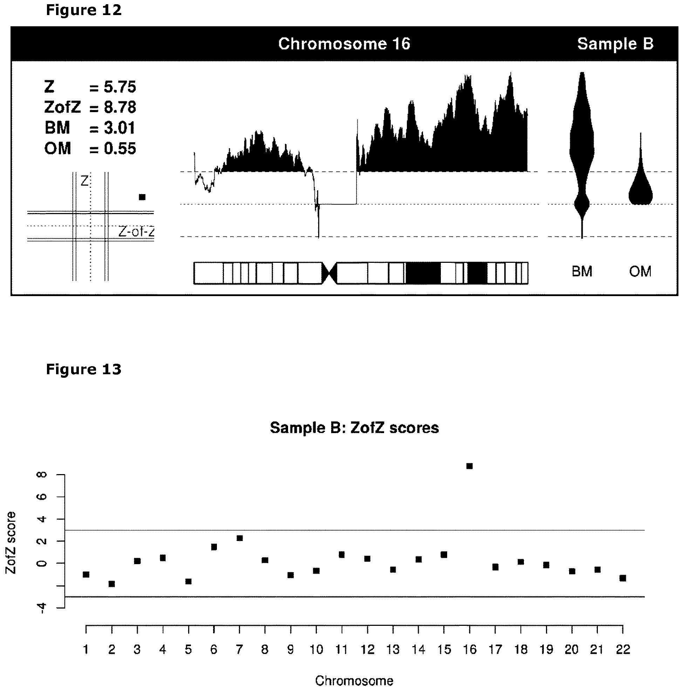

For sample B, the median of the Z-scores for all chromosomes equals -0.265 and the median absolute deviation equals 0.685. For chromosome 16, we have a Z-score of 5.754 which results in a ZofZ- parameter of 8.782, above the threshold of 3. Indeed, if we plot the Z-scores, this clearly hints at a trisomy 16 (figure 12). This sample was tested with an invasive test and the trisomy was confirmed.

Based on these ZofZ- parameters and a threshold of 3, none of the other chromosomes would be called aneuploidy (figure 13).

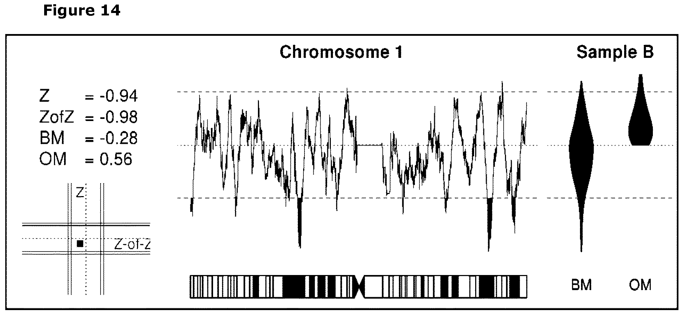

The plots of these other chromosomes are also not indicative for aneuploidy. For example, on chromosome 1 we have a ZofZ- parameter -0.980 (figure 14).

1.5 The sample quality assessment via OM and QS

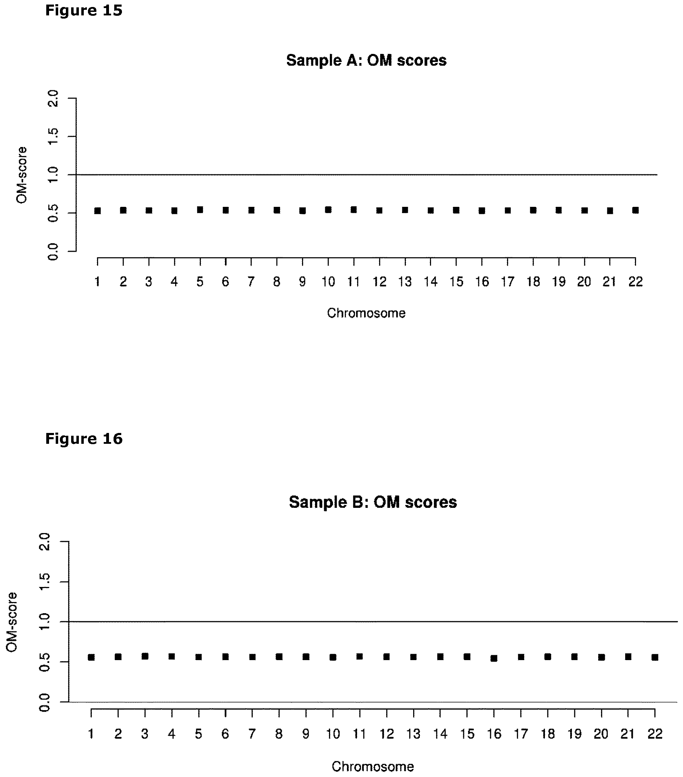

Samples A and B were two clear cases in which one chromosome is trisomic, while all other autosomes behave diploid. The corresponding plots show the OM values of the autosomes in samples A and B and all OM values confirm that this was a successful experiment. This is also confirmed by low QS scores (i.e., 0.576 for sample A and 0.652 for sample B, see figure 15 and 16)).

**Example 2**

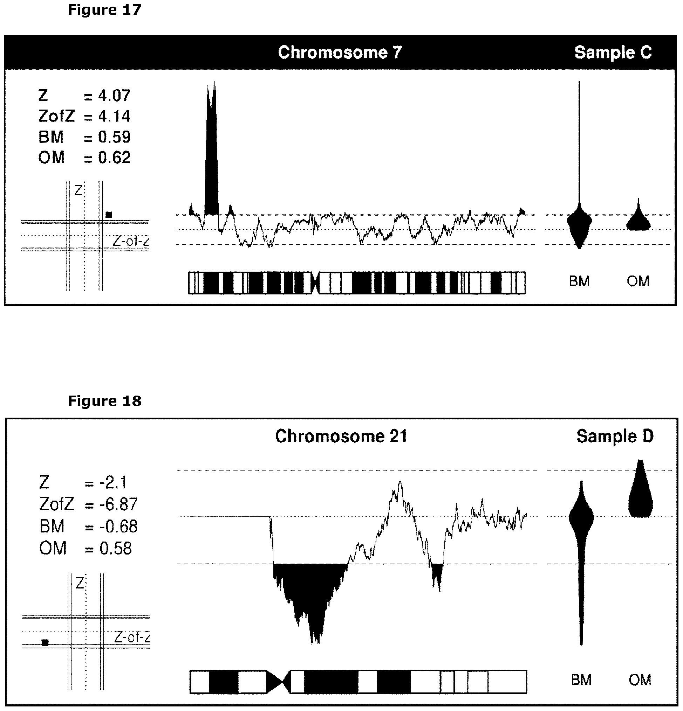

In sample C we observe a ZofZ- parameter of 4.141 for chromosome 7, while the BM score remains low. Figure 17 shows a plot of chromosome 7, where a part of the chromosome that is likely to have higher copy number is observed. This can indicate a maternal CNV.

Hence, ZofZ parameters can also be indicative for segmental CNVs

Automated classification of the chromosome using ZofZ>3 is very sensitive for picking up CNVs (see Sample C) or larger aneuploidies (see Sample A and B). Hence ZofZ can be used to identify abnormal chromosomes, and visual inspection of the plot can confirm the presence of such abnormalities.

Automated classification of the chromosome using a combination of ZofZ>3 with another parameter, can further improve the specificity of the automated classification, and add more granularity to the results. If e.g. ZofZ>3 and BM<1, this can indicate the presence of a CNV (see Sample C), while if e.g. ZofZ>3 and BM>1, this can indicate the presence of larger aneuploidy (see Samples A and B).

**Example 3: ZofZ more sensitive than ZZ**

The ZofZ- parameter can be more sensitive as compared to ZZ for the identification of CNVs. ZofZ- parameter can also be more sensitive as compared to ZZ for the identification of CNVs or larger aneuploidies in noisy samples.

3.1 Segmental aberration on chromosome 21

In sample D a ZofZ- parameter of -6.873 for chromosome 21 was observed, while the ZZ score as described in Bayindir et al. 2015 (which was used as reference method) resulted in a ZZ- parameter of -2.341 (i.e., not significant). In the plot of that chromosome as shown in figure 18, we observe a part of the chromosome that seems to be under-represented. This can indicate a maternal or a fetal CNV.

3.2 Segmental aberration on chromosome 15

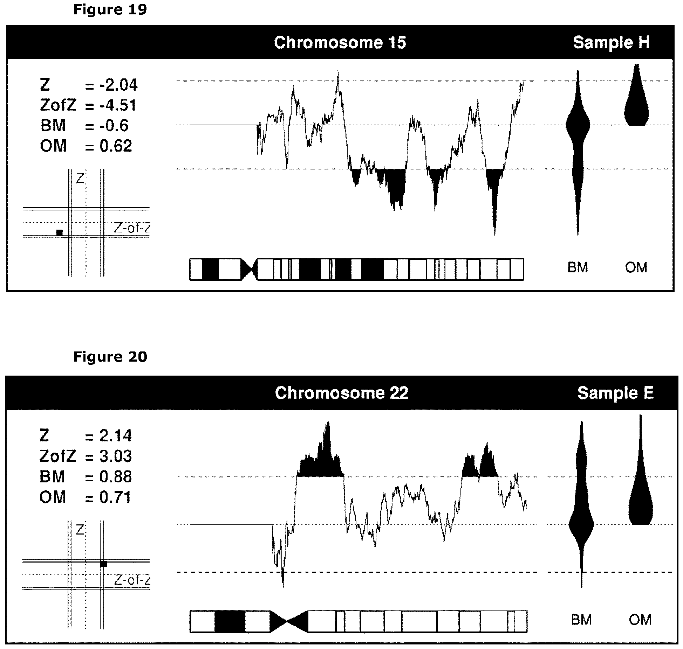

Based on the ZZ-score and the decision tree described in Bayindir et al. 2015, this chromosome 15 would be classified as a normal profile (ZZ =-2.730). The ZofZ parameter of -4.51 however draws attention to this chromosome (see figure 19). The BM score equals -0.6, which indicates that the aberration is partial, as could also be deduced from visual inspection of the plot.

3.3 Segmental aberration on chromosome 22

In sample E a ZofZ- parameter of 3.029 was observed for chromosome 22 (see figure 20), while the ZZ parameter as described in Bayindir et al. 2015 resulted in a ZZ- parameterof -0.629 (i.e., not significant).

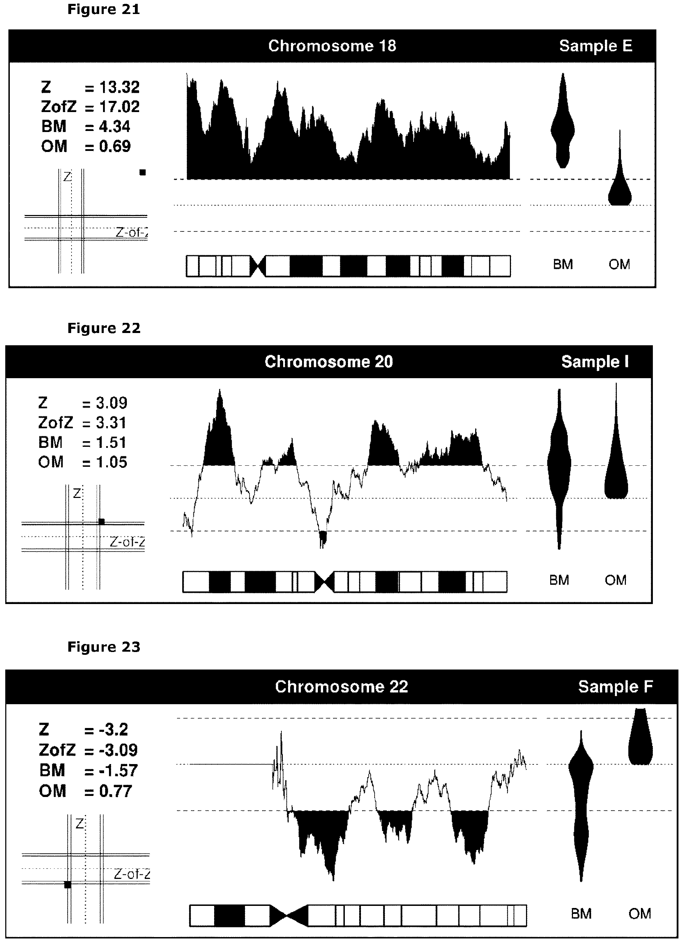

Based on the ZZ- parameter and the decision tree described in Bayindir et al. 2015, this chromosome would not be flagged and classified as normal. This could be due to the fact that this sample also has a trisomy 18 (see figure 21).

3.4 Segmental aberration on chromosome 20

In sample I, the ZZ-score as described in Bayindir et al. 2015 would be less than 3 (i.e., ZZ=2.195), while the ZofZ- parameter equals 3.31. Also the BM score equals 1.51, indicating that more than half of the Z-scores are increased. The OM score of 1.05 shows that this dataset was rather noisy (see figure 22).

3.5 Indication for monosomy of chromosome 22

In sample F we observe a ZofZ- parameter of -3.094 for chromosome 22, while the ZZ parameter calculated as described in Bayindir et al. 2015 equals -1.771 (i.e., not significant) (figure 23). The ZZ parameter would result in a normal value. Sample F has a trisomy 21 that perhaps masks this monosomic behavior of chromosome 22. Note that the trisomy 21 was confirmed via invasive follow-up. There were no follow-up data for chromosome 22.

The latter results all show that the method according to the present teachings are more sensitive than the methodologies known to date. The visual representation of the data as shown in the figures allows automated classification and interpretation. Moreover, the visualization according to the present teachings allow distinguishing between technical noise and noise which is due to aneuploidies.

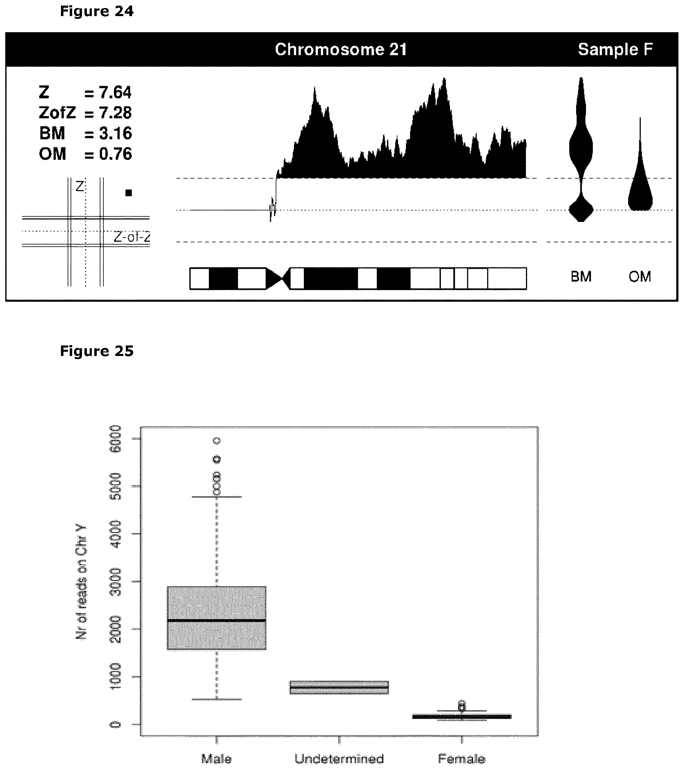

If the data are noisy, and vary along the Z=0 axis (i.e. both higher and lower than 0), then this is more likely to be technical noise. While if the data do not vary along the Z = 0 axis for a large chromosomal segment, this is more likely to be a real aneuploidy. From the plot in figure 24, it is obvious that the view is not due to technical noise but due to an aberrant situation.

**Example 4: Gender determination**

The gender of the samples can be determined by assessing the number of reads mapping to twenty 50kb bins on chromosome Y that were empirically selected to be specific for male pregnancies (see Bayindir 2015 for further details). In case at least 3 or more Y-specific bins contain more than 1 read, the gender was determined to be male. In case at most 1 bin contained 1 read or none of the 20 bins contained a read, the gender was said to be female. In all other cases, no gender specification was done and the gender was said to be undetermined. This could be due to e.g. a vanishing twin; where the blood sample contains fetal DNA of two fetuses instead of one.

In a set of 249 succeeded experiments (i.e., QS-score was lower than 1.5 and the number of reads remaining after all filtering steps was higher than 7,000,000), the gender was determined and this resulted in a set of 116 (46.59%) female pregnancies, 131 (52.61%) male pregnancies and 2 (0.80%) undetermined pregnancies. The plots as shown in figure 24 and 25 for these 249 samples show the number of reads that mapped to the Y-chromosome (after filtering the BAM file).

4.1 Fetal fraction determination for male pregnancies

Once the gender is determined, fetal fraction can be determined for the male pregnancies. For male pregnancies, one can benefit from the fact that the fetal DNA will only have 1 copy of X and a copy of Y, instead of the 2 copies of X that are present in the maternal DNA. This allows estimating the fetal fraction in two ways. Based on the X-chromosomes, fetal fraction can be computed as twice the difference at the 50kb bin level between the median number of reads mapping to the autosomes and the median number of reads mapping to chromosome X, divided by the median number of reads mapping to the autosomes. This can be written as the following formula:

$$\mathrm{FF_X} = 2 \times \left| 1 - \frac{\underset{\mathrm{on}\,X}{\mathrm{median}}(\mathrm{GC} - \mathrm{corr\ counts})}{\underset{\mathrm{on\ autosomes}}{\mathrm{median}}(\mathrm{GC} - \mathrm{corr\ counts})} \right|$$

Secondly, fetal fraction can also be estimated based on the Y-chromosome as all reads that map to chromosome Y should in theory originate from the fetal DNA. Chromosome Y-based fetal fraction is defined as twice the median number of GC-corrected reads mapping to Y over the median number of GC-corrected reads mapping to the autosomes, or in a formula:

$$\mathrm{FF_Y} = 2 \times \frac{\underset{\mathrm{over}\,Y}{\mathrm{median}}(\mathrm{GC} - \mathrm{corr\ counts})}{\underset{\mathrm{on\ autosomes}}{\mathrm{median}}(\mathrm{GC} - \mathrm{corr\ counts})}$$

Disadvantage of this approach is that fetal fraction can only be determined for about half of the samples (i.e. only for the male pregnancies). In our previous examples fetal fractions were computed. Results are shown in the Table I.

**Table I**

| Sample | Sample description | Gender | FF$_X$ | FF$_Y$ |
|--------|--------------------|--------|--------|--------|
| A | Trisomy 21 sample | Male | 8.6135 | 7.4662 |
| B | Trisomy 16 sample | Female | | |
| C | Maternal CNV on chr 7 | Female | | |
| E | Trisomy 18 and segmental aberration chr 22 | Female | | |
| D | Segmental aberration chr 21 | Female | | |
| F | Trisomy 21 and monosomic behavior for chr 22 | Male | 7.5631 | 6.3376 |
| G | Failed sample (QS = 27.526) | Male | 88.1395 | 48.7756 |
| H | Segmental aberration chr 15 | Male | 17.8092 | 15.9849 |
| I | Segmental aberration chr 20 | Female | | |

Figure 25 shows for the 131 male pregnancies, identified in example 4.1, the X and Y-based fetal fractions.

**Example 6: CNV based approach to determine a minority fraction (e.g Fetal fraction)**

The methodology of the present teachings can be exemplified if one assumes a sample with a fetal fraction of 10%, that was sequenced at a coverage of 0.1X to yield 50 bp reads. Further one should assume a CNV of 10 Mb.
In case of a normal sample (i.e., 2 copies of the CNV) consisting out of 100% DNA of the patient, 20,000 reads mapping to that CNV region is expected. In case the patient has only 1 copy of the particular CNV, 10,000 reads are expected in that region; in case of 0 copies, no reads are expected.
In case of a minority fraction of 10%, the following cases can be expected (Table II):

**Table II**

| # copies woman | # copies fetus | Expected # reads |
|---|---|---|
| 2 | 2 | ~20,000 |
| 2 | 1 | ~19,000 |
| 2 | 0 | ~18,000 |
| 1 | 2 | ~11,000 |
| 1 | 1 | ~10,000 |
| 1 | 0 | ~9,000 |
| 0 | 2 | ~2,000 |
| 0 | 1 | ~1,000 |
| 0 | 0 | ~0 |

In case the CNV is found with far too low, non-zero coverage, it can be concluded that the mother does not have this region (0 copies) and that the reads which are observed come from the minority DNA. Hence the CNV can be considered to be informative for the determination of the fetal fraction.

Hence the minority fraction can be determined as:

$$2*observed\ reads/expected\ reads = 2*1,000/20,000=10\%$$

As an estimate for the expected number of fragments, the global coverage of the sample can be used as well as the length of the CNV. A correction for overall read depth can occur and the expected coverage based on all samples can be computed, by e.g. taking the mean after excluding the top and bottom 10% of the samples or by using the median coverage.

In order to correct for recurrent technical noise, a CNV specific attribute can be calculated using samples with known fetal fraction, in which the attribute can correct the obtained read counts for recurrent technical noise on that particular CNV.

The sequences can be obtained as described above, except that filtering of reads that lie in blacklisted regions was not applied.

Computing coverage per CNV

A CNV reference genome database was used, containing 581774 CNVs. Note that the CNVs in this set were not all unique CNVs as the database contains overlaps.

The reads were aligned against the reference genome. For each CNV, the number of reads was counted that showed overlap of at least X bases with the CNV regions, whereby X can be any value between 1 and 50. In the current example, X was set to 1. This results in a matrix with the raw counts. Raw counts equal or less than X are defined as equal to 0, as they display minimal overlap with the CNV.

Because the raw counts were not corrected for the total number of reads, the total number of filtered reads was extracted for each sample and was corrected by the following equation:

$$normalized\ count = 10,000,000 * raw\ count/total\ number\ of\ reads;$$

whereby 10,000,000 was arbitrary chosen and could be replaced by any other value.

Previously determined fetal fractions obtained for a set of male pregnancies were imported. These fetal fractions are based on the counts on the X and Y chromosome and use the fact that in male pregnancies the fetal DNA only has 1 copy of chromosome X and one copy of chromosome Y; while the maternal DNA has two copies of chromosome X. Hence the X and Y-based fetal fraction method can only give an estimate on the fetal fraction for the male pregnancies (as opposed to the CNV based fetal fraction method presented here).

The previously obtained fetal fractions were filtered to remove samples with a fetal fraction smaller than Y. Y can be any value between 0.05 and 1. In the current example said Y was set to 0.3, as this is considered to be the maximal fetal fraction that one would routinely encounter

Determining of informative CNVs in a set of CNVs

In a subsequent step, informative CNVs were determined. For each CNV, samples were identified for which only the fetus had 1 or 2 copies and whereby the mother had no copies. All counts are thus derived from the fetus. Next it was checked whether for the male pregnancies these fetal counts correlate to the X/Y based fetal factions, thereby resulting in a X-based and Y-based correlation. This gave insight in which CNVs were informative for fetal fractions.
As an example, the following 3 random CNVs were considered, covering different lengths:

1. Chr 1: 72,773,259-72,798,581; this is a region of 25kb (25,323bp)
2. Chr 1: 148,539,255-149,765,886; this is a region of 1Mb (1,226,632bp)
3. Chr 9: 38,725,590-71,025,693; this is a region of 32Mb (32,300,104bp)

In a histogram (not shown) of the normalized counts (normalized with regard to the total number of reads) for CNV 1, 3 peaks are observed (likely to be corresponding to 0, 1 and 2 maternal copies). For CNV 2, 4 large peaks were observed (likely to be corresponding to 0, 1, 2 and 3 maternal copies). In the third and largest CNV no peaks were observable. Therefore, analysis is continued with CNV 1 and 2.
To find the local minima, a density function was fitted and the signs of the derivative of the density functions were checked.
The two local minima were at:

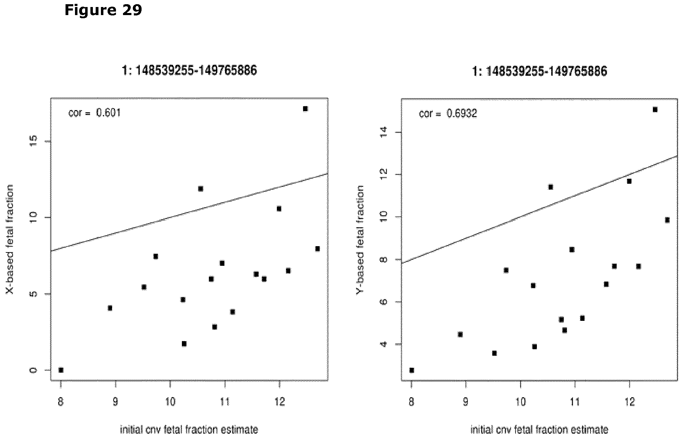

1. CNV 1: 18.39 and 67.96
2. CNV 2: 373.12; 766.69; 1160.26; 1538.69; 1568.97; 1705.20 and 1856.57 (see figure 29).

The normalized counts were extracted for those samples that have normalized counts smaller than the smallest local minimum and larger than 0. It is assumed that the counts for these samples are mainly derived from the fetal DNA and have minimal to low contribution from the maternal DNA.
Based on the normalized counts, the expected count was computed for each CNV. The normalized counts were normalized towards an arbitrary chosen value of 10,000,000 reads.
With this expected count, an estimate of the fetal fractions was computed as:

$$2 \text{ x } 100 \text{ x observed counts/expected counts.}$$

This estimate of the fetal fraction is correlated with the actual fetal fraction. However, it was found to be not identical. In fact, the estimated fetal fraction can be seen as:

$$\text{estimated fetal fraction} = \text{factor} * \text{actual fetal fraction.}$$

This factor is a constant factor that can be considered as an attribute of each CNV in the dataset, and which is to be empirically determined (see further).

Examples of determining fetal fraction:

a) for CNV 1:

Fetal fraction can be estimated as

$$2*100*\text{normalized count/expected counts}=2*100*\text{normalized count } /88.58$$

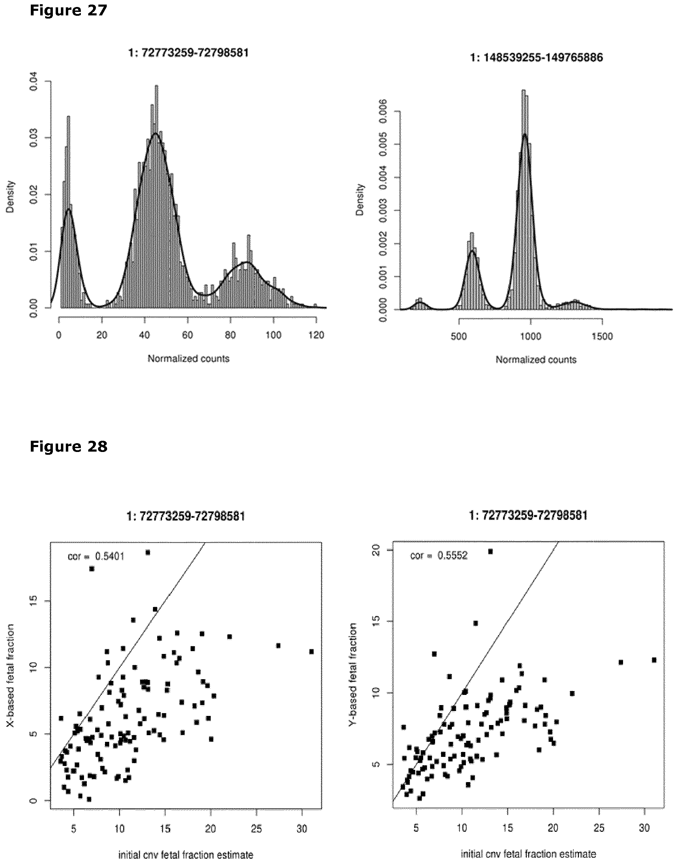

Figure 28 shows the computed fetal fractions for all male pregnancies with less than 18.54 counts for CNV 1 (i.e. 142 samples) versus the in-house fetal fraction computed via chromosome X (at the left) or chromosome Y (at the right). Correlations equal 0.54 and 0.56 with the X and Y-based fetal fractions respectively.

b) for CNV 2

Fetal fraction can be estimated as:

$$2*100*normalized\ count/expected\ counts = 2*100*normalized\ counts/4,290$$

Figure 29 shows the computed fetal fractions for all male pregnancies with less than 359.20 counts for CNV 2 (i.e., 17 samples) versus the fetal fraction computed via chromosome X (at the left) or chromosome Y (at the right). Correlations equal 0.601 and 0.963.

For all CNVs the local minimum, the local maximum, number of peaks, number of male pregnancies with a count lower than the smallest local minimum and the correlations with the X/Y-based fetal fractions was computed.

Based on this information, autosomal "pseudo-informative" CNVs were selected

- X or Y-based correlation larger than A (between 0.01 and 1, A was set as 0.5 in this example)
- based on at least B (between 1 and 100 or more, B was set as 8 in this example) male pregnancies
- having more than C peaks (between 0 and 5, C was set as 2).
- exclude the CNVs on the X and Y chromosome.
- Compare the first local minimum with the third local maximum. The ratio or correlation between the third local maximum and the first local minimum should be larger than D (between 0.1 and 100, D is set to 3).

This can strongly reduce the number of CNVs to anywhere between 1 and 100,000 or more "pseudo-informative" CNVs. In the current example about 5,000 "pseudo-informative" CNVs were identified.

Within the list of obtained pseudo-informative CNVs, a lot of overlapping CNVs were identified. The list was therefore cleaned by one or more of the following methodologies:

- Per set of overlapping CNVs, retain only A (A = between 1 and 100 or more, whereby A was set as 1 in this example) of them, i.e. the one with the highest average correlation (i.e., average of the X and Y based correlations.
- Removal of duplicates
- For very similar CNVs, only the longest CNV was retained

Note that a cleanup is optional but can strongly reduce the number of CNVs to anywhere between 1 and 100,000 or more "informative" CNVs. In the current example the number was reduced to about 100 "informative" CNVs.

In a subsequent step and for each of the informative CNVs, the normalized counts were scaled towards the X/Y based fetal fractions. As such, for each CNV it was evaluated how the read counts predict the fetal fraction. The method restricted to CNVs with a correlation above D (whereby D is between 0.01 and 1, and set as 0.5 in the current example). In case the X-based correlation was above D, while the Y-based correlation was below D, then only the X-based fetal fractions were taken into account. Note that the inverse is also possible.

As an example, CNV 1: 1,541,063-1,541,536 was considered. A regression line of the X(Y) based fetal fractions versus the normalized counts was fitted for the small counts seen in male pregnancies of succeeded experiments. This resulted in 2 regression lines, each with an intercept and slope, for all CNVs or all pseudo-informative CNVs, or all informative CNVs.

With the slope and intercept of these regression lines, for each CNV the corresponding, small normalized counts were scaled. Note that this scaling can be done for all samples (i.e., male and female samples). The following plot shows the scaled counts versus the X/Y based fetal fractions for the male pregnancies that had small counts for CNV 1: 1,541,063-1,541,536.

For each CNV and for all samples with small counts for that particular CNV two estimates of the fetal fraction have been obtained, the X and Y based fetal fraction. As third estimate, the average of the X and Y-based scaled counts is taken.

In case one of the 2 correlations, e.g. the X-based correlation, falls below E (whereby E is between 0.01 and 1, and set as 0.5 in the current example E = 0.5 in this example), then the average will be equal to the Y-based scaled counts.

For each sample three estimates for the fetal fraction for all CNVs which show a small number of reads within the sample are obtained:

- X based estimate
- Y based estimate
- averaged-scaled estimate

In a final step, the average of the CNV-based fetal fraction over all CNVs was obtained. In order to check whether this average reflects the chromosome X and Y-based fetal fractions for the male pregnancies, they were plotted versus each other for the succeeded experiments.

**Table III**

| Corr | Av CNV FF | X-scaled CNV FF | Y-scaled CNV FF |
|---|---|---|---|
| Chr-X FF | | | |
| | 0.7774543 | 0.7785994 | 0.7632756 |
| Chr-Y FF | 0.7988806 | 0.7963122 | 0.8066906 |

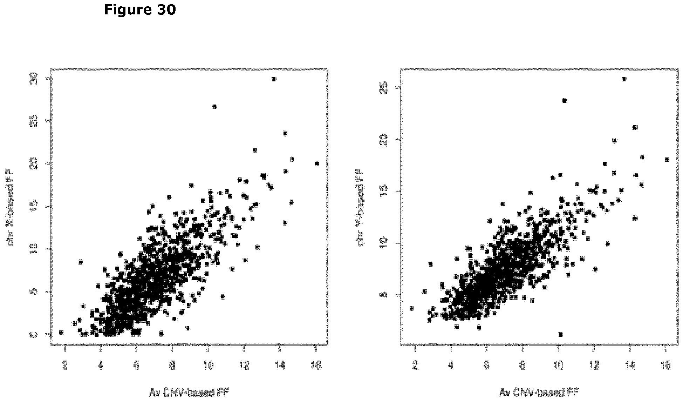

From figure 30 it is apparent that there is a clear correlation between the CNV based fetal fraction determination and the X and Y based fetal fraction determination. In order to use the CNV based fetal fraction method to estimate the actual fetal fraction, the scaling can be empirically adapted (i.e. the regression line can be adjusted to have a slope of 1).

**[0459]** * When referring to the Z score and/or the ZofZ parameter in the examples 7 to 10 which are described hereunder, the following formulas are being used throughout the examples:

Z- score:

$$Z_i = \frac{GR_i - \mu_i}{\sigma_i}$$

ZofZ parameter:

$$Zof Z_i = \frac{Z_i - \underset{j=1,\dots,22}{\mathrm{median}}(Z_j)}{\underset{j=1,\dots,22}{\mathrm{mad}}(Z_j)}$$

With the MAD-value for a data set $x_1, x_2, \dots, x_n$ computed as

$$\mathrm{MAD} = 1.4826 \times \mathrm{median}(|x_i - \mathrm{median}(x)|)$$

(an alternative MAD that does not use the factor 1.4826 can also be used).

### Example 7: Detection of a genome-wide instability in a sample

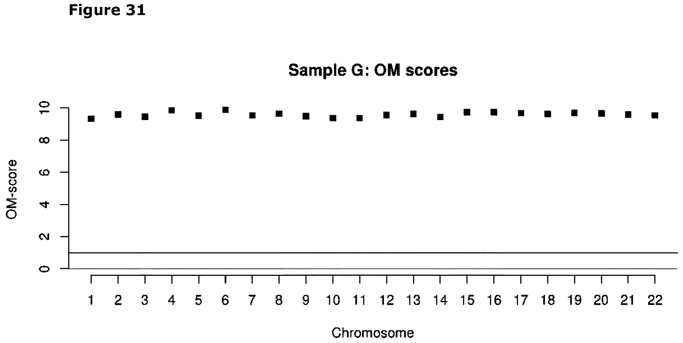

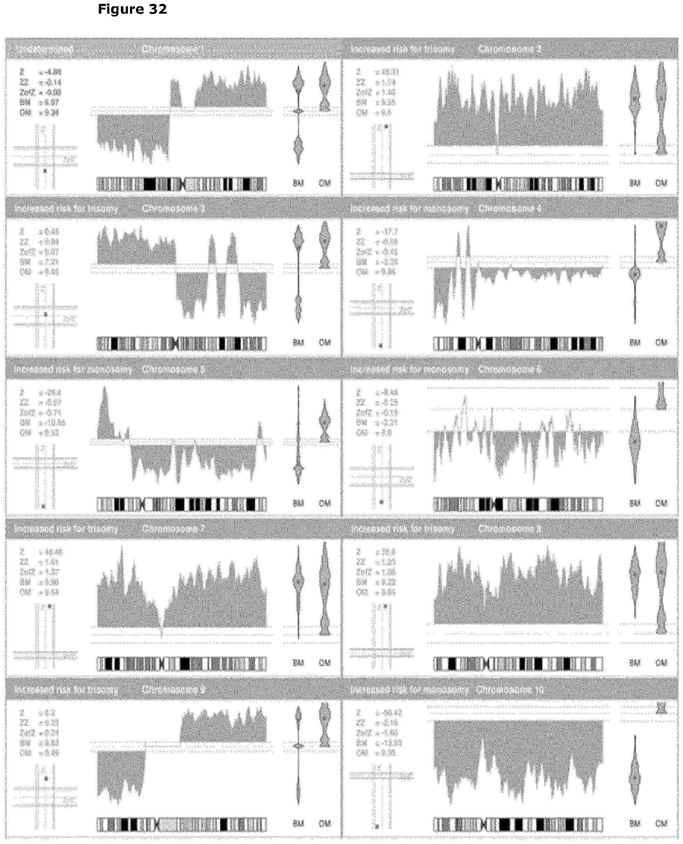

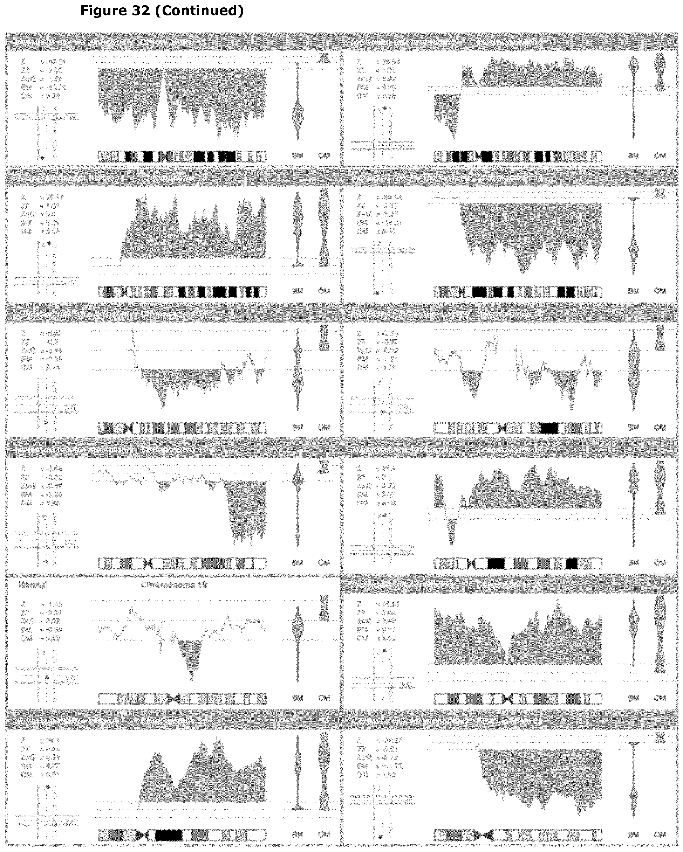

Analyzed sample G showed a QS score of 27.526. The plot of the OM values or the autosomes displayed extreme values (Figure 31). Visual inspection of the plots of the individual autosomes confirmed that this sample behaved abnormal (Figure 32). This type of overall highly variable patterns in the Z-scores is indicative for genome-wide instability.

### Example 8: Advantage of the ZofZ parameter vs prior art parameter from Bayindir et al. (2015)

Four samples were processed as describes above, and scores and parameters were calculated, either via the methodology as described in Bayindir et al. (2015) or via the methodology of the present teachings, resulting in respectively a ZZ parameter and a ZofZ parameter.

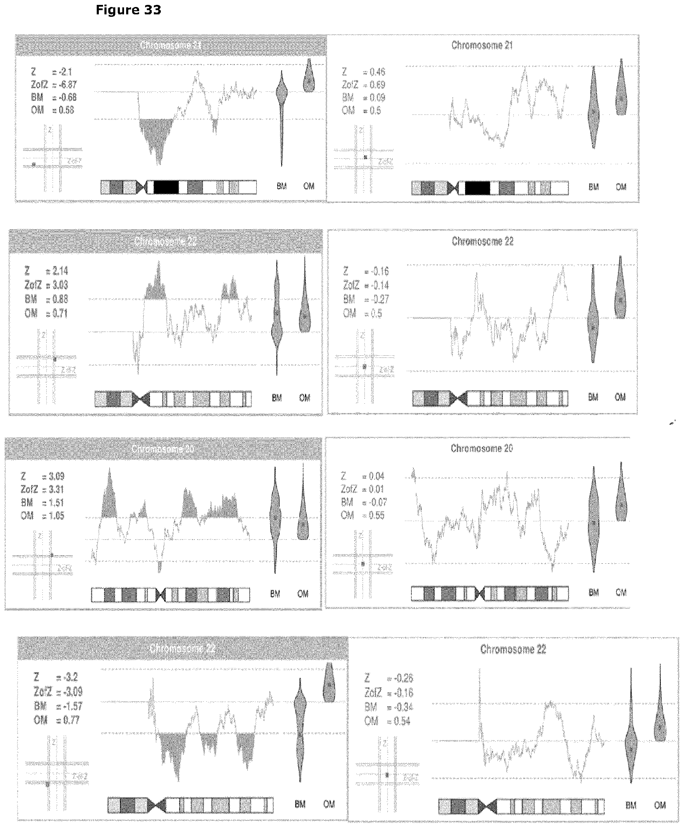

Figure 33 shows the results of these analyses, whereby the graphs in the left column depict the results of 4 chromosomes which, according to methodology of the present teachings, were clearly abnormal when compared to a normal chromosome (graphs in the right column).

Despite clearly been found to behave abnormal by the current methodology, these chromosomes were labeled as 'normal' by the prior art Bayindir methodology when using the same thresholds as for the present teachings (larger than +3 or smaller than -3). The ZZ score was not significant:

- ZZ= -2.341 for chromosome 21

- ZZ= -0.629 for chromosome 22
- ZZ= - 2.195 for chromosome 20
- ZZ= -0.629 for chromosome 22 (last line).

This shows that the prior art methodology was not sensitive enough to pick up these abnormalities. In contrast, the ZofZ parameter was significant, which shows that ZofZ has a better sensitivity and is capable of detecting abnormal chromosomes which are missed by using the prior art methodology.

**Example 8: Advantage of the ZofZ with respect to lack of critical dependency on training data**

Typical NIPT analysis pipelines known in the art rely on formulas that require training using "training data", i.e. data generated from large numbers of samples that are tested using a specific NGS library prep kit and NGS platform, and for which for each sample also a Gold Standard (i.e. the reference method that establishes the ground truth of the sample) needs to be available. These formulas are then being "empirically" trained by the test data to give the same result as the Gold Standard with an as high accuracy (sensitivity and specificity) as possible. Hence, users that want to start-up an NIPT analysis with a new NGS library prep kit or new NGS platform and which do not have these large numbers of samples available (along with the Gold Standard result) are unable to use currently known methodologies.

The present teachings overcome this burden as the methodology according to the present teachings (e.g. Z and ZofZ formulas) merely require the availability of a small set of samples (e.g., 24 in the example below) and optionally access to a database with excluded regions. This database of excluded regions can consist of e.g. regions that are commonly known to introduce noise during mapping or regions that are known to contain benign CNVs. The database can consist of e.g. a combination of common polymorphic CNVs, collapsed repeats, DAC blacklisted regions generated for the ENCODE project and the undefined portion of the reference genome (i.e., the N's). The database can be readily assembled based on the sequence of the reference genome and publicly available CNV databases, and does not require access to proprietary databases.

A reference set was created consisting out of 20 normal samples, 3 samples with a trisomy 21 and a sample with a trisomy 18 (i.e. a non-curated reference set that contains a mixture of normal and abnormal samples). For each of the 24 reference samples, the Z-score and the ZofZ-score against these 24 reference samples was computed, according to the methodology of the present teachings.

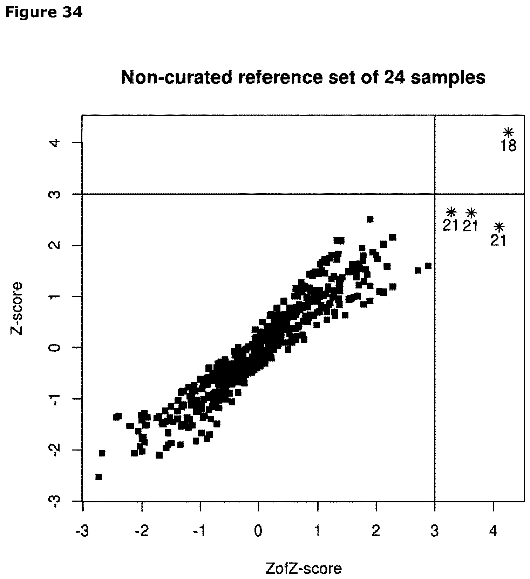

Figure 34 shows the plot of these Z and ZofZ-scores. Chromosome 21 and 18 in the 4 aneuploid samples are indicated in the plot, table IV provides the results obtained via the methodology of the present teachings.

**Table IV**

| Sample | Chr | Z-score | ZofZ-score | BM-score | OM-score |
|---|---|---|---|---|---|
| Trisomy 21 samples | 21 | 2.36 | 4.10 | 2.24 | 0.72 |
| | 21 | 2.63 | 3.62 | 2.29 | 0.59 |
| | 21 | 2.65 | 3.28 | 2.44 | 0.69 |
| Trisomy 18 sample | 18 | 4.21 | 4.25 | 2.49 | 0.73 |

Although the reference set is not perfect (4 out of 24 samples (16.67%) carry an aberration, while the prevalence of abnormal samples in routine NIPT testing is typically <5%), the method according to the present teachingsis robust enough to detect these aneuploidies. For the sample with the trisomy 18, both the Z-score and the ZofZ-parameter are larger than 3 which indicates the presence of an aneuploidy. For the three samples with trisomy 21, the Z-score is borderline (a.o. due to the higher standard deviation caused by the 3 trisomy 21 cases in the reference set), but it is clear that the ZofZ-parameter is robust enough to detect that these three chromosomes are outlying chromosomes (i.e. ZofZ parameter >3), compared to the other chromosomes within the sample.

Hence, both the Z and ZofZ parameter of the present teachings are straightforward to implement, and do not require large amounts of training data nor the availability of results from the Gold Standard to tune the parameter settings. This contrasts most of the alternative methodologies which do require large amounts of training data and the availability of results from the Gold Standard to enable for instance selection of reference and/or normalizing regions in the genome.

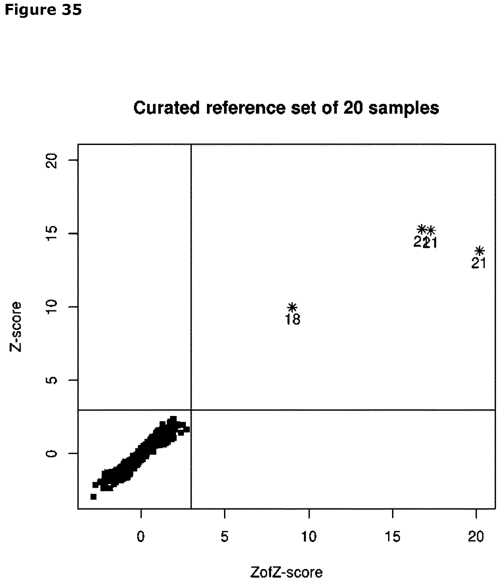

Optionally, one could further increase the sensitivity of the Z and ZofZ parameters by removing the identified abnormal samples from the reference set, thereby making a curated reference set that contains only normal (diploid) samples. After removing the 4 aneuploid samples from the reference set and repeating the analysis for the 24 samples based on the 20 selected diploid reference samples, the following Z score and ZofZ-parameter were obtained (Table V and figure 35)

**Table V**

| Sample | Chr | Z-score | ZofZ-score | BM-score | OM-score |
|---|---|---|---|---|---|
| Trisomy 21 samples | 21 | 13.83 | 20.19 | 6.20 | 0.77 |
| | 21 | 15.22 | 17.28 | 6.57 | 0.63 |
| | 21 | 15.31 | 16.75 | 6.50 | 0.73 |
| Trisomy 18 sample | 18 | 9.99 | 9.01 | 2.95 | 0.77 |

It is clear that both the Z score and the ZofZ-parameter after removing the previously identified abnormal samples are now even more pronounced for the 4 trisomies. Hence, the sensitivity of the Z and ZofZ parameters were further increased by cleaning up the reference set using a methodology that does not require the use of large amounts of training data nor the availability of a Gold Standard result.

In fact, it sufficed to have only 24 test samples available (which can be readily pooled and processed on a single NGS run, like e.g. NextSeq500 in High Output mode) of which only 20 samples were found to be normal.

### Example 9: Advantage of our combined Z and ZofZ parameter in situations where the normalizing chromosome is found to show abnormalities

Typical prior art NIPT analysis methods often require the use of one or more normalizing chromosomes and are thereby totally dependent on the assumption that the normalizing chromosomes within a sample are normal, i.e. diploid. In some settings, this can be a wrong assumption; leading to a wrong conclusion.

Bianchi et al. (2015) reported a set of cases from pregnant women with presymptomatic cancer. In those cases, the assumption that the normalizing chromosomes are normal was not fulfilled, because the majority of the DNA is maternal (fetal fraction typically constitutes only 2-20%) and that maternal DNA suffered from genome instability (i.e. cancer). In several cases, this led to a wrong conclusion with regard to the presence or absence of an aneuploidy (e.g. monosomy 18, while chromosome 18 was perfectly normal).

In contrast, the Z and ZofZ parameter according to the present teachings can be used to correctly assess the ploidy status of the sample, as it does not make use of any normalizing chromosome in the sample. As a consequence, the current methodology allows assessing the aneuploidy status of the target chromosome irrespectively of the aneuploidy status of the other chromosomes in the sample (as it merely compares to the same chromosome in the reference set) and can correctly identify whether a chromosome (or chromosome portion) is increased or decreased in read count as compared to that normal reference set.

In addition, the ZofZ parameter provides the advantage that the behavior of the target chromosome may be compared to the overall behavior of the chromosomes in the sample, and can aid in the identification of the presence of e.g. genome-wide instability and cancer.

Set up of the NCV score

A traditional analysis, known in the art and relying on an internal normalizing chromosome was compared to the analysis of the present teachings.

For the traditional analysis, the starting point were reads mapped to a reference genome and a cleaned BAM file whereby duplicate reads, non-unique reads, non-perfect match reads and reads in an excluded region are removed (note that this clean-up of the BAM file is optional and that BAM files can be cleaned up in alternative ways). The number of reads mapping to a chromosome A of interest and the number reads mapping to a normalizing chromosome B were counted. A chromosome dose could then be defined as:

$$dose_{A(B)} = \frac{\text{\# reads mapping to chr A} / \text{length of chr A}}{\text{\# reads mapping to chr B} / \text{length of chr B}}$$

Next, the mean $\mu_{A(B)}$ and standard deviation $\sigma_{A(B)}$ of the chromosome doses of chromosome A versus the normalizing chromosome B in a set of diploid samples was computed. The metrics $\mu_{A(B)}$ and $\sigma_{A(B)}$ reflect the chromosome doses that can be expected in a diploid sample. Based on this mean and standard deviation, a so-called *normalized chromosome value* (NCV) can be computed as the following significance score:

$$NCV_{A(B)} = \frac{dose_{A(B)} - \mu_{A(B)}}{\sigma_{A(B)}}$$

In case the $NCV_{A(B)}$ is larger than e.g. 3, more reads mapped to chromosome A than what is expected in a normal sample and one can conclude that it is highly likely that the test sample has more than 2 copies of chromosome A (hence aneuploidy). Analogously, a $NCV_{A(B)}$ score smaller than -3 indicates that the test sample has less than 2 copies.

To demonstrate this score, 20 normal, diploid samples and 4 samples with a trisomy (i.e., 3 x trisomy 21 and 1 x trisomy 18) were selected.

As a reference chromosome B, example chromosome 7 in one case (left) and chromosome 11 in another case (right) were chosen. Other reference chromosomes and/or combination of normalizing chromosomes can be selected based on the results obtained from testing large numbers of samples and the availability of the Gold Standard result. For all of the 24 samples, the chromosome doses for all other autosomes versus each of these normalizing chromosomes were computed. Next, the mean and the standard deviation of these chromosome doses over the 20 normal, diploid samples were computed (see Table VI)

**Table VI**

| chr | mean dose $\mu_{A(7)}$ | sd of dose $\sigma_{A(7)}$ | mean dose $\mu_{A(11)}$ | sd of dose $\sigma_{A(11)}$ |
|---|---|---|---|---|
| 1 | 1.0615 | 0.0040 | 0.9948 | 0.0044 |
| 2 | 1.0761 | 0.0025 | 1.0085 | 0.0039 |
| 3 | 1.0968 | 0.0032 | 1.0279 | 0.0030 |
| 4 | 1.0589 | 0.0020 | 0.9924 | 0.0036 |
| 5 | 1.0763 | 0.0033 | 1.0087 | 0.0020 |
| 6 | 1.0717 | 0.0024 | 1.0044 | 0.0044 |
| 7 | 1.0000 | 0.0000 | 0.9372 | 0.0034 |
| 8 | 1.0770 | 0.0032 | 1.0093 | 0.0024 |
| 9 | 0.9748 | 0.0028 | 0.9135 | 0.0029 |
| 10 | 1.0614 | 0.0035 | 0.9947 | 0.0043 |
| 11 | 1.0670 | 0.0039 | 1.0000 | 0.0000 |
| 12 | 1.0609 | 0.0030 | 0.9943 | 0.0039 |
| 13 | 1.0818 | 0.0032 | 1.0139 | 0.0043 |
| 14 | 1.0534 | 0.0035 | 0.9872 | 0.0043 |
| 15 | 1.0051 | 0.0045 | 0.9420 | 0.0048 |
| 16 | 0.9685 | 0.0068 | 0.9077 | 0.0054 |
| 17 | 0.9987 | 0.0054 | 0.9360 | 0.0061 |
| 18 | 1.1040 | 0.0040 | 1.0346 | 0.0028 |
| 19 | 0.8506 | 0.0083 | 0.7972 | 0.0079 |
| 20 | 1.1193 | 0.0068 | 1.0490 | 0.0038 |
| 21 | 1.0162 | 0.0047 | 0.9523 | 0.0043 |
| 22 | 0.9855 | 0.0052 | 0.9236 | 0.0053 |

Based on these average doses and standard deviations of the doses, the NCV scores for all 24 samples and all chromosomes versus the normalizing chromosome 7 (first graph) or chromosome 11 (second graph) were computed.

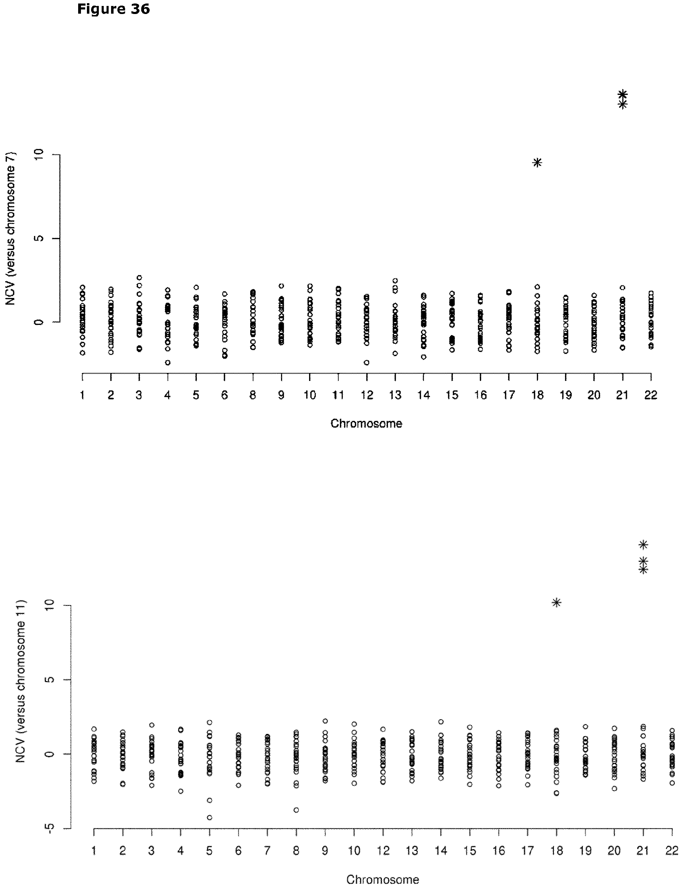

Figure 36 shows for all 24 samples and for all chromosomes the NCV scores. The scores of the 4 trisomies are indicated with a star.

Hence, by calling all chromosomes with a score larger than 3, the four trisomies were detected in both cases. However, as we will show next, this NCV methodology does not work properly if the normalizing chromosome is not normal, as can be the case when the mother has an amplification or deletion on the selected normalizing chromosome, or when the mother has cancer.

<u>First example of poor performance of methods relying on normalizing chromosomes</u>

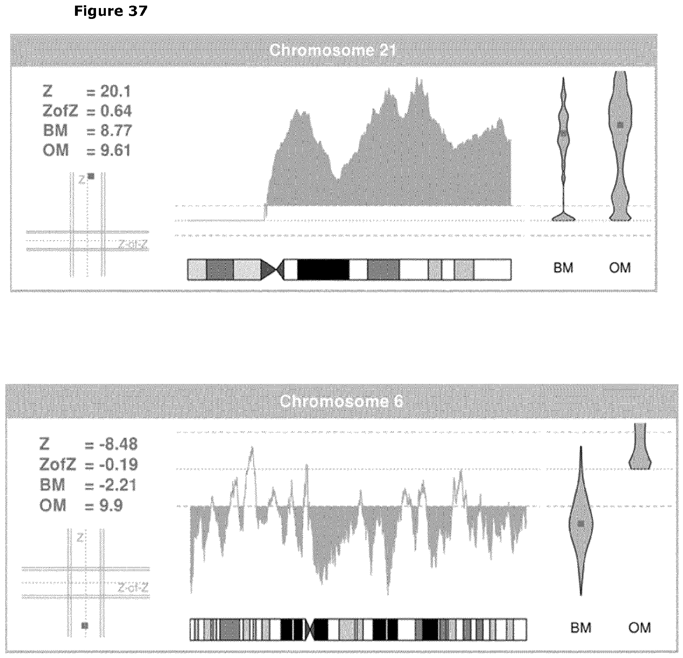

A sample derived from a pregnant woman with cancer with multiple chromosomal abnormalities that affect amongst others chromosome 21 and chromosome 6 was used in this particular example. From the plot in figure 37, it can be seen that there is a gain for chromosome 21 which is confirmed by the Z-score that is significantly increased (Z-score = 20.096). The ZofZ-parameter was not significant (ZofZ-parameter= 0.6354), as there are many abnormal chromosomes in the sample. Also from figure 37 it can be derived that there is a loss for chromosome 6, which is also confirmed by the Z-score that is significantly decreased (Z-score = -8.4795). Also here, the ZofZ-parameter was not significant (ZofZ-score = -0.1865), as there are many abnormal chromosomes in the sample.

When applying the traditional methodology, based on the NCV score, on this sample, the following results are obtained. In this example, chromosome 7 is used as normalizing chromosome, and the chromosome doses of chromosome 6 and 21 were calculated versus normalizing chromosome 7.

Next, the obtained values were compared with the average and standard deviation of the chromosome doses in the 20 diploid samples by computing the NCV-score. All numbers are shown in the following table VII.

**Table VII**

| Chr A | chr dose $dose_{A(7)}$ | mean dose $\mu_{A(7)}$ | sd of dose $\sigma_{A(7)}$ | NCV-**score** $NCV_{A(7)}$ |
|---|---|---|---|---|
| 6 | 0.9485 | 1.0717 | 0.0024 | -51.3059 |
| 21 | 1.0075 | 1.0162 | 0.0047 | -1.8255 |

With a cut-off of +3 the loss of chromosome 6 was correctly detected, but the gain of chromosome 21 was left undetected. In contrast, the NCV score for chromosome 21 is even slightly negative (-1.8255).

In case chromosome 11 was used as normalizing chromosome, the gain of chromosome 21 with a cut-off of $\pm3$, could be detected but the loss of chromosome 6 was not picked up. In fact, chromosome 6 would have been reported as a gain, which is completely the opposite of its true state (see Table VIII).

**Table VII**

| Chr A | chr dose $dose_{A(11)}$ | mean dose $\mu_{A(11)}$ | sd of dose $\sigma_{A(11)}$ | **NCV-score** $NCV_{A(11)}$ |
|---|---|---|---|---|
| 6 | 1.1360 | 1.0044 | 0.0044 | 29.6548 |
| 21 | 1.2066 | 0.9523 | 0.0043 | 59.1109 |

To conclude, the traditional NCV method is not robust in the case where multiple abnormalities in a sample are present as this can lead to incorrect diagnosis. The methodology of the present teachingson the other hand is completely independent from the presence of an internal normalizing chromosome and is able to robustly detect anomalies present in a sample.

<u>Second example of poor performance of methods relying on normalizing chromosomes</u>

The second sample is obtained from a pregnant woman with cancer with multiple chromosomal abnormalities that affect amongst others chromosome 16 and chromosome 13.

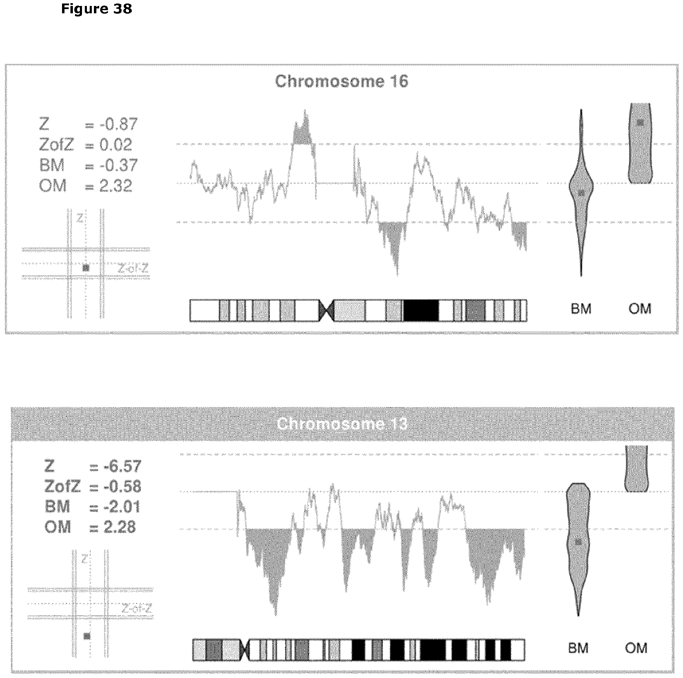

From the plot in figure 38, it can be derived that chromosome 16 does not display a clear whole-chromosome gain or loss, and this is also confirmed by the Z-score which is not significant (Z-score = -0.8746). Also the ZofZ-parameter is not significant (ZofZ-score = 0.0157).

Figure 38 does show that there is a loss for chromosome 13, which is also confirmed by the Z-score that is significantly decreased (Z-score = -6.5688). Also here, the ZofZ-parameter was not significant (ZofZ-score = -0.5782), as there are many abnormal chromosomes in the sample.,

In this case, we compute the NCV score for all possible normalizing chromosomes B (from 1 to 22) and this results in the following tables:

| chr B | Test chromosome 16 | | | | Conclusion |
|---|---|---|---|---|---|
| | $dose_{16(B)}$ | $\mu_{16(B)}$ | $\sigma_{16(B)}$ | $CNV_{16(B)}$ | |
| 1 | 0.9264 | 0.9124 | 0.0065 | 2.1334 | Normal |
| 2 | 0.8709 | 0.9000 | 0.0068 | -4.2897 | Monosomy |
| 3 | 0.8610 | 0.8831 | 0.0055 | -3.9771 | Monosomy |

(continued)

| chr B | Test chromosome 16 | | | | |
|---|---|---|---|---|---|
| | $dose_{16(B)}$ | $\mu_{16(B)}$ | $\sigma_{16(B)}$ | $CNV_{16(B)}$ | Conclusion |
| 4 | 0.9354 | 0.9147 | 0.0067 | 3.0941 | Trisomy |
| 5 | 0.8771 | 0.8999 | 0.0056 | -4.1031 | Monosomy |
| 6 | 0.9081 | 0.9038 | 0.0075 | 0.5771 | Normal |
| 7 | 0.9932 | 0.9685 | 0.0068 | 3.6038 | Trisomy |
| 8 | 0.8775 | 0.8993 | 0.0051 | -4.2361 | Monosomy |
| 9 | 0.9506 | 0.9936 | 0.0057 | -7.5086 | Monosomy |
| 10 | 0.9280 | 0.9125 | 0.0066 | 2.3428 | Normal |
| 11 | 0.9387 | 0.9077 | 0.0054 | 5.7277 | Trisomy |
| 12 | 0.8615 | 0.9129 | 0.0062 | -8.3576 | Monosomy |
| 13 | 0.9079 | 0.8953 | 0.0074 | 1.6912 | Normal |
| 14 | 0.9027 | 0.9195 | 0.0066 | -2.5550 | Normal |
| 15 | 0.9717 | 0.9636 | 0.0067 | 1.2110 | Normal |
| 16 | 1.0000 | 1.0000 | 0.0000 | - | - |
| 17 | 0.9551 | 0.9698 | 0.0060 | -2.4457 | Normal |
| 18 | 0.8755 | 0.8773 | 0.0048 | -0.3714 | Normal |
| 19 | 1.0678 | 1.1387 | 0.0102 | -6.9513 | Monosomy |
| 20 | 0.8602 | 0.8653 | 0.0044 | -1.1647 | Normal |
| 21 | 0.9713 | 0.9532 | 0.0078 | 2.3157 | Normal |
| 22 | 1.0067 | 0.9828 | 0.0064 | 3.7487 | Trisomy |

| chr B | Test chromosome 13 | | | | |
|---|---|---|---|---|---|
| | $dose_{13(B)}$ | $\mu_{13(B)}$ | $\sigma_{13(B)}$ | $CNV_{13(B)}$ | Conclusion |
| 1 | 1.0204 | 1.0192 | 0.0052 | 0.2320 | Normal |
| 2 | 0.9592 | 1.0053 | 0.0038 | -12.1948 | Monosomic |
| 3 | 0.9484 | 0.9864 | 0.0038 | -9.9412 | Monosomic |
| 4 | 1.0303 | 1.0217 | 0.0033 | 2.5790 | Normal |
| 5 | 0.9660 | 1.0052 | 0.0040 | -9.7196 | Monosomic |
| 6 | 1.0003 | 1.0095 | 0.0039 | -2.3932 | Normal |
| 7 | 1.0939 | 1.0818 | 0.0032 | 3.7431 | Trisomic |
| 8 | 0.9665 | 1.0045 | 0.0041 | -9.2688 | Monosomic |
| 9 | 1.0470 | 1.1098 | 0.0049 | -12.8155 | Monosomic |
| 10 | 1.0221 | 1.0193 | 0.0045 | 0.6367 | Normal |
| 11 | 1.0339 | 1.0139 | 0.0043 | 4.6718 | Trisomic |
| 12 | 0.9489 | 1.0197 | 0.0045 | -15.8900 | Monosomic |
| 13 | 1.0000 | 1.0000 | 0.0000 | - | - |
| 14 | 0.9943 | 1.0270 | 0.0049 | -6.6998 | Monosomic |
| 15 | 1.0703 | 1.0763 | 0.0058 | -1.0410 | Normal |
| 16 | 1.1015 | 1.1170 | 0.0093 | -1.6768 | Normal |
| 17 | 1.0520 | 1.0832 | 0.0071 | -4.3887 | Monosomic |
| 18 | 0.9644 | 0.9799 | 0.0043 | -3.6181 | Monosomic |
| 19 | 1.1762 | 1.2719 | 0.0128 | -7.4612 | Monosomic |

(continued)

|  | Test chromosome 13 | | | | |
| --- | --- | --- | --- | --- | --- |
| chr B | dose$_{13(B)}$ | $\mu_{13(B)}$ | $\sigma_{13(B)}$ | CNV$_{13(B)}$ | Conclusion |
| 20 | 0.9475 | 0.9666 | 0.0062 | -3.0830 | Monosomic |
| 21 | 1.0698 | 1.0647 | 0.0050 | 1.0396 | Normal |
| 22 | 1.1089 | 1.0978 | 0.0069 | 1.6000 | Normal |

Hence, depending on which normalizing chromosome was chosen, the test chromosome was classified as normal, monosomic or trisomic.

When using chromosome 7 as normalizing chromosome, both chromosome 13 and 16 are reported as a gain, which is incorrect. In reality chromosome 13 is a loss, whereas chromosome 16 is neither a whole-chromosome gain nor whole-chromosome loss.

When using chromosome 11 as normalizing chromosome, also here both chromosome 13 and 16 would have been reported as a gain, which is again incorrect.

We can conclude that the traditional NCV method is not robust in case there are multiple abnormalities in a sample as it can miss the presence of an abnormality and can even report the opposite results (gain versus loss). The methodology of the present teachings however is independent of such internal references.

**Example 10: Advantage of the ZofZ parameter according to the present teachings to assess the behavior of the target chromosome within the context of the sample:**

Typical prior art methodologies are not capable of assessing the behavior of the target chromosome within the context of the sample. However, this is important, as this brings (i) additional information (to increase sensitivity, see the next first sample) and (ii) more accurate information (to increase specificity, see the next 2nd and 3rd samples) to the user.

A first sample shows a clear gain based on the visual representation for chromosome 21: the overall signal is above 0 across the entire length of the chromosome, and the noise does not scatter around 0. However, when being calculated, the Z-score of chromosome 21 is not significantly increased (Z-score = 2.7965) and therefore this sample would not have been picked up for proper follow-up (e.g. request a second blood draw, report a moderately increased risk for trisomy, recommend additional biochemical/ultrasound testing, suggest an invasive test, ...) using the Z-score on its own. The same most probably holds for any other Z-score that is merely assessing an increase of read count vis-à-vis a normalizing chromosome or reference sample.

However, the ZofZ parameter as calculated according to the present teachingsis 3.4080, which indicates that this chromosome behaves significantly abnormal as compared to the population of chromosomes in the test sample. Hence having the ZofZ parameter increases the sensitivity of the analysis, and will enable giving proper follow-up to this woman. Also note that the Quality Score (QS) equals 0.7292, thereby confirming that this is not a noisy sample.

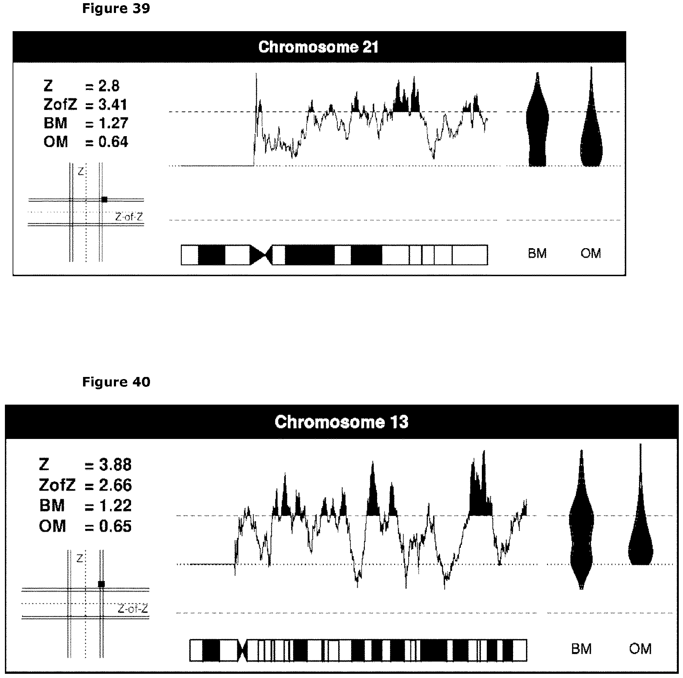

A second sample was retrieved which showed a lot of noise (QS = 1.63) (figure 40). The Z-score is significantly increased (3.8761), and hence based on this Z-score, one would predict the presence of a fetal trisomy, thereby recommending further testing (e.g. invasive test with the risk of inducing a miscarriage).

However, when calculating the ZofZ parameter, the latter does not show a specific increase (2.6649), hence leading the practitioner to understand that the observed increase of Z-score is unlikely to be caused by a fetal trisomy. Further testing (like e.g. invasive testing) should not be immediately advised, but rather re-testing of the sample or resampling should occur.

Hence a significantly different Z-score does not automatically mean that there is a fetal trisomy, and having the ZofZ parameter increases the specificity of the analysis.

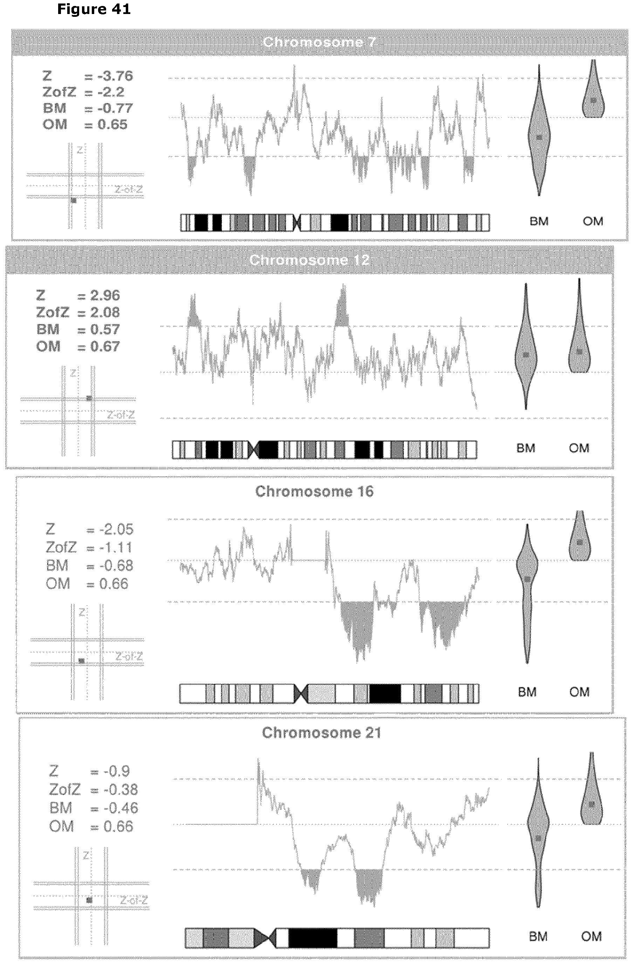

Note that this sample contains multiple chromosomes with a noisy pattern (see examples for chromosome 7, 12, 16 and 21 for the same sample), further confirming that an invasive test should not be recommended, and that rather e.g. a second blood re-draw or re-test of the sample should be recommended (see figure 41).

A third sample is a sample from a pregnant woman with cancer. The Z-score for chromosome 21 is significantly increased (Z-score = 20.1), and hence if only considering this Z-score, one would assume the presence of a fetal trisomy 21, thereby recommending an invasive test (with the risk of inducing a miscarriage). However, as the ZofZ parameter is not significantly increased (ZofZ-score = 0.64), the practitioner will realize that the increase of Z-score is unlikely to be caused by a fetal trisomy.

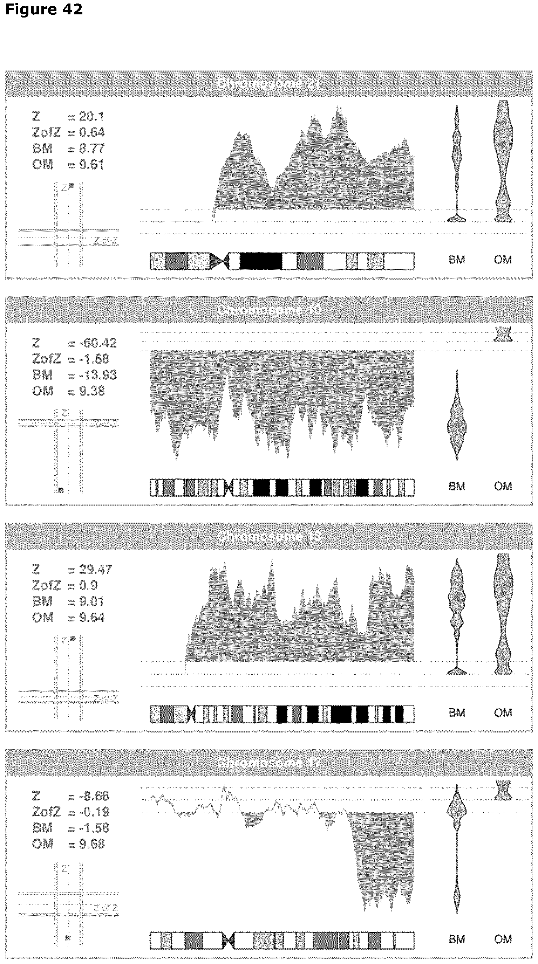

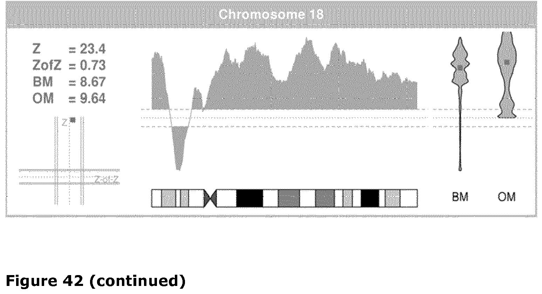

Moreover, the entire set of chromosomes (see plots in figure 42) strongly suggests that the pregnant woman has genome-wide instability (cancer), which makes that re-testing or blood re-draw in order to assess the fetal trisomy is unlikely to enable the evaluation of the true aneuploidy state of the fetus. Hence a significantly different Z-score does not automatically mean that there is a fetal trisomy, and having the ZofZ parameter increases the specificity of the analysis.

In this case, one could e.g. decide to start cancer therapy while the woman is still pregnant, decide to initiate the delivery a few weeks earlier (to decrease the time to treatment). Hence the ZofZ parameter provides better treatment options for the pregnant woman with cancer.

**Example 11: Minimal reference set size**

A reference set was gradually expanded, starting from 3 reference samples and expanding to 5, 10, 15, 20, 25, 50 and 100 reference samples. For each of these reference sets, the Z-score and Z-of-Z parameter of 50 samples known to be normal (normal samples in the figures), 5 samples known to carry a trisomy 21 and 3 samples with a trisomy 18 was calculated.

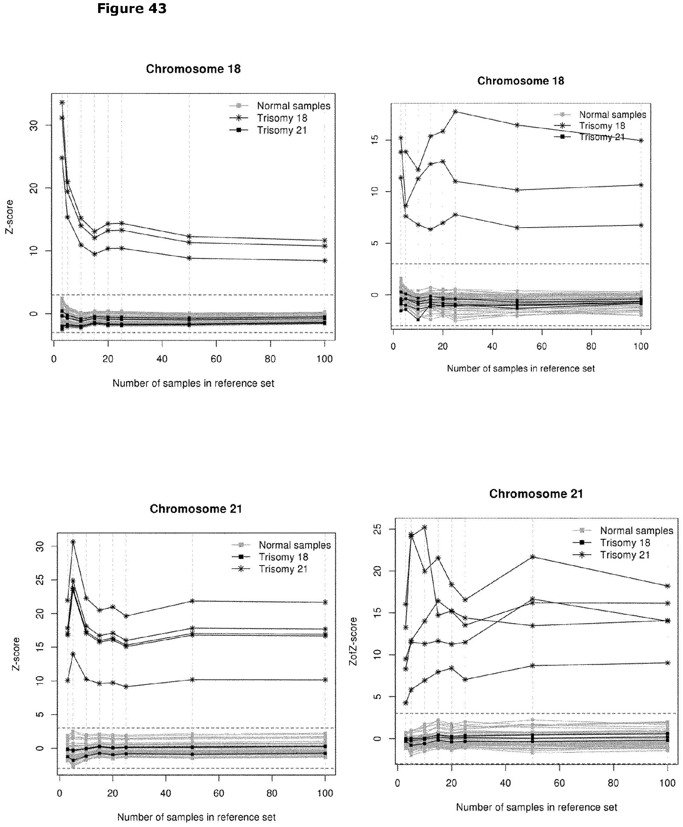

Figure 43 show the Z score at the left and the ZofZ-parameter at the right versus the number of samples included in the reference set. The horizontal dashed line shows a cut-off on the Z or ZofZ-parameter of +3 or -3, so values in between the dashed lines can be considered not significant, while values above the dashed in lines can be considered to be significantly increased (and hence indicate the presence of a trisomy). From the graphs it is clear that even with the smallest reference set of only 3 samples, all normal samples are categorized as having a normal chromosome 18 and 21 ($-3 < Z < 3$ and $-3 < ZofZ < 3$) and all trisomy cases are correctly picked up as significantly abnormal ($Z > 3$ and $ZofZ > 3$).

It is supposed that the present teachings are not restricted to any form of realization described previously and that some modifications can be added to the presented example of fabrication without reappraisal of the appended claims.

**Claims**

1. A method for identifying the presence of tumor-derived cell-free DNA in a mammal, said method comprising:

   - providing the sequences of at least a segment of the nucleic acid molecules contained in a biological sample obtained from a subject, said biological sample comprises cell-free DNA;
   - aligning said obtained sequences to a reference genome;
   - counting the number of reads on a set of chromosomal segments and/or chromosomes thereby obtaining read counts;
   - normalizing said read counts or a derivative thereof into a normalized number of reads;
   - obtaining a first score of said normalized reads and obtaining a collection of scores of said normalized reads, whereby said first score is derived from the normalized reads for a target chromosome or chromosomal segment and whereby said collection of scores is a set of scores derived from the normalized number of reads for a set of chromosomes or chromosome segments that include said target chromosomal segment or chromosome;
   - calculating a parameter p from said first score and said collection of scores, whereby said parameter represents a ratio or correlation between

       * said first score, corrected by a summary statistic of said collection of scores, and
       * a summary statistic of said collection of scores; and

   - comparing said parameter by a cutoff value, whereby said cutoff value is indicative for the presence or absence of one or more aneuploidies in said target chromosome or chromosome segment which is an indicator of the presence of tumor-derived cell-free DNA.

2. The method according to claim 1, **characterized in that** said number of reads are recalibrated to correct for GC content and/or total number of reads obtained from said sample.

3. The method according to claim 1 or 2, **characterized in that** said normalization occurs via comparison with data obtained from the corresponding chromosomal segments or chromosomes from a reference set.

4. The method according to any one of the claims 1 to 3, **characterized in that** said summary statistic is the mean, median, standard deviation, mean absolute deviation or the median absolute deviation.

5. The method according to any one of the previous claims, wherein the sequencing is performed randomly on a segment of the nucleic acid molecules contained in the biological sample.

6. The method according to any one of the previous claims, wherein the biological sample is cerebrospinal fluid, blood, plasma, serum, urine, transcervical fluid or saliva.

7. The method according to any one of the previous claims, wherein said cutoff value or range is established using standard statistical considerations, or empirically established by using biological samples

8. The method according to any one of the previous claims whereby said first score is calculated as:

$$Zi = \frac{GRi - \mu\,ref,i}{\sigma\,ref,i}$$

whereby i is a chromosome or chromosomal segment or the target chromosome or target chromosomal segment.

9. The method according to anyone of the previous claims, **characterized in that** said parameter p is calculated as:

$$Z\,of\,Z_i = \frac{Z_i - \operatorname*{median}_{j=i,a,b,\ldots}(Z_j)}{\operatorname*{mad}_{j=i,a,b,\ldots}(Z_j)}$$

whereby (Zj) represents a collection of scores that are derived from chromosomes or chromosomal segments i, a, b, .. whereby i corresponds to the target chromosomal segment or chromosome.

10. The method according to any of the previous claims, comprising the calculation of secondary parameters, whereby said secondary parameters are indicative of the amount of said aneuploidy if found present and/or a measure of the quality of the sample.

11. The method according to claim 10, whereby said secondary parameters are compared to a cutoff value or range.

12. The method according to any one of the claims 10 or 111, said presence or absence of an aneuploidy is determined by the comparison of said parameter to a cutoff value or range and the comparison of one or more secondary parameters and corresponding cutoff values or range.

13. The method of any one of the preceding claim, wherein said aneuploidy comprise whole chromosome aneuploidy, a loss, a gain, an amplification or a deletion of a substantial arm level segment of a chromosome.

14. The method of claim 13, wherein said whole chromosome aneuploidy comprises a gain or a loss as shown in Table 2.

15. The method of claim 14, wherein said target chromosomal segments are substantially arm-level segments comprising a p arm or a q arm of any one or more of chromosomes 1-22, X and Y.

16. The method of claim 15, wherein said target chromosomal segment comprises one or more arms selected from the group consisting of 1q, 3q, 4p, 4q, 5p, 5q, 6p, 6q, 7p, 7q, 8p, 8q, 9p, 9q, 10p, 10q, 12p, 12q, 13q, 14q, 16p, 17p, 17q, 18p, 18q, 19p, 19q, 20p, 20q, 21q, and/or 22q, more preferably selected from the group consisting of 1q, 3q, 4p, 4q, 5p, 5q, 6p, 6q, 7p, 7q, 8p, 8q, 9p, 9q, 10p, 10q, 12p, 12q, 13q, 14q, 16p, 17p, 17q, 18p, 18q, 19p, 19q, 20p, 20q, 21q, 22q.

17. The method of any of the preceding claims, wherein said chromosomal segments are segments that comprise a region and/or a gene shown in Table 4 and/or Table 5 and/or Table 6 and/or Table 7.

18. The method of any of the claims 1 to 17, wherein said aneuploidy comprises an amplification of a region and/or a gene shown in Table 4 and/or Table 6.

19. The method of any of the claims 1 to 17, wherein said aneuploidy comprises a deletion of a region and/or a gene shown in Table 5 and/or Table 7.

20. The method of any of the claims 1 to 17, wherein said aneuploidy comprises an amplification of one or more regions selected from the group consisting of 20Q13, 19q12, 1q21-1q23, 8p11-p12, MYC, ERBB2 (EFGR), CCND1 (Cyclin D1), FGFR1, FGFR2, HRAS, KRAS, MYB, MDM2, CCNE, NRAS, MET, ERBB1, CDK4, MYCB, ERBB2, AKT2, MDM2, BRAF, ARAF, CRAF, PIK3CA, AKT1, PTEN, STK11, MAP2K1, ALK, ROS1, CTNNB1, TP53, SMAD4, FBX7, FGFR3, NOTCH1, ERBB4 and CDK4, and the like.

21. The method of any of the claims 1 to 12, wherein said cancer is a cancer selected from the group consisting of leukemia, ALL, brain cancer, breast cancer, colorectal cancer, dedifferentiated liposarcoma, esophageal adenocarcinoma, esophageal squamous cell cancer, GIST, glioma, HCC, hepatocellular cancer, lung cancer, lung NSC, lung SC, medulloblastoma, melanoma, MPD, myeloproliferative disorder, cervical cancer, ovarian cancer, prostate cancer, and renal cancer.

22. A computer program product comprising a computer readable medium encoded with a plurality of instructions for controlling a computing system to perform an operation for performing the analysis the presence of a cancer and/or an increased risk of a cancer in a mammal in a biological sample obtained from a subject, wherein the biological sample includes nucleic acid molecules, the operation comprising the steps of

- receiving the sequences of at least a segment of the nucleic acid molecules contained in a biological sample obtained from subject, said biological sample comprises cell-free DNA;
- aligning said obtained sequences to a reference genome;
- counting the number of reads on a set of chromosomal segments and/or chromosomes thereby obtaining the read counts;
- normalizing said read counts or a derivative thereof into a normalized number of reads;
- obtaining a first score of said normalized reads and obtaining a collection of scores of said normalized reads, whereby said first score is derived from the normalized reads for a target chromosome or chromosomal segment and whereby said collection of scores is a set of scores derived from the normalized reads for a set of chromosomes or chromosomal segments that include said target chromosomal segment or chromosome;
- calculating a parameter p from said first score and said collection of scores, whereby said parameter represents a ratio or correlation between

    * said first score, corrected by a summary statistic of said collection of scores, and
    * a summary statistic of said collection of scores; and

- comparing said parameter by a cutoff value or range, whereby said cutoff value or range is indicative for the presence or absence of one or more aneuploidies in said target chromosome or chromosome segment which is an indicator of the presence and/or increased risk of cancer .

23. Computer program product according to claim 22, further comprising operations for calculating one or more secondary parameters, whereby said secondary parameters are indicative for the intensity of said aneuploidy if found present and/or a measure of quality of the sample.

## Figure 1

## Figure 2

**Figure 3**

Chromosome 11                                                        Sample A

Z    = 0.63
ZZ2  = 0.32
BM   = 0.22
OM   = 0.55

BM        OM

**Figure 4**

Sample A: BM scores

Chromosome

**Figure 5**

Chromosome 16                                                        Sample B

Z    = 5.75
ZZ2  = 4.11
BM   = 3.01
OM   = 0.55

BM        OM

## Figure 6

**Sample B: ZZ2 scores**

## Figure 7

**Figure 8**

### Sample B: BM scores

**Figure 9**

**Figure 10**

Sample A: ZofZ scores

**Figure 11**

**Figure 12**

**Figure 13**

**Figure 14**

**Figure 15**

## Sample A: OM scores

**Figure 16**

## Sample B: OM scores

**Figure 17**

**Figure 18**

**Figure 19**

**Figure 20**

**Figure 21**

Chromosome 18 — Sample E

Z = 13.32
ZofZ = 17.02
BM = 4.34
OM = 0.69

**Figure 22**

Chromosome 20 — Sample I

Z = 3.09
ZofZ = 3.31
BM = 1.51
OM = 1.05

**Figure 23**

Chromosome 22 — Sample F

Z = -3.2
ZofZ = -3.09
BM = -1.57
OM = 0.77

**Figure 24**

**Figure 25**

**Figure 26**

**Figure 27**

**Figure 28**

**Figure 29**

1: 148539255-149765886                    1: 148539255-149765886

**Figure 30**

**Figure 31**

Sample G: OM scores

**Figure 32**

## Figure 32 (Continued)

**Figure 33**

**Figure 34**

Non-curated reference set of 24 samples

**Figure 35**

Curated reference set of 20 samples

**Figure 36**

**Figure 37**

**Figure 38**

**Figure 39**

Chromosome 21

Z = 2.8
ZofZ = 3.41
BM = 1.27
OM = 0.64

BM    OM

**Figure 40**

Chromosome 13

Z = 3.88
ZofZ = 2.66
BM = 1.22
OM = 0.65

BM    OM

**Figure 41**

**Figure 42**

Chromosome 18

Z = 23.4
ZofZ = 0.73
BM = 8.67
OM = 9.64

BM    OM

**Figure 42 (continued)**

**Figure 43**

Chromosome 18

Chromosome 18

Chromosome 21

Chromosome 21

**Figure 44**

Step 1: Sample preparation and sequencing

- Blood collection and plasma separation
- cfDNA extraction and quantification
- NGS library preparation, quality control and normalisation
- Pooling of NGS libraries of different samples
- Performing NGS run

Step 2: Mapping of reads and filtering of mapped reads

Step 3: Calculation of Genomic Representation

Step 4: Calculation of Scores and Parameters

- Z, ZZ2, ZofZ, BM and OM per chromosome and optionally per window
- Determination of gender
- Quantification of the fetal fraction in sample

Step 5: Interpretation, verification and/or diagnosis

| | Europäisches Patentamt |
|---|---|
| | European Patent Office |
| | Office européen des brevets |

## EUROPEAN SEARCH REPORT

Application Number

EP 19 20 4072

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/039556 A1 (GUARDANT HEALTH INC [US]; TALASAZ AMIRALI [US]; ELTOUKHY HELMY [US]) 13 March 2014 (2014-03-13) | 1-13, 21-23 | INV. C12Q1/6869 G16B30/00 |
| Y | * abstract; claims 1-3,6,8,9,13-15,42,43,57,61,66,70,77,103; example 1 * <br> * paragraphs [0076], [0085], [0294] * | 1-23 | |
| Y | DAVID J. GORDON ET AL: "Causes and consequences of aneuploidy in cancer", NATURE REVIEWS GENETICS, vol. 13, no. 3, 1 March 2012 (2012-03-01), pages 189-203, XP55654691, GB ISSN: 1471-0056, DOI: 10.1038/nrg3123 | 14-21 | |
| A | * abstract; table 1 * | 1-13,22, 23 | |
| X | WO 2014/209597 A2 (ARIOSA DIAGNOSTICS INC [US]) 31 December 2014 (2014-12-31) | 1-13,22, 23 | |
| Y | * abstract * <br> * paragraphs [0004] - [0006], [0008], [0009], [0012], [0018] - [0022], [0039], [0104], [0105], [0122] * | 1-23 | TECHNICAL FIELDS SEARCHED (IPC) <br><br> C12Q |
| Y | WO 2011/102998 A2 (HELICOS BIOSCIENCES CORP [US]; LAPIDUS STANLEY N [US] ET AL.) 25 August 2011 (2011-08-25) <br> * abstract * <br> * page 2 * <br> * page 20 * <br> * page 22, last paragraph * <br> * page 27, paragraph 2 - paragraph 3 * <br> * page 29, paragraph 31 - paragraph 33 * | 1-23 | |

-/--

The present search report has been drawn up for all claims

| Place of search | Date of completion of the search | Examiner |
|---|---|---|
| Munich | 8 January 2020 | Celler, Jakub |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

| | Europäisches Patentamt<br>European Patent Office<br>Office européen des brevets | **EUROPEAN SEARCH REPORT** | **Application Number**<br>EP 19 20 4072 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | BARAN BAYINDIR ET AL: "Noninvasive prenatal testing using a novel analysis pipeline to screen for all autosomal fetal aneuploidies improves pregnancy management",<br>EUROPEAN JOURNAL OF HUMAN GENETICS.,<br>vol. 23, no. 10,<br>14 January 2015 (2015-01-14), pages 1286-1293, XP55378014,<br>CH<br>ISSN: 1018-4813, DOI:<br>10.1038/ejhg.2014.282<br>* abstract *<br>* "Discussion";<br>page 1291 - page 1292 *<br>----- | 1-23 | |
| | | | TECHNICAL FIELDS SEARCHED (IPC) |

The present search report has been drawn up for all claims

| Place of search | Date of completion of the search | Examiner |
|---|---|---|
| Munich | 8 January 2020 | Celler, Jakub |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

**EP 3 636 777 A1**

## ANNEX TO THE EUROPEAN SEARCH REPORT
## ON EUROPEAN PATENT APPLICATION NO.

EP 19 20 4072

08-01-2020

| Patent document cited in search report | Publication date | Patent family member(s) | Publication date |
|---|---|---|---|
| WO 2014039556 A1 | 13-03-2014 | BR 112015004847 A2 | 04-07-2017 |
| | | CA 2883901 A1 | 13-03-2014 |
| | | CN 104781421 A | 15-07-2015 |
| | | DK 2893040 T3 | 11-03-2019 |
| | | EP 2893040 A1 | 15-07-2015 |
| | | EP 3470533 A1 | 17-04-2019 |
| | | EP 3591073 A1 | 08-01-2020 |
| | | ES 2711635 T3 | 06-05-2019 |
| | | GB 2533006 A | 08-06-2016 |
| | | HK 1201080 A1 | 21-08-2015 |
| | | HK 1212396 A1 | 10-06-2016 |
| | | IL 237480 A | 31-10-2019 |
| | | JP 6275145 B2 | 07-02-2018 |
| | | JP 2015535681 A | 17-12-2015 |
| | | JP 2018027096 A | 22-02-2018 |
| | | KR 20150067161 A | 17-06-2015 |
| | | KR 20190112843 A | 07-10-2019 |
| | | MX 367963 B | 11-09-2019 |
| | | PL 2893040 T3 | 31-05-2019 |
| | | PT 2893040 T | 01-04-2019 |
| | | SG 11201501662T A | 28-05-2015 |
| | | US 2015299812 A1 | 22-10-2015 |
| | | US 2015368708 A1 | 24-12-2015 |
| | | US 2017218459 A1 | 03-08-2017 |
| | | US 2017218460 A1 | 03-08-2017 |
| | | US 2018171415 A1 | 21-06-2018 |
| | | US 2018223374 A1 | 09-08-2018 |
| | | US 2018327862 A1 | 15-11-2018 |
| | | US 2019177802 A1 | 13-06-2019 |
| | | US 2019177803 A1 | 13-06-2019 |
| | | US 2019185940 A1 | 20-06-2019 |
| | | US 2019185941 A1 | 20-06-2019 |
| | | WO 2014039556 A1 | 13-03-2014 |
| WO 2014209597 A2 | 31-12-2014 | EP 3014001 A2 | 04-05-2016 |
| | | US 2015004601 A1 | 01-01-2015 |
| | | WO 2014209597 A2 | 31-12-2014 |
| WO 2011102998 A2 | 25-08-2011 | AU 2011218382 A1 | 11-10-2012 |
| | | AU 2015246128 A1 | 12-11-2015 |
| | | CN 103108960 A | 15-05-2013 |
| | | CN 109411017 A | 01-03-2019 |
| | | EP 2536852 A2 | 26-12-2012 |
| | | US 2010216153 A1 | 26-08-2010 |
| | | US 2013022977 A1 | 24-01-2013 |
| | | US 2014322709 A1 | 30-10-2014 |

page 1 of 2

## ANNEX TO THE EUROPEAN SEARCH REPORT
## ON EUROPEAN PATENT APPLICATION NO.

EP 19 20 4072

This annex lists the patent family members relating to the patent documents cited in the above-mentioned European search report.
The members are as contained in the European Patent Office EDP file on
The European Patent Office is in no way liable for these particulars which are merely given for the purpose of information.

08-01-2020

| Patent document cited in search report | Publication date | Patent family member(s) | Publication date |
|---|---|---|---|
| | | WO 2011102998 A2 | 25-08-2011 |

For more details about this annex : see Official Journal of the European Patent Office, No. 12/82

EPO FORM P0459

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2183693 A
- US 8195415 B
- US 20130034546 A
- US 20130310263 A

### Non-patent literature cited in the description

- **VANDENBERGHE et al.** *Non-invasive detection of genomic imbalances in Hodgkin/Reed-Sternberg cells in early and advanced stage Hodgkin's lymphoma by sequencing of circulating cell-free DNA: a technical proof-of-principle study,* 2015
- **BENJAMINI et al.** *Nucleic Acid Research,* 2012
- **LIN et al.** *Cancer Res,* 2008, vol. 68, 664-673
- **GEORGE et al.** *PLoS ONE,* 2007, vol. 2, e255
- **DEMICHELIS et al.** *Genes Chromosomes Cancer,* 2009, vol. 48, 366-380
- **BEROUKHIM et al.** *Nature,* 2010, vol. 463 (7283), 899-905
- **PARK et al.** *Clinical Breast Cancer,* 2008, vol. 8, 392-401
- **VARMUS H.** *Ann Rev Genetics,* 1984, vol. 18, 553-612
- **WATSON et al.** Molecular Biology of the Gene. Benjamin/Cummings Publishing Co, 1987
- **HOWE et al.** *Proc Natl Acad Sci (USA),* 1990, vol. 87, 5883-5887
- **RYGAARD et al.** *Cancer Res,* 1990, vol. 50, 5312-5317
- **BOWCOCK et al.** *Am J Hum Genet,* 1990, vol. 46, 12
- **CARAMAZZA et al.** *Eur J Hematol,* 2010, vol. 84, 191-200
- **EISENMANN et al.** *Oncogene,* 2009, vol. 28, 3429-3441
- **ANGELONI D.** *Briefings Functional Genomics,* 2007, vol. 6, 19-39
- **FONATSCH C.** *Gene Chromosomes Cancer,* 2010, vol. 49, 497-508